(19) Europäisches Patentamt
European Patent Office
Office européen des brevets

(11) EP 0 576 350 B1

(12) **FASCICULE DE BREVET EUROPEEN**

(45) Date de publication et mention de la délivrance du brevet:
**29.10.1997 Bulletin 1997/44**

(21) Numéro de dépôt: **93401603.1**

(22) Date de dépôt: **22.06.1993**

(51) Int. Cl.[6]: **C07D 277/42**, C07D 417/12, A61K 31/425

(54) **Dérivés alkylamino ramifiés du thiazole, leurs procédés de préparation et les compositions pharmaceutiques qui les contiennent**

Verzweigte Alkylaminothiazolederivate, Verfahren zu ihrer Herstellung und pharmazeutische Zusammenstellungen die sie enthalten

Branched alkylamino thiazole derivatives, process for their preparation and pharmaceutical compositions containing them

(84) Etats contractants désignés:
**AT BE CH DE DK ES FR GB GR IE IT LI LU MC NL PT SE**

(30) Priorité: **24.06.1992 FR 9207736**

(43) Date de publication de la demande:
**29.12.1993 Bulletin 1993/52**

(73) Titulaire: **SANOFI**
**75008 Paris (FR)**

(72) Inventeurs:
- **Courtemanche, Gilles**
  **F-95270 Saint Martin du Tertre (FR)**
- **Gautier, Claudie**
  **F-75013 Paris (FR)**
- **Gully, Danielle**
  **F-31600 Saubens (FR)**
- **Roger, Pierre**
  **F-78180 Montigny le Bretonneux (FR)**
- **Valette, Gérard**
  **F-31120 Lacroix Falgarde (FR)**
- **Wermuth, Camille Georges**
  **F-67100 Strasbourg (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
**EP-A- 0 283 390**
**WO-A-91/09857**
**GB-A- 2 022 085**

EP 0 576 350 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.19/3.4

## Description

La présente invention concerne des dérivés alkylamino ramifiés du thiazole, leurs procédés de préparation et les compositions pharmaceutiques qui les contiennent.

Plusieurs dérivés du 2-amino thiazole sont déjà connus. La demande de brevet EP-0 462 264 décrit des dérivés du 2-amino thiazole, dont l'amine tertiaire en position 2, comporte deux substituants ayant chacun au moins un hétéroatome. Ces substituants sont des dérivés d'amines aromatiques ou aliphatiques ou encore des dérivés d'acides, de cétones, d'amides ou de thiocétones. Tous ces composés sont des antagonistes du PAF-acéther et trouvent leurs applications dans le traitement de l'asthme, de certains états allergiques ou inflammatoires, de maladies cardiovasculaires, de l'hypertension et de diverses pathologies rénales ou encore comme agents contraceptifs. La demande GB-2 022 285 décrit des composés possédant une activité régulatrice de la réponse immunitaire et ayant des propriétés anti-inflammatoires. Il s'agit de dérivés du thiazole substitués en position 2 par des groupes amines secondaires.

Certains dérivés hétérocycliques de 2-acylamino thiazole ont été décrits dans la demande de brevet EP-0 432 040. Ces composés sont des antagonistes de la cholecystokinine et de la gastrine. Des dérivés du 2-amino 4,5-diphényl thiazole ayant des propriétés antiinflammatoires sont aussi connus (demande de brevet JP-01 75 475). On connaît aussi des dérivés du 2-amino 4-(4-hydroxyphényl) thiazole utiles comme intermédiaires de synthèse pour la préparation des dérivés du 2,2-diaryl chroméno thiazole (demande de brevet EP-0 205 069). Des dérivés du 2-(N-méthyl N-benzyl amino) thiazole sont aussi décrits dans J. Chem. Soc. Perkin, Trans 1, (1984), 2, pp. 147-153 et dans J. Chem. Soc. Perkin, Trans 1, (1983), 2, pp. 341-347.

La demande de brevet EP-0 283 390, décrit et revendique parmi d'autres dérivés du thiazole, des dérivés du 2-[N-alkyl N-pyridyl alkyl amino] thiazole de formule :

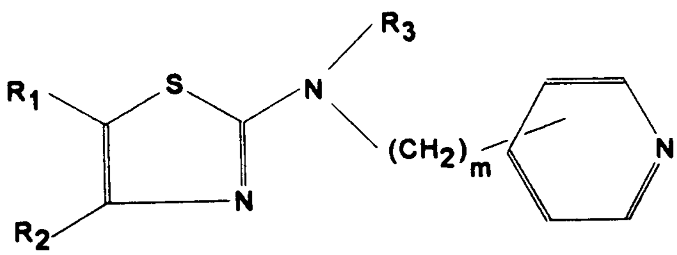

Ces dérivés, dont l'amine en position 2 est substituée par un radical pyridyl alkyle non ramifié, sont doués de propriétés pharmacologiques intéressantes et possèdent notamment une activité stimulante de la transmission cholinergique centrale. Ils peuvent donc être utilisés comme agonistes des récepteurs muscariniques et trouvent leurs applications dans le traitement des troubles de la mémoire et des démences séniles.

Les composés de la présente invention se distinguent d'autres dérivés du 2-amino thiazole décrits dans la littérature, par leurs structures originales et par leurs propriétés pharmacologiques nouvelles.

Il s'agit de dérivés du 2-amino thiazole dont l'amine en position 2 est une amine tertiaire ayant un substituant alkyle ou aralkyle ramifié.

Cette structure particulière, confère aux produits de l'invention des propriétés pharmacologiques très intéressantes. En effet, les composés de l'invention déplacent à de très faibles concentrations - inférieures à 10 μM -, la liaison du $^{125}$I-CRF aux récepteurs spécifiques présents sur membranes de cortex du rat. Les composés de l'invention sont donc des modulateurs des effets du facteur de libération de l'hormone corticotrope (CRF), neuropeptide qui contrôle l'activité de l'axe hypothalamo-hypophyso-surrénalien et trouvent leurs applications dans le traitement des maladies liées au stress, et plus généralement dans le traitement des pathologies impliquant le CRF, telles que par exemples, des désordres psychiatriques, anxiété, anorexie nerveuse ou autres.

La présente invention a plus particulièrement pour objet, les dérivés alkylamino ramifiés du thiazole de formule I :

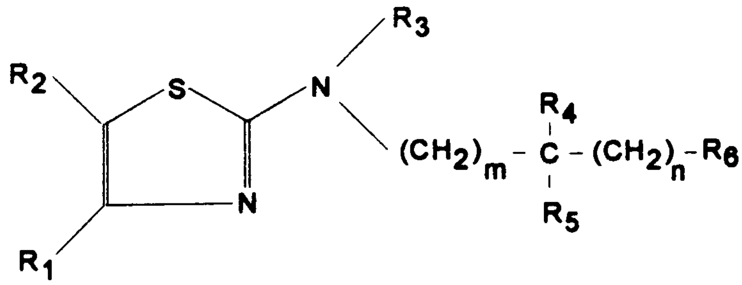
(I)

dans laquelle,

- $R_1$ représente un radical de formule $A_1$ ou un radical de formule $A_2$ :

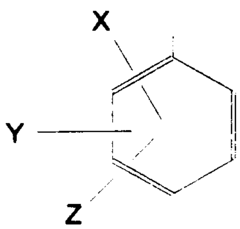
($A_1$)

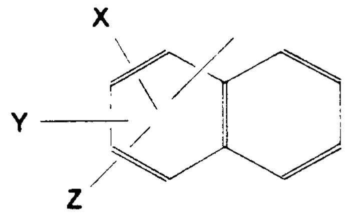
($A_2$)

(dans lesquelles X, Y, Z, identiques ou différents, représentent chacun un atome d'hydrogène, un atome d'halogène, un radical alcoxy de 1 à 5 atomes de carbone, un radical alkyle de 1 à 5 atomes de carbone, un radical hydroxy, un radical cyano, un radical nitro, un radical trifluorométhyle ou un radical aralkyle de 7 à 9 atomes de carbone),
- $R_2$ représente un atome d'hydrogène, un atome d'halogène, un radical alkyle de 1 à 5 atomes de carbone, un radical hydroxyméthyle ou un radical formyle,
- $R_3$ représente un radical alkyle de 1 à 5 atomes de carbone, un radical cycloalkyle de 3 à 8 atomes de carbone, un radical alcényle de 2 à 6 atomes de carbone, un radical cycloalkylalkyle de 4 à 8 atomes de carbone, ou un radical phényle,
- $R_4$ représente un atome d'hydrogène, un radical alkyle de 1 à 5 atomes de carbone, un radical cycloalkyle de 3 à 6 atomes de carbone, ou un radical cyclo alkylalkyle de 4 à 8 atomes de carbone ayant une chaîne linéaire ou ramifiée,
- $R_5$ représente un radical alkyle de 1 à 5 atomes de carbone, un radical cycloalkyle de 3 à 8 atomes de carbone éventuellement substitué par des radicaux alkyle de 1 à 5 atomes de carbone, un radical cycloalkylalkyle de 4 à 8 atomes de carbone ayant une chaîne linéaire ou ramifiée, un radical alcényle de 2 à 6 atomes de carbone, ou un radical de formule B :

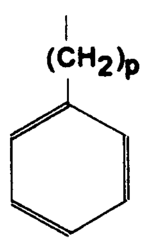
(B)

(dans laquelle p est égal à 0, 1, 2, ou 3),
- $R_6$ représente un radical phényle, un radical pyridyle, un radical imidazolyle, un radical pyrrolyle, un radical thiényle ou un radical furyle (éventuellement substitués par un ou plusieurs atomes d'halogène, par des radicaux alcoxy de 1 à 5 atomes de carbone, par des radicaux alkyle de 1 à 5 atomes de carbone, par des radicaux hydroxy, par des radicaux cyano, par des radicaux nitro, par des radicaux trifluorométhyle, par des radicaux méthylthio ou par des radicaux de formule B), ou un radical cycloalkyle de 3 à 8 atomes de carbone éventuellement substitué par des

radicaux alkyle de 1 à 5 atomes de carbone,

- m, n, identiques ou différents représentent chacun 0 ou 1,

leurs stéréoisoméres et leurs sels d'addition à un acide minéral ou organique.

Les composés préférés de l'invention sont les composés de formule I, dans laquelle,

- $R_1$ représente un radical de formule $A_1$,
- $R_2$ représente un atome d'halogène ou un radical alkyle de 1 à 5 atomes de carbone,
- $R_3$ représente un radical alkyle de 1 à 5 atomes de carbone, un radical cycloalkyle de 3 à 8 atomes de carbone, ou un radical alcényle de 2 à 6 atomes de carbone,
- $R_5$ représente un radical alkyle de 1 à 5 atomes de carbone, un radical cycloalkyle de 3 à 8 atomes de carbone, éventuellement substitué par des radicaux alkyle de 1 à 5 atomes de carbone, ou un radical cycloalkylalkyl de 4 à 8 atomes de carbone ayant une chaîne linéaire ou ramifiée,
  et,
- $R_4$, $R_6$, m et n, ont la même signification que pour la formule I,

leurs stéréoisomères ainsi que leurs sels d'addition à un acide minéral ou organique.

Parmi eux, un groupe de composés particulièrement préféré, peut être représenté par la formule $I_A$ :

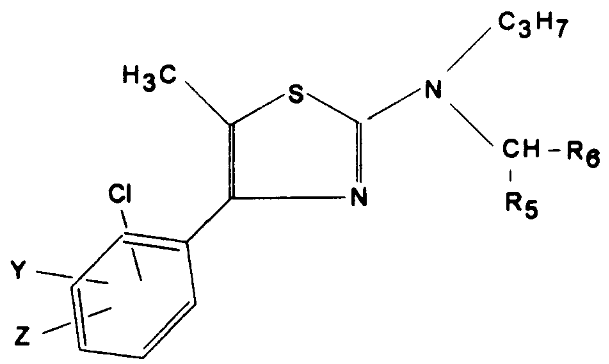

$(I_A)$

dans laquelle,

- Y et Z ont la même signification que pour la formule I,
- $R_5$ représente un radical alkyle de 1 à 5 atomes de carbone ou un radical cycloalkyle de 3 à 8 atomes de carbone, éventuellement substitué par des radicaux alkyle de 1 à 5 atomes de carbone ou un radical cycloalkylalkyle de 4 à 8 atomes de carbone ayant une chaîne linéaire ou ramifiée,
- $R_6$ représente un radical phényle ou un radical pyridyle (éventuellement substitués par un ou plusieurs atomes d'halogène, des radicaux alcoxy de 1 à 5 atomes de carbone, par des radicaux alkyle de 1 à 5 atomes de carbone, des radicaux hydroxy, des radicaux cyano, des radicaux nitro, des radicaux trifluorométhyle ou par des radicaux méthylthio), un radical imidazolyle éventuellement substitué par un radical alkyle de 1 à 5 atomes de carbone, ou un radical cycloalkyle de 3 à 8 atomes de carbone éventuellement substitué par des radicaux alkyle de 1 à 5 atomes de carbone,

leurs stéréoisomères et leurs sels d'addition à un acide minéral ou organique.

Le radical $-C_3H_7$ de la formule $I_A$, représente un radical n-propyle.

Par le terme radical alkyle ou radical alcényle, on entend des radicaux linéaires ou ramifiés.

Parmi les composés préférés de l'invention on peut citer les composés suivants :

Le 4-(4-chloro 2-méthoxy phényl) 5-méthyl 2-[N-propyl N-(1-pyrid-4-yl éth-1-yl) amino] thiazole,
le 4-(4-chloro 2-méthyl phényl) 5-méthyl 2-[N-propyl N-(1-pyrid-4-yl éth-1-yl) amino] thiazole,
le 4-(2-chloro 4-méthyl phényl) 5-méthyl 2-[N-propyl N-(1-pyrid-4-yl éth-1-yl) amino] thiazole,
le 4-(2-chloro 4-méthoxy phényl) 5-méthyl 2-[N-propyl N-(1-pyrid-4-yl éth-1-yl) amino] thiazole,
le 5-bromo 4-(2,4-dichloro phényl) 2-[N-propyl N-(1-pyrid-4-yl éth-1-yl) amino] thiazole,
le 4-(2,4-dichloro phényl) 5-méthyl 2-[N-propyl-2-éne N-(1-pyrid-4-yl éth-1-yl) amino] thiazole,
le 4-(2,4-dichloro phényl) 5-méthyl 2-[N-(α-méthyl benzyl) N-propyl amino] thiazole,
le 4-(2,4-dichloro phényl) 5-méthyl 2-[N-(cyclopropyl pyrid-4-yl méthyl) N-propyl amino] thiazole,

le 4-(2,4-dichloro phényl) 5-méthyl 2-[N-propyl N-(1-pyrid-4-yl éth-1-yl) amino] thiazole,
le 4-(2,4-dichloro phényl) 5-méthyl 2-{N-[1-(2-méthyl pyrid-4-yl) éth-1-yl] N-propyl amino} thiazole,
le 4-(2,4-dichloro phényl) 5-méthyl 2-[N-(1-imidazol-4-yl éth-1-yl) N-propyl amino] thiazole,
le 4-(2,4-dichloro phényl) 5-méthyl 2-[N-propyl N-(1-pyrid-4-yl prop-1-yl) amino] thiazole,
le 4-(2,4-dichloro phényl) 5-méthyl 2-[N-(2-méthyl 1-pyrid-4-yl prop-1-yl) N-propyl amino] thiazole,
le 4-(2,4-dichloro phényl) 5-méthyl 2-[N-(α-cyclopropyl benzyl) N-propyl amino] thiazole,
le 4-(2,4-dichloro phényl) 5-méthyl 2-[N-(α-cyclopropyl 4-methoxy benzyl) N-propyl amino] thiazole,
le 4-(2,4-dichloro phényl) 5-méthyl 2-[N-(cyclopropyl thién-2-yl méthyl) N-propyl amino] thiazole,
le 4-(2,4-dichloro phényl) 5-méthyl 2-[N-(dicyclopropyl méthyl) N-propyl amino] thiazole,
le 4-(2,4-dichloro phényl) 5-méthyl 2-[N-(α-cyclopentyl benzyl) N-propyl amino] thiazole,
le 4-(2,4-dichloro phényl) 5-méthyl 2-[N-(cyclopentyl pyrid-4-yl méthyl) N-propyl amino] thiazole,
le 4-(2,4-dichloro phényl) 5-méthyl 2-[N-(α-cyclopropyl 4-fluoro benzyl) N-propyl amino) thiazole,
le 4-(2,4-dichloro phényl) 5-méthyl 2-{N-[(3-chloro pyrid-4-yl) cyclopropyl méthyl] N-propyl amino} thiazole,
le 4-(2,4-dichloro phényl) 5-méthyl 2-[N-propyl N-(α-pyrid-4-yl benzyl) amino] thiazole,
le 4-(2,4-dichloro phényl) 5-méthyl 2-[N-(cyclopropyl imidazol-4-yl méthyl) N-propyl amino) thiazole,
le 4-(2,4-dichloro phényl) 5-méthyl 2-[N-(α-cyclopropyl 3-trifluorométhyl benzyl) N-propyl amino] thiazole,
le 4-(2,4-dichloro phényl) 5-méthyl 2-[N-(cyclopentyl cyclopropyl méthyl) N-propyl amino] thiazole,
le 4-(2,4-dichloro phényl) 5-méthyl 2-{N-[cyclopropyl (1-méthyl imidazol-4-yl) méthyl] N-propyl amino} thiazole,
le 4-(2,4-dichloro phényl) 5-méthyl 2-{N-[cyclopropyl (1-benzyl imidazol-4-yl) méthyl] N-propyl amino} thiazole,
le 4-(2,4-dichloro phényl) 5-méthyl 2-[N-(cyclopropyl pyrid-2-yl méthyl) N-propyl amino] thiazole,
le 4-(2-chloro 4-méthyl phényl) 5-méthyl 2-[N-(dicyclopropyl méthyl) N-propyl amino] thiazole,
le 4-(2,4-dichloro phényl) 5-méthyl 2-[N-(cyclopropyl pyrid-3-yl méthyl) N-propyl amino] thiazole,
le 4-(2-chloro 4-méthoxy phényl) 5-méthyl 2-[N-(dicyclopropyl méthyl) N-propyl amino] thiazole,
le 4-(2,4-dichloro phényl) 5-méthyl 2-[N-(α-cyclobutyl benzyl) N-propyl amino] thiazole,
le 4-(2,4-dichloro phényl) 5-méthyl 2-[N-(2,2-dicyclopropyl éth-1-yl) N-propyl amino] thiazole.

Tous ces composés peuvent être soit sous forme de base libre, soit sous forme salifiée.

La présente invention a également pour objet un procédé de préparation des composés de formule I, caractérisé en ce que l'on fait réagir un dérivé carbonylé alpha-halogéné, de préférence alpha-bromé, de formule II :

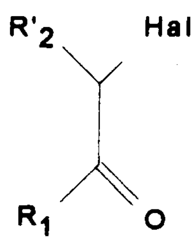

(II)

dans laquelle,
$R_1$ a la même signification que pour la formule I, $R'_2$ représente un atome d'hydrogène ou un radical alkyle de 1 à 5 atomes de carbone, et Hal représente un atome d'halogène,
soit

avec une thiourée de formule III :

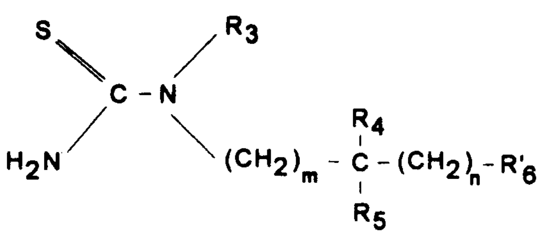

(III)

dans laquelle $R_3$, $R_4$, $R_5$, m et n, ont la même signification que pour la formule I, et $R'_6$ a la même signification que $R_6$ sauf dans le cas où $R_6$ contient des fonctions avec des atomes d'azote réactifs où $R'_6$ désigne alors le radical correspondant à $R_6$ dans lequel un hydrogène de ladite fonction réactive a été remplacée par un groupe protecteur

résistant à l'hydrolyse en milieu alcalin, pour former un composé de formule I' :

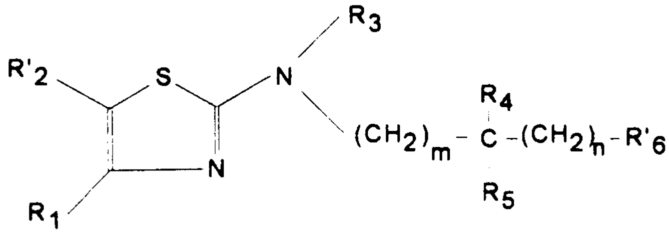

(I')

dans laquelle,

- $R_1$, $R_3$, $R_4$, $R_5$, m et n ont la même signification que pour la formule I,
- $R'_2$ a la signification donnée pour la formule II,
  et,
- $R'_6$ a la signification donnée pour la formule III,

<u>soit</u>

avec une thiourée de formule $III_A$ :

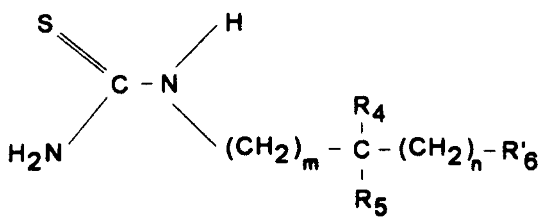

($III_A$)

dans laquelle $R_4$, $R_5$, m et n ont la même signification que pour la formule I, et $R'_6$ a la signification donnée pour la formule III,

pour former les composés de formule IV :

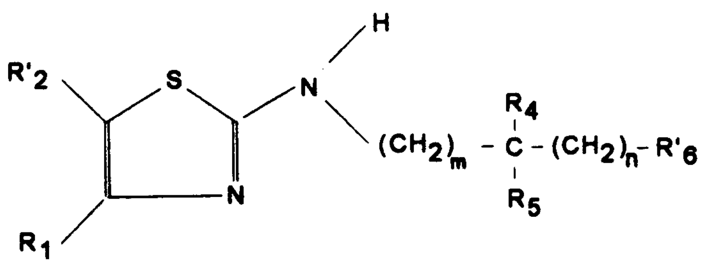

(IV)

dans laquelle $R_1$, $R_4$, $R_5$, m et n ont la même signification que pour la formule I, $R'_2$ a la signification donnée pour la formule II et $R'_6$ a la signification donnée pour la formule III,

que l'on fait réagir avec un halogénure de formule V :

$$Hal - R_3 \qquad (V)$$

dans laquelle Hal représente un atome d'halogéne et $R_3$ a la même signification que pour la formule I,

pour former le composé de formule I',

<u>et ensuite,</u>

- l'on soumet les composés de formule I', dans laquelle $R'_2$ représente un atome d'hydrogène,

* soit à l'action d'un halogène, pour former les composés de formule I dans laquelle $R_2$ représente un atome d'halogène, lesquels ensuite, lorsque $R_2$ représente un atome de brome, peuvent être soumis à l'action d'un autre halogène, pour former les composés de formule I dans laquelle $R_2$ représente cet atome d'halogène,
* soit à l'action de chlorure d'oxalyle pour préparer les composés de formule I dans laquelle $R_2$ représente un radical formyle, lesquels peuvent être ensuite soumis à une réduction, pour obtenir les composés de formule I dans laquelle $R_2$ représente un radical hydroxyméthyle,

ou

- l'on soumet les composés de formule I' dans laquelle $R'_6$ représente un radical $R_6$ contenant des fonctions avec des atomes d'azote réactifs ayant un groupe protecteur, à une hydrolyse acide, pour obtenir les composés de formule I, dans laquelle $R_6$ représente un radical contenant une amine primaire ou secondaire,

<u>et le cas échéant, les composés de formule I,</u>

sont ensuite séparés en leurs stéréoisomères possibles et/ou salifiés par un acide organique ou minéral pour former les sels correspondants.

Les composés de formule IV, intermédiaires utiles pour la préparation des composés de formule I, font aussi partie de l'invention.

Les dérivés de formule II peuvent être obtenus à partir des cétones correspondantes non halogénées de formule $R_1$-CO-$CH_2$-$R'_2$, soit par action du brome dans un solvant organique approprié, tel que l'acide acétique, le tétrachlorure de carbone ou l'éther éthylique, soit par action des tribromures d'ammonium quaternaires selon la méthode décrite dans Bull. Chem. Soc. Japan (1987), <u>60</u>, pp. 1159-1160 et pp. 2667-2668, soit encore par action du bromure cuivrique dans un solvant organique, tel qu'un mélange de chloroforme et d'acétate d'éthyle (J. Org. Chem. (1964), <u>29</u>, pp. 3451-3461).

Les cétones de formule $R_1$-CO-$CH_2$-$R'_2$, sont en général des produits connus et disponibles dans le commerce. Ces composés peuvent être préparés par la réaction de Friedel et Crafts, entre un composé de formule $R_1H$ et un halogénure d'acyle de formule $R'_2CH_2COHal$, de préférence un chlorure d'acyle de formule $R'_2CH_2COCl$, en présence d'un acide de Lewis.

Les composés de formule II, dans laquelle $R_1$ représente un radical de formule $A_1$ substitué en positions 2 et 4 par un atome d'halogène, et $R'_2$ représente un radical méthyle, peuvent être obtenus à partir des dérivés halogénés du benzène et notamment par les benzènes 1,3-dihalogénés, sur lesquels on fait réagir le bromure de 2-bromo propionyle en présence de chlorure d'aluminium.

Les composés de formule III et $III_A$, sont obtenus à partir des composés de formule VI :

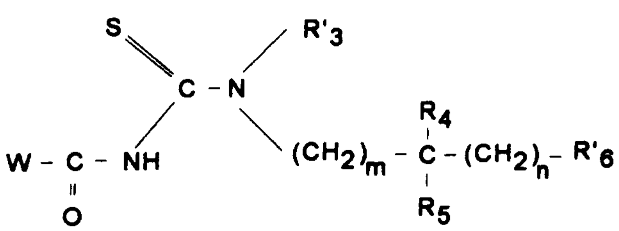

(VI)

dans laquelle $R'_3$ a la même signification que pour la formule I, ou représente un atome d'hydrogène, $R'_6$ à la signification donnée pour la formule III et W représente un radical phényle ou un radical *tert*-butyle,

soit par un traitement acide en utilisant de préférence l'acide chlorhydrique,

soit par un traitement basique en utilisant de préférence l'hydroxyde de sodium.

Quand W est un radical phényle, le traitement par un acide minéral est particulièrement utilisé lorsque $R'_6$ représente un radical pyridyle. On procède à un traitement basique lorsque $R_5$ est un groupement cycloalkyle, par exemple un cyclopropyle et lorsque $R_6$ est un radical imidazolyle, substitué au niveau de l'azote par un groupe protecteur résistant à l'hydrolyse en milieu alcalin.

Quand W représente un radical *tert*-butyle, les dérivés de thiourée de formule III et $III_A$, sont obtenus à partir des composés de formule VI par action d'un acide fort, par exemple de l'acide chlorhydrique concentré, à une température comprise entre 10°C et 100°C.

Les composés de formule VI sont obtenus en faisant réagir l'isothiocyanate de benzoyle ou l'isothiocyanate de pivaloyle sur les amines de formule VII.

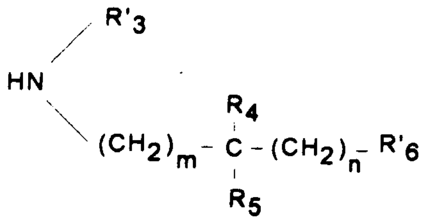

(VII)

dans laquelle,
$R_4$, $R_5$, m et n, ont la même signification que pour la formule I, $R'_3$ a la signification donnée pour la formule VI, et $R'_6$ a la signification donnée pour la formule III.

Les amines de formule VII, lorsqu'il s'agit d'amines secondaires, peuvent être préparées par des méthodes classiques.

Selon une première méthode on procède à l'alcoylation de l'amine primaire correspondante $VII_A$ :

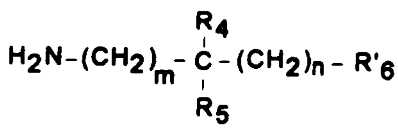

$(VII_A)$

par un halogénure d'alkyle Hal-$R_3$ de formule V, de préférence à chaud, en présence d'un sel alcalin dans un solvant organique polaire, par exemple le diméthylformamide.

Selon une autre méthode d'alcoylation les amines de formule $VII_A$ sont soumises à l'action d'un halogénure d'acide ou d'un anhydride d'acide dans un solvant organique choisi parmi les hydrocarbures halogénés, tel que le chlorure de méthylène, en présence d'un accepteur de protons, de préférence la triéthylamine. L'amide issue de cette réaction est ensuite réduite par des hydrures ($AlLiH_4$ ou autres) dans des solvants organiques de type éther.

Les deux méthodes citées ci-dessus sont utilisées de manière préférentielle pour la préparation des composés de formule VII, sous forme d'isomères purs.

Une autre méthode de préparation des composés de formule VII consiste à condenser une amine primaire de formule $R_3NH_2$ avec une cétone en milieu déshydratant, pour former l'imine correspondante laquelle ensuite est réduite de façon classique par un hydrure métallique, de préférence le borohydrure de sodium, ou par l'hydrogène en présence d'un catalyseur approprié. Lors de la réaction de l'amine primaire de formule $R_3NH_2$ avec une cétone en milieu déshydratant, on utilise de manière préférentielle, soit le chlorure de titane IV ($TiCl_4$), soit une catalyse par l'acide paratoluéne sulfonique.

Les amines de formule VII, dans laquelle m et n sont égal à zéro, sont préparées de manière préférentielle selon une méthode dont le principe est donné dans le schéma suivant :

**Etape A**

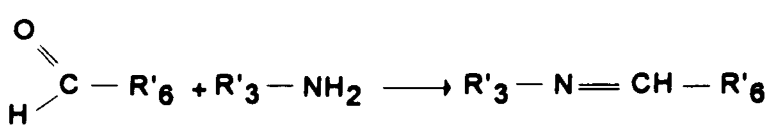

**Etape B**

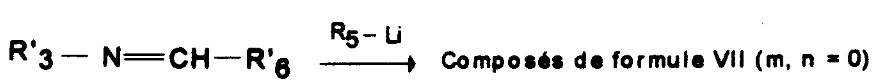

La condensation de l'aldéhyde avec l'amine primaire à l'étape A est réalisée de préférence dans l'éthanol ou dans le toluène, à température ambiante et la réaction de l'imine avec un dérivé d'alkyllithium à l'étape B, est réalisée dans l'éther éthylique ou le tétrahydrofurane à une température comprise entre 0°C et 15°C.

Comme il a déjà été mentionné ci-dessus, lorsque le substituant $R_6$ possède des fonctions réactives, ces fonctions doivent être protégées de façon classique. Par exemple quand $R_6$ représente un radical imidazolyle, son groupement réactif (NH) peut être bloqué par un groupe trityle. Après formation des dérivés de formule I, on procède à l'élimination du groupe protecteur à l'aide d'un acide minéral, par exemple l'acide chlorhydrique. Si on le désire, il est ensuite possible de préparer des dérivés substitués au niveau du radical imidazole. Pour ce faire, on fait réagir les composés de formule I dans laquelle $R_6$ représente un groupe imidazolyle avec par exemple un halogénure d'alkyle ou un halogénure d'aralkyle. La réaction est effectuée en présence d'un sel alcalin dans un solvant organique polaire par exemple le diméthylformamide, de préférence à chaud. On obtient ainsi des dérivés N-alkyl ou N-aralkyl imidazolyle.

L'alcoylation des composés de formule IV est effectuée en présence d'une base (hydrure de sodium, carbonate de césium, carbonate de potassium, etc...). Lorsque la réaction est effectuée en présence de carbonates alcalins, on utilise comme solvant, des solvants polaires par exemple le diméthylformamide. Quand l'alcoylation est effectuée en présence d'hydrures on utilise de manière préférentielle le tetrahydrofurane. On peut aussi utiliser des hydrocarbures aromatiques. Lorsque la réaction est effectuée en présence d'amidure de lithium, on utilise comme solvant de préférence le tétrahydrofurane. La réaction des composés de formule II avec les thiourées de formule III ou IIIa est effectuée en milieu organique en présence d'une base organique, par exemple la triéthylamine.

Pour la préparation des dérivés du thiazole de formule I, substitués en position 5 par un atome d'halogéne, à partir des composés de formule I non substitués en position 5, on opère à température ambiante en utilisant comme solvant un halogénure d'alkyle, et de préférence en présence d'un accepteur de protons.

Les composés de formule I, substitués en position 5 par un atome d'halogène peuvent être préparés à partir de leurs analogues, composés de formule I, substitués en position 5 par un atome de brome. Ces derniers composés sont soumis à l'action d'un agent d'halogénation après un échange halogène-métal.

Les dérivés du thiazole de formule I substitués en position 5 par un radical formyle sont obtenus à partir des dérivés correspondants non substitués en position 5 après réaction avec le chlorure d'oxalyle. La réaction est effectuée de préférence dans un solvant organique tel que le diméthylformamide. En soumettant les dérivés du thiazole de formule I substitués en position 5 par un radical formyle à l'action d'un agent réducteur tel que le borohydrure de sodium, on obtient les composés de formule I substitués en position 5 de l'hétérocycle thiazole avec un radical hydroxyméthyle. La réaction est effectuée dans un solvant alcoolique à une température d'environ 0°C-35°C.

Les composés de formule I dans laquelle $R_1$ représente un radical de formule $A_1$ ou $A_2$ substitué par au moins un radical hydroxy, sont obtenus à partir des composés de formule I dans laquelle $R_1$ représente un radical de formule $A_1$ ou de formule $A_2$ substitué par un ou plusieurs radicaux méthoxy. Pour ce faire, on soumet ces derniers produits à l'action d'un acide par exemple l'acide bromhydrique. Dans ce cas, la réaction est effectuée à chaud.

Les sels des composés de formule I avec des acides ou des bases pharmaceutiquement acceptables sont les sels préférés, mais ceux qui peuvent permettre d'isoler les composés de formule I, notamment de les purifier ou d'obtenir des isoméres purs, sont aussi objets de l'invention.

Parmi les acides pharmaceutiquement acceptables pour la préparation des sels d'addition aux composés de formule I, on peut citer les acides chlorhydrique, phosphorique, fumarique, citrique, oxalique, sulfurique, ascorbique, tartrique, maléique, mandélique, méthanesulfonique, lactobionique, gluconique, glucarique, hydroxyéthyl méthanesulfonique, succinyle sulfonique etc...

Les composés de la présente invention possèdent des propriétés pharmacologiques fort intéressantes. Les composés de l'invention déplacent notamment à des concentrations inférieures à 10 µM (0,01-10 µM) la liaison du $^{125}$I-CRF aux récepteurs spécifiques présents sur membranes de cortex de rat, selon la méthode décrite par De Souza E.B. (J. Neurosci (1987), 7 (1), pp. 88-100). Cela est étonnant et inattendu, puisque des composés de structure proche de celle des composés de l'invention, mais dont l'amine en position 2 du noyau thiazole ne comporte pas de substituant ramifié, ne déplacent pas de manière significative la liaison $^{125}$I-CRF.

En effet, le 2-[N-méthyl N-(pyrid-3-yl méthyl) amino] 4-(2,4,6-triméthylphényl) thiazole, composé décrit dans l'exemple 112 de la demande de brevet EP-0 283 390, ne provoque qu'un déplacement d'environ 8 %, à la concentration $10^{-5}$ M.

Le facteur de libération de l'hormone corticotrope (C.R.F.) est un neuropeptide qui contrôle l'activité de l'axe hypothalamo-hypophyso-surrénalien. Ce facteur est responsable des réponses endocrines et comportementales liées au stress.

En effet, il a été démontré que le CRF peut moduler le comportement comme aussi certaines fonctions du système nerveux autonome (G.F. Koob, F.E. Bloom, Fed. Proc. (1985), 44, p. 259 ; M.R. Brown, L.A. Fisher, Fed. Proc. (1985), 44, p. 243). Plus particulièrement, le CRF induit la sécrétion de la corticotropine (ACTH), de β-endorphine et autres peptides dérivés de la pro-opiomélanocortine (A. Tazi et al, Régul. Peptides (1987) 18, p. 37 ; M.R. Brown et al, Regul. Peptides (1986) 16, p. 321 ; C.L. Williams et al., Am. J. Physiol.. (1987), G 582, p. 253).

Les composés de l'invention peuvent être donc utiles à la régulation de la sécrétion de ces substances endogènes.

Ils trouvent plus spécialement leurs applications pour diminuer la réponse au stress (comportement, états émotionnels, troubles gastro-intestinaux et cardiovasculaires, désordres du système immunitaire) et plus généralement dans les pathologies impliquant le CRF, par exemple désordres psychiatriques, anxiété, anorexie nerveuse ou autres.

L'invention s'étend aussi aux compositions pharmaceutiques contenant comme principe actif au moins un composé de formule générale I, ou l'un de ses sels avec un acide minéral ou organique pharmaceutiquement compatible, en association avec un ou plusieurs excipients inertes et appropriés.

Les compositions pharmaceutiques ainsi obtenues sont présentées avantageusement sous des formes diverses telles que par exemple comprimés, dragées, gélules, suppositoires, solutions injectables ou buvables.

La posologie peut largement varier en fonction de l'âge, du poids du patient, de la nature et de la sévérité de l'affection ainsi que de la voie d'administration. D'une manière générale, la posologie unitaire s'échelonnera entre 0,5 mg et 200 mg et la posologie journalière utilisable en thérapeutique humaine, entre 0,5 mg et 800 mg.

La voie d'administration préférée est la voie orale ou parentérale.

Les exemples suivants illustrent l'invention.

Les points de fusion ont été mesurés selon la technique Micro-Köfler.

Les spectres de résonance magnétique nucléaire du proton (RMN-$^1$H) des composés de formule I ont été enregistrés, selon le cas, à 200 MHz à 100 MHz ou à 80 MHz.

Les composés de l'invention présentent une analyse centésimale conforme à la théorie.

**PREPARATIONS**

**PREPARATION DES COMPOSES DE FORMULE II**

PREPARATION I

**2-bromo 1-(2,4,6-triméthyl phényl) éthan-1-one** ***(Composé 1)***

Dissoudre 0,3 mole de 1-(2,4,6-triméthyl phényl) éthan-1-one dans 200 ml d'acide acétique glacial et ajouter goutte à goutte 31,8 g de brome, en maintenant le milieu réactionnel à une température inférieure à 10°C. En fin d'addition, laisser revenir le milieu réactionnel à température ambiante et abandonner à cette température pendant 2 heures. Verser ensuite le milieu réactionnel sur 500 ml d'eau glacée et extraire la phase aqueuse avec de l'éther éthylique. Laver les extraits organiques avec une solution aqueuse saturée en bicarbonate de sodium, puis avec de l'eau salée et sécher sur du sulfate de magnésium anhydre.

Après évaporation du solvant, on obtient une huile qui peut être utilisée sans autre purification.

**Autres composés** ***(Composés 2 à 13)***

Les composés suivants ont été obtenus selon la méthode décrite pour la préparation de 2-bromo-1-(2,4,6-triméthyl phényl) éthan-1-one, en utilisant comme matières premières, les cétones adéquates.

***Composé 2*** : 2-bromo 1-napht-2-yl propan-1-one
***Composé 3*** : 2-bromo 1-(2,4-diméthyl phényl)propan-1-one
***Composé 4*** : 2-bromo 1-(4-chloro 2-méthyl phényl) propan-1-one
***Composé 5*** : 2-bromo 1-(2-chloro 4-méthyl phényl) propan-1-one
***Composé 6*** : 2-bromo 1-(2-chloro 4-méthoxy phényl) propan-1-one
***Composé 7*** : 2-bromo 1-(2,4-diméthoxy phényl) propan-1-one
***Composé 8 :*** 2-bromo 1-(4-chloro phényl) propan-1-one
***Composé 9*** : 2-bromo 1-napht-1-yl propan-1-one
***Composé 10 :*** 2-bromo 1-(2,4-dichloro phényl) éthan-1-one
***Composé 11*** : 2-bromo 1-( 4-méthoxy phényl) propan-1-one
***Composé 12 :*** 2-bromo 1-(4-chloro 2-méthoxy phényl)propan-1-one
***Composé 13*** : 2-bromo 1-(4-méthyl phényl)propan-1-one

PREPARATION II

**2-bromo 1-(2,4,6-triméthoxy phényl) propan-1-one** ***(Composé 14)***

Porter au reflux une suspension de 45,3 g de bromure cuivrique dans 150 ml d'acétate d'éthyle et ajouter rapidement à cette température 25,1 g de 1-(2,4,6-trimethoxy phényl) propan-1-one en solution dans 150 ml de chloroforme. Il apparaît un abondant précipité jaune verdâtre.

Porter le milieu réactionnel au reflux pendant 2 heures et 30 minutes. Laisser ensuite revenir à température ambiante, filtrer les sels insolubles et laver avec de l'acétate d'éthyle.

Les phases organiques sont traitées avec du noir animal. Après élimination du solide par filtration, concentrer sous pression réduite pour obtenir une huile. Purifier par chromatographie sur colonne de silice en utilisant comme éluant un mélange de cyclohexane et d'acétate d'éthyle (6 : 4 V/V).
Huile.
Rendement : 60 %

PREPARATION III

**2-bromo 1-(2,4-dichloro phényl) propan-1-one** ***(Composé 15)***

A 7 g de 1-(2,4-dichloro phényl) propan-1-one en solution dans un mélange de 420 ml de chlorure de méthylène et de 140 ml de méthanol, ajouter 17,4 g de tribromure de *tert*-butyl ammonium à température ambiante.

Après 24 heures, évaporer à sec le milieu réactionnel, sous vide.

Reprendre à l'eau, extraire avec de l'acétate d'éthyle, sécher la phase organique par du sulfate de sodium.

Evaporer sous vide, puis purifier sur colonne de silice, en utilisant comme éluant un mélange de cyclohexane et d'acétate d'éthyle (20 : 1 V/V).
Huile.
Rendement : 78 %

De la même manière, peut aussi être obtenue la 2-bromo 1-(2-chloro 4-méthoxy phényl) propan-1-one ***(Composé 6)***.

PREPARATION IV

**2-bromo 1-(2,4-dibromo phényl) propan-1-one** ***(Composé 16)***

A 25 g de 1,3-dibromobenzène dans 250 ml de sulfure de carbone, ajouter avec précaution à 0°C, 15 g de chlorure d'aluminium puis couler lentement 22,86 g de bromure de 2-bromo propionyle.

Porter au reflux 8 heures puis évaporer sous vide le sulfure de carbone et verser le milieu réactionnel sur de la glace pilée.

Extraire à deux reprises à l'héptane, sécher, évaporer à sec puis purifier sur colonne de silice en utilisant comme éluant un mélange de cyclohexane et d'acétate d'éthyle (10 : 1 V/V), pour obtenir le produit attendu.
Rendement : 76 %

Le procédé décrit ci-dessus peut être utilisé pour préparer la 2-bromo 1-(2,4-dichlorophényl) propan-1-one ***(Composé 15)***, comme aussi la 2-bromo 1-(3,5-dichloro phényl) propan-1-one ***(Composé 17)***.

De la même manière, a été préparé la 2-bromo 1-(2-chloro 4-iodo phényl) propan-1-one ***(Composé 18)*** en utilisant comme produit de départ le 1-chloro 3-iodobenzène au lieu du 1,3-dibromobenzène.

**PREPARATION DES COMPOSES DE FORMULE VII**

PREPARATION V

**N-(α-cyclopropyl benzyl) propylamine** ***(Composé 19)***

A 10 g de cyclopropyl phényl cétone dans 60 ml de toluène anhydre, ajouter du tamis moléculaire 4 Å et 100 mg d'acide paratoluène sulfonique, puis 6 g de propylamine.

La formation d'imine est suivie par dosage en chromatographie phase gazeuse. Au bout de six jours de chauffage à 55°C, refroidir le mélange réactionnel, filtrer le tamis moléculaire et évaporer à sec sous vide.

Reprendre le résidu dans 100 ml d'éthanol anhydre. Refroidir à 0°C, ajouter par petites portions, 2,65 g de borohydrure de sodium. Après une nuit d'agitation à température ambiante, évaporer à sec sous vide, reprendre dans de l'eau, hydrolyser avec l'acide chlorhydrique N pour amener le pH à 2, laver avec de l'acétate d'éthyle. Amener à pH 9 par addition d'hydroxyde de sodium 2 N puis extraire à plusieurs reprises au chlorure de méthylène.

La phase organique après séchage et évaporation donne une huile qui peut être utilisée directement.
Rendement : 60 %

Spectre de résonance magnétique nucléaire du proton (solvant $CDCl_3$) :
0,15-1,7 ppm, m, 11H ; 2,4 ppm, t+d, 2H ; 2,80 ppm, d, 1H ; 7,3-7,4 ppm, m, 5H.

**Autres Composés** *(Composés 20 à 47)*

Les amines indiquées dans le tableau I sont obtenues selon le procédé décrit ci-dessus.

## TABLEAU I

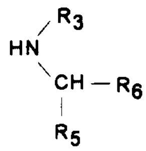

| Composés | R3 | R5 | R6 |
|---|---|---|---|
| 20 | $-C_3H_7$ | 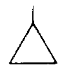 | 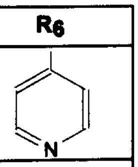 |
| 21 | $-C_3H_7$ | 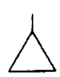 | 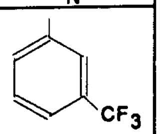 |
| 22 | $-C_3H_7$ | $-CH_3$ | 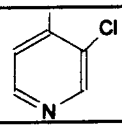 |
| 23 | $-C_3H_7$ | $-CH_3$ | 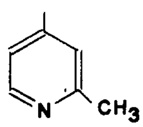 |
| 24 | $-C_3H_7$ | $-CH_3$ | 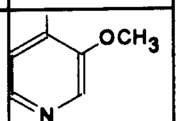 |
| 25 | $-C_3H_7$ | $-C_2H_5$ | 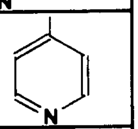 |
| 26 | $-C_3H_7$ | 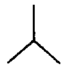 | 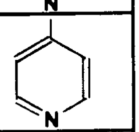 |
| 27 | $-C_3H_7$ | 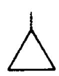 | 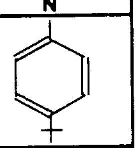 |
| 28 | $-C_3H_7$ | 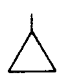 | 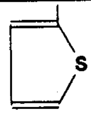 |
| 29 | $-C_3H_7$ | 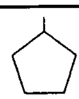 | 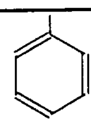 |

| Composés | R3 | R5 | R6 |
|---|---|---|---|
| 30 | $-C_3H_7$ | 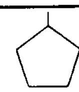 | 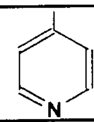 |
| 31 | $-C_3H_7$ | 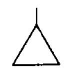 | 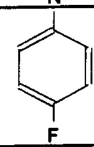 |
| 32 | $-C_3H_7$ | 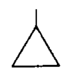 | 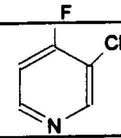 |
| 33 | $-C_3H_7$ | $-CH_3$ | 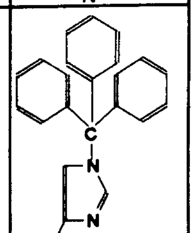 |
| 34 | $-C_3H_7$ | 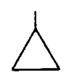 | 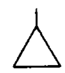 |
| 35 | $-C_3H_7$ | $-CH_3$ | 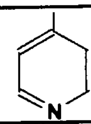 |
| 36 | $-C_3H_7$ | $-CH_3$ | 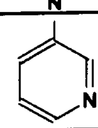 |
| 37 | 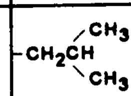 | $-CH_3$ | 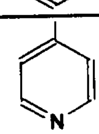 |
| 38 | $-CH_3$ | $-CH_3$ | 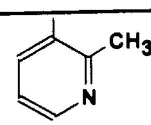 |
| 39 | $-C_2H_5$ | $-CH_3$ | 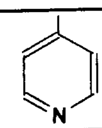 |

## TABLEAU I (suite 1)

| Composés | R3 | R5 | R6 |
|---|---|---|---|
| 40 | 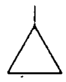 | $-CH_3$ | 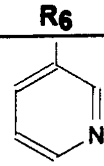 |
| 41 | $-C_3H_7$ | 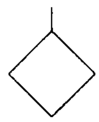 | 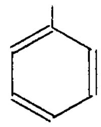 |
| 42 | $-C_3H_7$ | 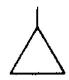 | 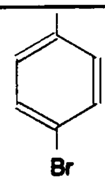 |
| 43 | $-C_3H_7$ | 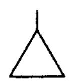 | 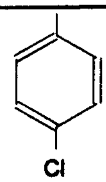 |
| 44 | $-C_3H_7$ | $-CH_3$ | 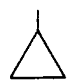 |
| 45 | $-C_3H_7$ | 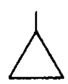 | 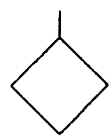 |
| 46 | $-C_3H_7$ | 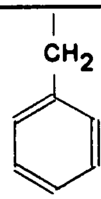 | 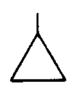 |
| 47 | $-C_3H_7$ | $-CH_3$ | 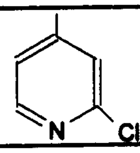 |

PREPARATION VI

**N-(cyclopropyl pyrid-4-yl méthyl) propylamine** *(Composé 20)*

La N-(cyclopropyl pyrid-4-yl méthyl) propylamine peut aussi être préparée de la façon suivante :

Etape A

Dissoudre 1,07 g de pyrid-4-yl carbaldéhyde dans 10 ml d'éthanol absolu et ajouter lentement 0,8 g de n-propylamine.

Après 30 minutes d'agitation, évaporer à sec pour obtenir 1,48 g d'huile.
Rendement : 99 %

Etape B

Dissoudre l'imine obtenue à l'étape précédente dans 10 ml d'éther isopropylique anhydre.

Ajouter cette solution sous agitation à 0°C dans 30 ml d'une solution de cyclopropyl lithium (20 mmol) dans l'éther isopropylique. Après deux heures d'agitation à température ambiante refroidir à 0°C et additionner goutte à goutte 3 ml de méthanol puis 10 ml d'une solution aqueuse de chlorure d'ammonium à 30 %.

Extraire la phase éthérée par l'acide chlorhydrique N.

Neutraliser la phase aqueuse acide par du bicarbonate de sodium puis extraire par l'acétate d'éthyle.

Sécher sur sulfate de sodium anhydre et évaporer à sec pour obtenir une huile incolore.
Rendement : 80 %

Spectre de résonance magnétique nucléaire du proton (solvant $CDCl_3$) :
0,28-1,76 ppm, m, 8H ; 0,88 ppm, t, 3H ; 1,48 ppm, m, 2H ; 2,31-2,49 ppm, m, 2H ; 2,78 ppm, d, 1H ; 7,35 ppm, dd, 2H ; 8,54 ppm, dd, 2H.

**Autres composés** ***(Composés 48 à 60)***

Les amines indiquées dans le tableau II, sont obtenues selon le procedé décrit ci-dessus.

## TABLEAU II

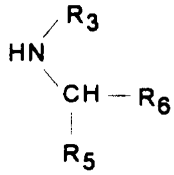

| Composés | R3 | R5 | R6 |
|---|---|---|---|
| 48 | $-C_3H_7$ | 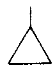 | 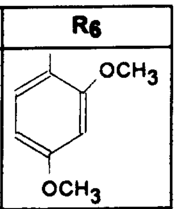 |
| 49 | $-C_3H_7$ | 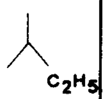 | 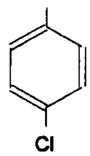 |
| 50 | $-C_3H_7$ | 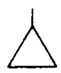 | 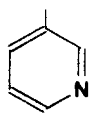 |
| 51 | $-C_3H_7$ | 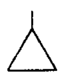 | 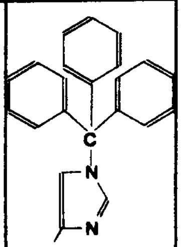 |
| 52 | $-C_3H_7$ | 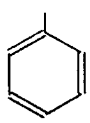 | 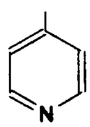 |
| 53 | $-C_3H_7$ | 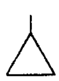 | 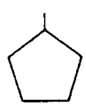 |

| Composés | R3 | R5 | R6 |
|---|---|---|---|
| 54 | $-C_3H_7$ | 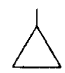 | 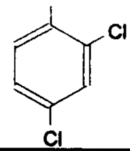 |
| 55 | $-C_3H_7$ | 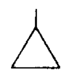 | 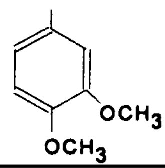 |
| 56 | $-C_3H_7$ | 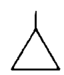 | 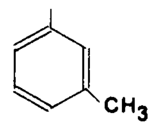 |
| 57 | 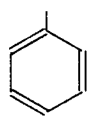 | 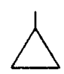 | 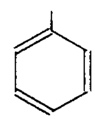 |
| 58 | $-C_3H_7$ | 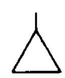 | 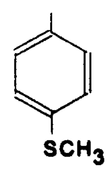 |
| 59 | $-C_3H_7$ | 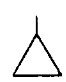 | 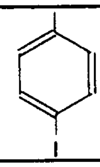 |
| 60 | $-C_3H_7$ | 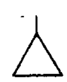 | 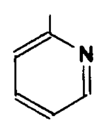 |

PREPARATION VII

**N-(cyclopropyl méthyl) 1-pyrid-4-yl éthylamine** *(Composé 61)*

Etape A

Dissoudre sous argon dans un tricol de 250 ml, 3,2 ml d'acide cyclopropane carboxylique dans 20 ml de chlorure de méthylène anhydre. Refroidir avec un bain de glace et ajouter 8,4 g de dicyclohexylcarbodiimide dissous dans 20 ml de chlorure de méthylène anhydre. Agiter pendant 30 minutes et ajouter 5 g de 1-pyrid-4-yl éthylamine. Après une nuit d'agitation à température ambiante, filtrer et éliminer les cristaux blancs. Evaporer le filtrat et purifier sur colonne de silice le résidu obtenu, en utilisant comme éluant un mélange d'acétate d'éthyle et d'hexane (1 : 9 V/V), pour obtenir une poudre blanche.
Rendement : 95 %

Etape B

Dans un tricol de 250 ml, muni d'une ampoule à brome et d'un réfrigérant, dissoudre 6 g d'amide obtenue à l'étape A dans 20 ml de tétrahydrofurane anhydre. Chauffer à reflux et ajouter 63 ml d'un complexe borane diméthylsulfure en solution (2 N) dans du tetrahydrofurane. Chauffer à reflux pendant 3 heures. Refroidir et ajouter goutte à goutte 20 ml d'une solution d'acide chlorhydrique 3 N. Chauffer à reflux pendant une heure. Alcaliniser avec de la lessive de soude et extraire à l'acétate d'éthyle. Sécher sur sulfate de sodium anhydre et évaporer à sec.
Rendement : 88 %

Spectre de résonance magnétique nucléaire du proton (solvant $CDCl_3$) :
0,02-0,07 ppm, m, 2H ; 0,37-0,50 ppm, m, 2H ; 0,87-0,94 ppm, m, 1H ; 1,33 ppm, d, 3H ; 2,29 ppm, dd, 2H ; 3,77 ppm, q, 1H ; 7,22-7,25 ppm, dd, 2H ; 8,53 ppm, d, 2H.

PREPARATION VIII

**N-propyl α-méthylbenzylamine** *(Composé 62)*

Introduire 23 ml de propylamine dans 200 ml de diméthylformamide, ajouter 32 g de carbonate de césium et 9,25 g de bromure de α-méthyl benzyle. Laisser 4 heures à température ambiante. Evaporer à sec, et reprendre à l'eau. Extraire par l'acétate d'éthyle, sécher sur sulfate de sodium anhydre et évaporer à sec pour obtenir le produit attendu.
Rendement : 95 %

Spectre de résonance magnétique nucléaire du proton (solvant $CDCl_3$) :
0,86 ppm, t, 3H ; 1,34 ppm, d, 3H ; 1,17-1,64 ppm, m, 2H ; 2,19-2,54 ppm, m, 2H ; 3,75 ppm, q, 1H ; 7,19-7,29 ppm, m, 5H.

PREPARATION IX

**N-(cyclopropyl pyrid-3-yl méthyl) propylamine** *(Composé 63)*

Etape A

A 3 g de pyrid-3-yl carbaldéhyde dans 70 ml d'éther éthylique anhydre, ajouter à -78°C sous agitation et goutte à goutte, 45 ml d'une solution de cyclopropyl lithium 0,625 M dans l'éther éthylique anhydre. Laisser revenir à température ambiante. Additionner d'eau saturée en chlorure d'ammonium et extraire à l'éther éthylique puis au chlorure de méthylène.
Rendement : 78 %

Etape B

Dissoudre 3,35 g de cyclopropyl pyrid-3-yl méthanol, composé obtenu à l'étape précédente, dans 75 ml de dioxane et ajouter 13,66 g de dioxyde de manganèse. Après 4 heures d'agitation sous reflux, filtrer le milieu réactionnel à chaud sur célite. La solution organique conduit après évaporation sous vide à un résidu qui est purifié par chromatographie sur colonne de silice, en utilisant comme éluant un mélange de cyclohexane et d'acétate d'éthyle (1:1 V/V), pour obtenir la cyclopropyl pyrid-3-yl cétone.

Rendement : 80 %

Spectre de résonance magnétique nucléaire du proton (solvant $CDCl_3$) :
1,06-1,34 ppm, m, 4H ; 2,68 m, 1H ; 7,42 ppm, m, 1H ; 8,23 ppm, m, 1H ; 8,78 ppm, m, 1H ; 9,26 ppm, m, 1H.

Etape C

Procéder comme il est décrit dans la préparation V en utilisant la cétone obtenue à l'étape précédente pour obtenir la N-(cyclopropyl pyrid-3-yl méthyl) propylamine.

PREPARATION X

**N-[cyclopropyl (2-méthyl pyrid-4-yl) méthyl] propylamine** ***(Composé 64)***

Etape A

A 7,3 g de 4-cyano-2-méthyl pyridine dans 100 ml de tetrahydrofurane anhydre, ajouter à -65°C 130 ml d'une solution de cyclopropyl lithium 0,7 M dans le tetrahydrofurane. Après 4 heures d'agitation ajouter du méthanol et une solution de sulfate d'ammonium (6,3 g dans 20 ml d'eau). Après extraction à l'éther éthylique, laver la phase organique à l'eau, sécher et évaporer sous vide. Le résidu est purifié sur colonne de silice en utilisant comme éluant un mélange de chlorure de méthyléne et de méthanol (98 : 2 V/V).

La cyclopropyl (2-méthyl pyrid-4-yl) cétone est ainsi obtenue.
Rendement : 59 %

Spectre de résonance magnétique nucléaire du proton (solvant $CDCl_3$) :
0,94-1,38 ppm, m, 4H ; 2,62 ppm, s, 3H ; 2,52-2,65 ppm, m, 1H ; 7,57 ppm, m, 2H ; 8,65 ppm, d, 1H.

Etape B

Procéder selon la méthode décrite dans la préparation V, en utilisant la cétone obtenue à l'étape précédente pour obtenir la N-[cyclopropyl (2-méthyl pyrid-4-yl) méthyl] propylamine.

PREPARATION XI

**N-(1,2-dicyclopropyl éthyl) propylamine** ***(Composé 65)***

A 2,2 g de cydopropylméthyl cyclopropylcétone dans 85 ml de chlorure de méthylène, ajouter 6,9 ml de propylamine. Additionner goutte à goutte à température ambiante 7 ml d'une solution à 1 M de chlorure de titane (IV) dans le chlorure de méthylène. Après 18 heures d'agitation ajouter 40 ml de méthanol anhydre. Refroidir à 0°C et additionner par petites quantités 1,0 g de borohydrure de sodium. Après une nuit d'agitation à température ambiante, ajouter 10 ml d'eau et évaporer sous vide. Reprendre dans de l'eau et extraire avec du chlorure de méthylène. Extraire par de l'acide chlorhydrique 2 N. Laver par de l'éther éthylique. Neutraliser la phase aqueuse avec une solution d'hydroxyde de sodium concentré. Extraire par le chlorure de méthylène, sécher sur sulfate de sodium anhydre et évaporer à sec pour obtenir une huile incolore.
Rendement : 76 %

Spectre de résonance magnétique nucléaire du proton (solvant $CDCl_3$) :
0,0-0,9 ppm, m, 13H ; 1,2-1,75 ppm, m, 5H ; 2,3-2,7 ppm, m, 2H.

**Autres Composés (Composés 66 à 70)**

Les amines indiquées dans le tableau III sont obtenues selon le procédé décrit dans la préparation XI. Le composé 70 a été préparé à partir de la cyclopropylméthyl phényl cétone. Ce dernier composé a été préparé à partir de cyclopropyl acétonitrile et du bromure de phényl magnésium.

## TABLEAU III

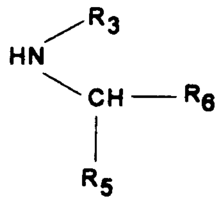

| Composés | $R_3$ | $R_5$ | $R_6$ |
|---|---|---|---|
| 66 | $-C_3H_7$ | 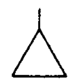 | 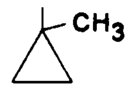 |
| 67 | $-C_3H_7$ | 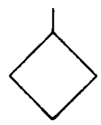 | 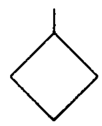 |
| 68 | $-C_3H_7$ | 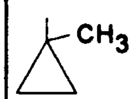 | 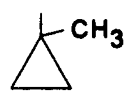 |
| 69 | 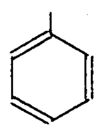 | 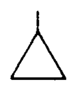 | 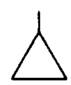 |
| 70 | $-C_3H_7$ | 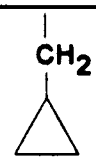 | 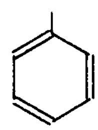 |

PREPARATION XII

**N-(2,2-dicyclopropyl éth-1-yl) propylamine** *(Composé 71)*

Ce composé a été préparé à partir de 2,2-dicyclopropyl acétaldéhyde, selon le procédé décrit dans la préparation V.

PREPARATION XIII

**N-(1-phényl pent-4-ène-1-yl) propylamine** *(Composé 72)*

Etape A

Agiter sous argon à -70°C, 0,48 ml de bromure de cyclopropyl méthyle en solution dans 20 ml d'éther éthylique, puis additionner 3 ml d'une solution de *tert*-butyl lithium (1,7 M dans le pentane).

Laisser sous agitation pendant 30 minutes.

Etape B

Procéder comme il est décrit dans la préparation VI, Etape A, en utilisant la benzaldéhyde au lieu de la pyrid-4-yl carbaldéhyde.

Etape C

Ajouter lentement dans la solution réactionnelle obtenue à l'Etape A, 730 mg de l'imine obtenue à l'Etape B en solution dans 2 ml d'éther éthylique.

Laisser la température remonter progressivement à 0°C puis additionner goutte à goutte une solution de chlorure d'ammonium.

La phase éthérée est séparée de la phase aqueuse et la phase aqueuse est extraite par l'éther éthylique. Les phases éthérées sont réunies et extraites par 2 fois 50 ml d'acide chlorhydrique 1 N.

Les phases aqueuses sont alcalinisées par une solution d'hydroxyde de sodium 1 N puis extraites par 3 fois 50 ml de chlorure de méthylène.

Les extraits organiques réunis sont lavés à l'eau et à l'eau saturée en chlorure de sodium, séchés sur sulfate de sodium anhydre puis évaporés à sec pour obtenir une huile.
Rendement : 81 %

Spectre de résonance magnétique nucléaire du proton (solvant $CDCl_3$) :
0,77-0,92 ppm, m, 3H ; 1,27-1,93 ppm, m, 6H ; 2,23-2,45 ppm, m, 2H ; 3,57 ppm, t, 1H ; 4,87-5,03 ppm, m, 2H ; 5,60-5,82 ppm, m, 1H ; 7,25-7,41 ppm, m, 5H.

PREPARATION XIV

**N-(α-*tert*-butyl benzyl) propylamine** ***(Composé 73)***

A 3 ml d'une solution de tert-butyl lithium (1 M dans le pentane), ajouter 20 ml d'éther éthylique et laisser sous agitation à -70°C pendant environ 30 minutes. Ajouter ensuite 730 mg de l'imine dont la préparation est décrite au stade B de la préparation XIII, en solution dans 2 ml d'éther éthylique.

Augmenter la température progressivement à 0°C puis hydrolyser avec une solution aqueuse de chlorure d'ammonium.

Séparer la phase éthérée et extraire la phase aqueuse par l'éther éthylique. Réunir les phases éthérées et extraire par 100 ml d'acide chlorhydrique.

Les phases aqueuses acides sont ensuite alcalinisées par une solution d'hydroxyde de sodium, puis extraites par le chlorure de méthylène.

L'extrait organique est ensuite lavé à l'eau puis à l'eau saturée en chlorure de sodium, séché sur sulfate de sodium anhydre et évaporé à sec pour obtenir une huile.
Rendement : 88 %

Spectre de résonance magnétique nucléaire du proton (solvant $CDCl_3$) :
0,84-1,0 ppm, m, 12H ; 1,36-1,55 ppm, m, 2H ; 2,29-2,46 ppm, m, 2H ; 3,30 ppm, s, 1H ; 7,19-7,35 ppm, m, 5H.

**PREPARATION DES COMPOSES DE FORMULE III**

**PREPARATION XV**

**N-(cyclopropyl pyrid-4-yl méthyl) N-propyl thiourée** ***(Composé 74)***

Etape A

N'-benzoyl N-(cyclopropyl pyrid-4-yl méthyl) N-propyl thiourée

Dissoudre 0,88 g de thiocyanate d'ammonium dans 6 ml d'acétone anhydre. Refroidir à 0°C et ajouter lentement 1,1 ml de chlorure de benzoyle dans 1 ml d'acétone. Porter au reflux 10 minutes puis ajouter 2 g de N-(cyclopropyl pyrid-4-yl méthyl) propylamine ***(Composé 20)*** en solution dans 10 ml d'acétone. Après 1 heure de chauffage, évaporer le solvant.

Reprendre le résidu dans de l'eau pour obtenir le composé attendu sous forme de cristaux blancs.
Rendement : 75 %

Point de fusion : 171°C

Etape B

A 2,5 g du composé obtenu dans l'étape précédente en solution dans 50 ml d'éthanol, ajouter 18 ml d'hydroxyde de sodium 1 N. Porter le milieu réactionnel pendant 48 heures à 80°C puis rajouter 10 ml d'hydroxyde de sodium 1N et porter à nouveau 24 heures à 80°C.

Après évaporation de l'éthanol sous vide, extraire la phase aqueuse résultante par du chlorure de méthylène.

Sécher la phase organique et évaporer sous vide.

Purifier le résidu par chromatographie sur colonne de silice, en utilisant comme éluant un mélange de chlorure de méthylène et de méthanol (98 : 2 V/V).

Rendement : 68 %

Point de fusion : Huile

Spectre de résonance magnétique nucléaire du proton (solvant $CDCl_3$) :

0,85 ppm, t, 3H ; 0,54-1,25 ppm, m, 8H ; 1,78 ppm, m, 2H ; 2,84-3,35 ppm, m, 2H ; 6,03 ppm, d, 1H ; 6,19 ppm, s, 2H ; 7,42 ppm, d, 2H ; 8,58 ppm, dd, 2H.

**Autres composés** ***(Composés 75 à 113)***

Les dérivés de thiourée indiqués dans le tableau IVa et IVb sont obtenus selon le procédé décrit pour la N-(cyclopropyl pyrid-4-yl méthyl) N-propyl thiourée en utilisant les amines adéquates, dont la préparation est indiquée précédemment (Préparation des composés de formule VII), ou en utilisant des amines qui sont disponibles dans le commerce.

## TABLEAU IVa

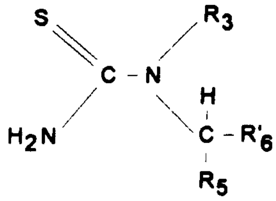

(III)

| Composés | $R_3$ | $R_5$ | $R'_6$ |
|---|---|---|---|
| 75 | $-C_3H_7$ | 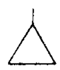 | 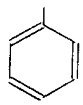 |
| 76 | $-C_3H_7$ | 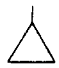 | 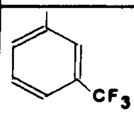 |
| 77 | $-C_3H_7$ | 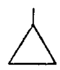 | 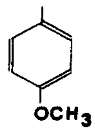 |
| 78 | $-C_3H_7$ | 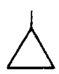 | 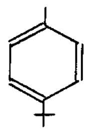 |
| 79 | $-C_3H_7$ | 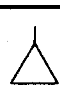 | 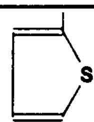 |
| 80 | $-C_3H_7$ | 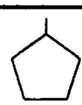 | 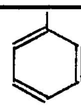 |
| 81 | $-C_3H_7$ | 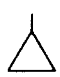 | 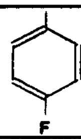 |
| 82 | $-C_3H_7$ | 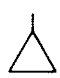 | 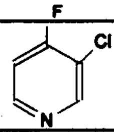 |
| 83 | $-C_3H_7$ | 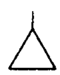 | 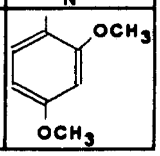 |
| 84 | $-C_3H_7$ | 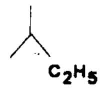 | 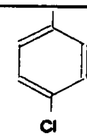 |
| 85 | $-C_3H_7$ | 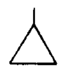 | 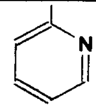 |
| 86 | $-C_3H_7$ | 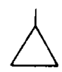 | 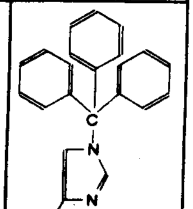 |
| 87 | $-C_3H_7$ | $-CH_3$ | 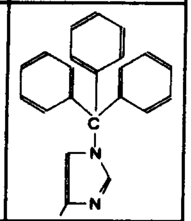 |
| 88 | $-C_3H_7$ | 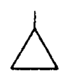 | 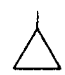 |
| 89 | $-C_3H_7$ | 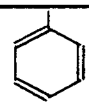 | 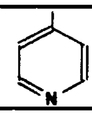 |
| 90 | $-C_3H_7$ | 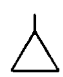 | 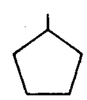 |
| 91 | $-C_3H_7$ | $-CH_3$ | 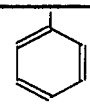 |
| 92 | H | $-CH_3$ | 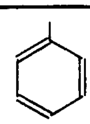 |

## TABLEAU IVa (suite 1)

| Composés | $R_3$ | $R_5$ | $R'_6$ |
|---|---|---|---|
| 93 | $-C_3H_7$ | 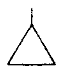 | 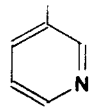 |
| 94 | $-C_3H_7$ | 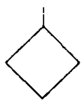 | 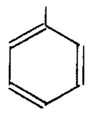 |
| 95 | $-C_3H_7$ | 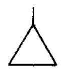 | 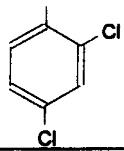 |
| 96 | $-C_3H_7$ | 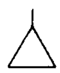 | 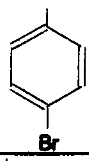 |
| 97 | $-C_3H_7$ | 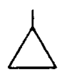 | 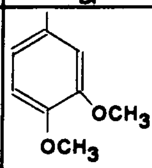 |
| 98 | $-C_3H_7$ | 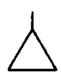 | 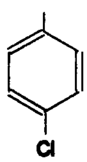 |
| 99 | $-C_3H_7$ | 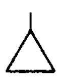 | 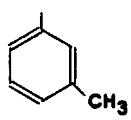 |

| Composés | $R_3$ | $R_5$ | $R'_6$ |
|---|---|---|---|
| 100 | $-C_3H_7$ | 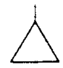 | 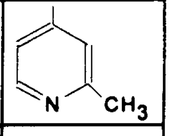 |
| 101 | $-C_3H_7$ | $-CH_3$ | 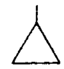 |
| 102 | $-C_3H_7$ | 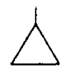 | 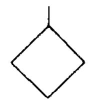 |
| 103 | $-C_3H_7$ | $-(CH_2)_2-CH=CH_2$ | 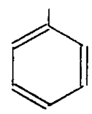 |
| 104 | $-C_3H_7$ | 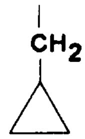 | 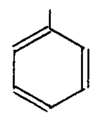 |
| 105 | $-C_3H_7$ | 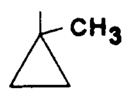 | 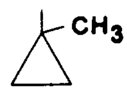 |
| 106 | $-C_3H_7$ | 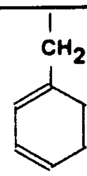 | 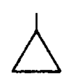 |

## TABLEAU IVa (suite 2)

| Composés | $R_3$ | $R_5$ | $R'_6$ |
|---|---|---|---|
| 107 | $-C_3H_7$ | 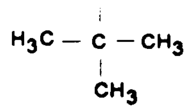 | 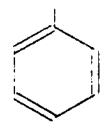 |
| 108 | $-C_3H_7$ | 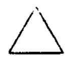 | 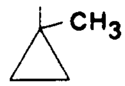 |
| 109 | $-C_3H_7$ | 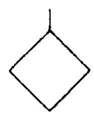 | 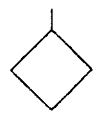 |
| 110 | $-C_3H_7$ | 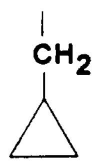 | 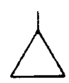 |
| 111 | 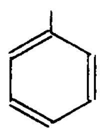 | 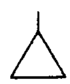 | 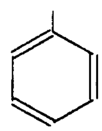 |
| 112 | 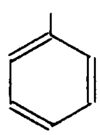 | 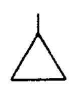 | 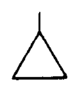 |
| 113 | $C_3H_7$ | 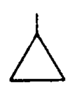 | 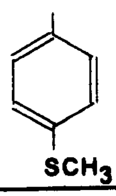 |
| 114 | $C_3H_7$ | 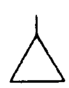 | 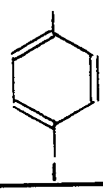 |

## TABLEAU IVb

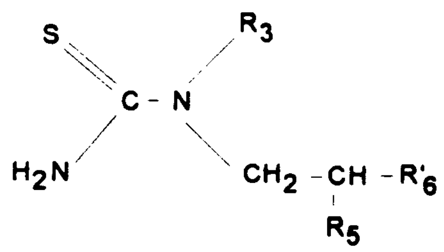

| ***Composés*** | **$R_3$** | **$R_5$** | **$R'_6$** |
|---|---|---|---|
| ***115*** | $-C_3H_7$ | 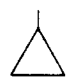 | 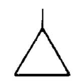 |

PREPARATION XVI

**N-[1-(3-chloro pyrid-4-yl) éth-1-yl] N-propyl thiourée** ***(Composé 116)***

Etape A

N'-benzoyl N-[1-(3-chloro pyrid-4-yl) éth-1-yl] N-propyl thiourée

Préparer ce composé selon le procédé indiqué à l'étape A de la préparation XV à partir de 1,14 g de thiocyanate d'ammonium, 1,74 ml de chlorure de benzoyle et 2,7 g de N-[1-(3-chloro pyrid-4-yl) éth-1-yl] propylamine ***(Composé 22)***.

Etape B

A 1,18 g du composé obtenu dans l'étape A, ajouter 6 ml d'acide chlorhydrique à 32 %. Porter le milieu réactionnel pendant une heure à 80°C puis refroidir et rajouter de l'eau. Extraire au chlorure de méthylène et éliminer la phase organique. Alcaliniser la phase aqueuse avec du carbonate de sodium et extraire au chlorure de méthylène. Sécher la phase organique et évaporer sous vide. Purifier le résidu par chromatographie sur colonne de silice, en utilisant comme éluant un mélange de chlorure de méthylène et de méthanol (98 : 2 V/V)
Rendement : 98 %
Point de fusion : Huile

Spectre de résonance magnétique nucléaire du proton (solvant $CDCl_3$) :
0,73 ppm, t, 3H ; 1,05-1,66 ppm, m, 2H ; 1,63 ppm, d, 3H ; 3,16 ppm, t, 2H ; 5,87 ppm, s, 2H ; 6,77 ppm, q, 1H ; 7,29 ppm, d, 1H ; 8,53 ppm, d, 1H ; 8,59 ppm, s, 1H.

**Autres composés** ***(Composés 117 à 121)***

Les dérivés de thiourée indiqués dans le tableau V, sont obtenus selon le procédé décrit pour la N-[1-(3-chloro pyrid-4-yl) éth-1-yl] N-propyl thiourée.

## TABLEAU V

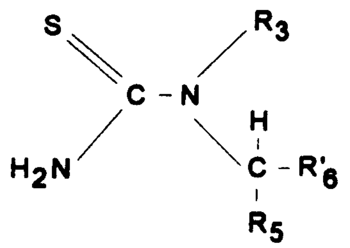

(III)

| Composés | $R_3$ | $R_5$ | $R'_6$ |
|---|---|---|---|
| **117** | $-C_3H_7$ | $-CH_3$ | 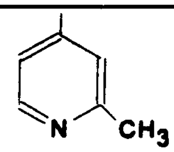 |
| **118** | $-C_3H_7$ | $-CH_3$ | 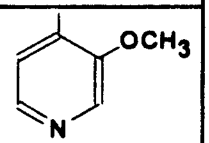 |
| **119** | $-C_3H_7$ | $-C_2H_5$ | 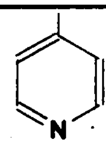 |
| **120** | $-C_3H_7$ | 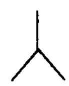 | 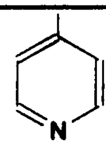 |
| **121** | $-C_3H_7$ | 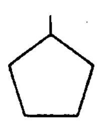 | 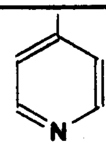 |

PREPARATION XVII

**N-cyclopropyl méthyl N-(1-pyrid-4-yl éth-1-yl) thiourée** *(Composé 122)*

Etape A

N'-pivaloyl N-cyclopropyl méthyl N-(1-pyrid-4-yl éth-1-yl) thiourée

Dans un tricol de 100 ml, dissoudre sous argon 2,9 g de thiocyanate de potassium dans 30 ml d'acétone. Refroidir à 0°C et ajouter goutte à goutte 3,4 ml de chlorure de pivaloyle. Agiter à 0°C pendant 5 heures puis ajouter 4,8 g de N-(cyclopropyl méthyl) 1-pyrid-4-yl éthylamine ***(Composé 61)***. Agiter à température ambiante pendant une nuit. Evaporer à sec. Le résidu est repris dans de l'eau et extrait dans du chlorure de méthylène. Sécher sur sulfate de sodium anhydre et évaporer à sec. La poudre obtenue est lavée à l'hexane et filtrée.

Rendement : 75 %

Spectre de résonance magnétique nucléaire du proton (solvant $CDCl_3$) :
0,02-0,07 ppm, m, 2H ; 0,4-0,5 ppm, m, 2H ; 0,96 ppm, m, 1H ; 1,28 ppm, s, 9H ; 1,77 ppm, d, 3H ; 2,90 ppm, dd, 2H ; 3,4 ppm, m, 1H ; 6,5 ppm, s, 1H ; 7,4 ppm, m, 2H ; 8,61 ppm, d, 2H.

Etape B

Dissoudre 2 g du produit obtenu à l'étape précédente dans 15 ml d'acide chlorhydrique à 32 % et chauffer à 70°C pendant une heure. Refroidir et alcaliniser avec une solution aqueuse de bicarbonate de sodium saturée. Extraire à l'acétate d'éthyle, sécher sur sulfate de sodium anhydre et évaporer à sec. Reprendre le résidu dans un mélange d'hexane et d'acétate d'éthyle (8 : 2 V/V).

Filtrer pour isoler des cristaux blancs.

Rendement : 87 %

**Autres composés** ***(Composés 123 à 129)***

Les thiourées indiquées dans le tableau VI ont été obtenues selon le procédé décrit dans la préparation XVII.

## TABLEAU VI

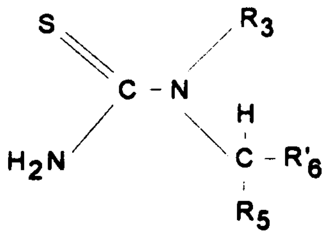

(III)

| Composés | $R_3$ | $R_5$ | $R'_6$ |
|---|---|---|---|
| 123 | $-C_3H_7$ | $-CH_3$ | 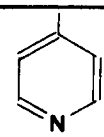 |
| 124 | $-C_3H_7$ | $-CH_3$ | 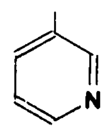 |
| 125 | 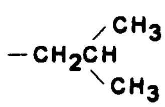 | $-CH_3$ | 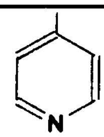 |
| 126 | $-CH_3$ | $-CH_3$ | 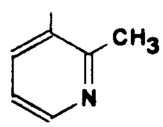 |
| 127 | $-C_2H_5$ | $-CH_3$ | 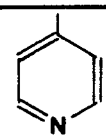 |
| 128 | 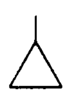 | $-CH_3$ | 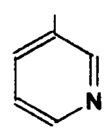 |
| 129 | $-C_3H_7$ | $-CH_3$ | 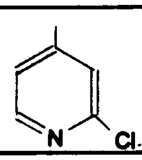 |

## EXEMPLES

### Exemple 1

**4-(2,4-dichloro phényl) 5-méthyl 2-[N-(α-cyclopropyl benzyl) N-propyl amino] thiazole**

A 1,35 g de N-(α-cyclopropyl benzyl) N-propyl thiourée ***(Composé 75)*** dans 35 ml d'éthanol ajouter 546 mg de triéthylamine, puis lentement 1,52 g de 2-bromo 1-(2,4-dichloro phényl) propan-1-one ***(Composé 15)***. Après 3 heures de chauffage à 75°C, éliminer par filtration le précipité formé. La solution éthanolique est évaporée à sec. Reprendre le résidu dans l'éther éthylique et laver par l'eau jusqu'à la disparition des ions brome. Sécher la phase organique sur sulfate de sodium anhydre puis évaporer à sec. Purifier le résidu sur colonne de silice en utilisant comme éluant un mélange de cyclohexane et de l'acétate d'éthyle (10 : 1 V/V), pour obtenir le produit attendu. Recristalliser dans l'acétonitrile (cristaux blancs).
Rendement : 79 %
Point de fusion : 78°C - 81 °C
Le spectre de résonance magnétique nucléaire du proton est indiqué dans le tableau VII.

### Exemple 2

**4-(2,4-dichloro phényl) 5-méthyl 2-[N-(cyclopropyl pyrid-4-yl méthyl) N-propyl amino] thiazole**

Ce composé a été préparé selon le procédé décrit dans l'exemple 1 à partir de 1,2 g de N-(cyclopropyl pyrid-4-yl méthyl) N-propyl thiourée ***(Composé 74)*** et de 1,52 g de 2-bromo 1-(2,4 dichloro phényl) propan-1-one ***(Composé 15)***. Le produit a été purifié sur colonne de silice en utilisant comme éluant un mélange de chlorure de méthylène et de méthanol (98 : 2 V/V).
Huile.
Rendement : 78 %

A partir de cette base, en utilisant les acides adéquats en solution dans l'éthanol, ont été obtenus les sels suivants :

- Hémifumarate : point de fusion : 98°C
- Chlorhydrate : point de fusion : 68°C
- Disulfate : point de fusion : 186°C

Le spectre de résonance magnétique nucléaire du proton de ce produit est indiqué dans le tableau VII.

### Exemples 3 à 40

Selon le procédé décrit dans l'exemple 1 ont été obtenus les composés des exemples 3-40 en utilisant les dérivés de bromocétones et de thiourées adéquats. Leurs caractéristiques spectrales sont indiquées dans le tableau VII.

### Exemple 41

**4-(2,4-dichloro phényl) 5-méthyl 2-[N-(α-méthyl benzyl) N-cyclopropyl méthyl amino] thiazole**

A 0°C, additionner 0,5 g de 4-(2,4-dichloro phényl) 5-méthyl 2-(N-α-méthyl benzyl amino) thiazole (préparé à partir du ***Composé 92*** et du ***Composé 15*** selon le procédé décrit dans l'exemple 1), en solution dans 5 ml de tétrahydrofurane anhydre, 66 mg d'hydrure de sodium. Après 30 minutes d'agitation à température ambiante, couler, goutte à goutte, 0,67 ml de bromure de cyclopropyl méthyle. Laisser huit heures au reflux, refroidir et diluer le milieu réactionnel par du chlorure de méthylène puis le verser sur de la glace.

Sécher et évaporer la phase organique pour obtenir une huile qui est ensuite purifiée sur colonne de silice en utilisant comme éluant un mélange de cyclohexane et d'acétate d'éthyle (40 : 1 V/V).
Rendement : 57 %
Le spectre de résonance magnétique nucléaire du proton du 4-(2,4-dichloro phényl) 5-méthyl 2-[N-(α-méthyl benzyl) N-cyclopropyl méthyl amino] thiazole est indiqué dans le tableau VII.

Exemple 42

**4-(2,4-dichloro phényl) 2-[N-(cyclopropyl imidazol-4-yl méthyl) N-propyl amino] 5-méthyl thiazole**

Etape A

4-(2,4-dichloro phényl) 5-méthyl 2-{N-[cyclopropyl (1-trityl imidazol-4-yl) méthyl] N-propyl amino} thiazole

Ce composé a été préparé à partir de N-[cyclopropyl (1-trityl imidazol-4-yl) méthyl] N-propyl thiourée ***(Composé 86)*** et de 2-bromo 1-(2,4-dichloro phényl) propan-1-one ***(Composé 15)*** selon le procédé décrit dans l'exemple 1.

Etape B

A 3 g du produit obtenu à l'étape précédente en solution dans 45 ml d'acétone, ajouter 45 ml d'acide chlorhydrique 2 N. Après une nuit à température ambiante, évaporer l'acétone, laver à l'éther éthylique la phase aqueuse restante, puis additionner du bicarbonate de sodium. Extraire le précipité formé par 3 fois 100 ml d'acétate d'éthyle. Laver la phase organique avec une solution aqueuse saturée en chlorure de sodium, puis sécher sur sulfate de sodium anhydre. Evaporer sous vide pour obtenir sous forme de poudre blanche le 4-(2,4-dichloro phényl) 5-méthyl 2-[N-(cyclopropyl imidazol-4-yl méthyl) N-propyl amino] thiazole.
Rendement : 90 %
Point de fusion : 83°C.
Le spectre de résonance magnétique nucléaire du proton de ce produit est indiqué dans le tableau VII.

Exemple 43

**4-(2,4-dichloro phényl) 5-méthyl 2-[N-(1-imidazol-4-yl éth-1-yl) N-propyl amino] thiazole**

Ce composé a été préparé selon le procédé décrit dans l'exemple 42 et en utilisant à l'étape A comme dérivé de thiourée, la N-[1-(1-trityl imidazol-4-yl) éth-1-yl] N-propyl thiourée ***(Composé 87).***
Le spectre de résonance magnétique nucléaire du proton de ce produit est indiqué dans le tableau VII.

Exemple 44

**4-(2,4-dichloro phényl) 5-méthyl 2-{N-[cyclopropyl (1-benzyl imidazol-4-yl) méthyl] N-propyl amino} thiazole**

A 492 mg du composé de l'exemple 42, en solution dans 10 ml de diméthylformamide, ajouter 320 mg de carbonate de potassium, puis à 0°C, ajouter lentement 0,12 ml de chlorure de benzyle en solution dans 1 ml de diméthylformamide. Laisser agir pendant 3 heures à 60°C environ, puis 3 heures à 80°C. Additionner de l'eau pour former un précipité. Extraire par l'acétate d'éthyle. Laver la phase organique avec une solution saturée en chlorure de sodium. Evaporer à sec et purifier le résidu par chromatographie sur colonne de silice en utilisant comme éluant un mélange de cyclohexane et d'acétate d'éthyle (2 : 1 V/V).
Rendement : 67 %

Ajouter une quantité adéquate d'acide chlorhydrique 0,1 N en solution dans l'isopropanol pour former le dichlorhydrate correspondant.
Point de fusion : 115°C
Le spectre de résonance magnétique nucléaire du proton 4-(2,4-dichloro phényl) 5-méthyl 2-{N-[cyclopropyl (1-benzyl imidazol-4-yl) méthyl] N-propyl amino} thiazole est indiqué dans le tableau VII.

Exemple 45

**4-(2,4-dichloro phényl) 5-méthyl 2-{N-[cyclopropyl (1-méthyl imidazol-4-yl) méthyl] N-propyl amino} thiazole**

A 856 mg du composé de l'exemple 42 en solution dans 10 ml d'acétone, ajouter 570 mg d'hydroxyde de postassium en poudre. Agiter pendant 5 minutes puis additionner 0,14 ml d'iodure de méthyle. Après 15 minutes à température ambiante, diluer le mélange réactionnel dans 100 ml de dichlorméthane puis laver à l'eau et à l'eau saturée en chlorure de sodium. Sécher la phase organique sur sulfate de sodium, évaporer à sec puis soumettre le résidu à une chromatographie sur colonne de silice en utilisant comme éluant un mélange de cyclohexane et d'acétate d'éthyl (1 : 1 V/V).

On sépare ainsi le 4-(2,4-dichloro phényl) 5-méthyl 2-{N-[cyclopropyl (1-méthyl imidazol-4-yl) méthyl] N-propyl

amino} thiazole et le 4-(2,4-dichloro phényl) 5-méthyl 2-{N-[cyclopropyl (3-méthyl imidazol-4-yl) méthyl] N-propyl amino} thiazole (37 : 63).
Rendement : 33 %

<u>Exemple 46</u>

**5-bromo 4-(2,4-dichloro phényl) 2-[N-propyl N-(1-pyrid-4-yl éth-1-yl) amino] thiazole**

Dissoudre 1 g de 4-(2,4-dichloro phényl) 2-[N-propyl N-(1-pyrid-4-yl éth-1-yl) amino] thiazole, composé de l'exemple 25, dans 20 ml de chlorure de méthylène. Ajouter 0,15 ml de brome. Agiter pendant une nuit. Evaporer à sec. Dissoudre le résidu dans un minimum d'isopropanol et précipiter à l'éther éthylique. Filtrer et laver à l'éther éthylique puis dissoudre dans une solution aqueuse de carbonate de potassium à 5 %. Extraire à l'acétate d'éthyle, sécher sur sulfate de magnésium anhydre et évaporer à sec pour obtenir le produit attendu sous forme d'huile.
Rendement : 80 %
Le spectre de résonance magnétique nucléaire du proton du 5-bromo 4-(2,4-dichloro phényl) 2-[N-propyl N-(1-pyrid-4-yl éth-1-yl) amino] thiazole est indiqué dans le tableau VII.

<u>Exemple 47</u>

**4-(2,4-dichloro phényl) 5-méthyl 2-[N-(dicyclopropyl méthyl) N-propyl amino] thiazole**

A 1 g de N-(dicyclopropyl méthyl) N-propyl thiourée ***(Composé 88)*** en solution dans 25 ml d'éthanol anhydre, ajouter 0,54 ml de triéthylamine, puis lentement 1 g de 2-bromo 1-(2,4-dichloro phényl) propan-1-one ***(Composé 15)***. Après deux heures de chauffage au reflux de l'éthanol, évaporer à sec. Reprendre le résidu dans du chlorure de méthylène, et laver avec de l'eau jusqu'à élimination totale des ions brome. Sécher la phase organique sur sulfate de sodium anhydre puis évaporer à sec. Purifier le résidu sur colonne de silice en utilisant comme éluant un mélange de cyclohexane et d'acétate d'éthyle (20 : 1 V/V), pour obtenir le produit attendu, sous forme d'huile.
Rendement : 88 %
Le spectre de résonance magnétique nucléaire du proton est indiqué dans le tableau VII.

Pour obtenir le sulfate correspondant, ajouter à cette base une quantité adéquate d'acide sulfurique 1 M dans l'éthanol.
Point de fusion : 140 °C.

<u>Exemple 48</u>

**4-(2,4-dichloro phényl) 5-méthyl 2-[N-(cyclopentyl cyclopropyl méthyl) N-propyl amino] thiazole**

Ce composé a été obtenu selon le procédé décrit dans l'exemple 47 mais en utilisant comme dérivé de thiourée la N-(cyclopentyl cyclopropyl méthyl) N-propyl thiourée ***(Composé 90).*** Lors de la purification sur colonne de silice, utiliser comme éluant un mélange de cyclohexane et d'acétate d'éthyle (10 : 1 V/V).
Rendement : 95 %
Le spectre de résonance magnétique nucléaire du proton est indiqué dans le tableau VII.

<u>Exemple 49</u>

**4-(2,4-dichloro phényl) 5-formyl 2-[N-propyl N-(1-pyrid-4-yl éth-1-yl) amino] thiazole**

Dans un ballon de 100 ml, introduire 3 ml de diméthylformamide. Refroidir à -30°C et ajouter goutte à goutte 0,45 ml de chlorure d'oxalyle. Agiter pendant 30 minutes à 0°C puis laisser augmenter la température. Ajouter alors 0,5 g du composé de l'exemple 25. Agiter pendant 6 heures. Laisser agir une nuit. Ajouter de l'eau et alcaliniser avec une solution d'hydroxyde de sodium 1 N. Filtrer le précipité formé pour obtenir une gomme. Dissoudre dans un minimum d'éther éthylique et ajouter de l'hexane jusqu'à obtention d'un léger trouble. Laisser précipiter, filtrer et laver à l'hexane pour obtenir le produit attendu sous forme de cristaux orange.
Rendement : 75 %
Point de fusion : 114°C
Le spectre de résonance magnétique nucléaire du proton 4-(2,4-dichloro phényl) 5-formyl 2-[N-propyl N-(1-pyrid-4-yl éth-1-yl) amino] thiazole est indiqué dans le tableau VII.

**Exemple 50**

**4-(2,4-dichloro phényl) 5-hydroxyméthyl 2-[N-propyl N-(1-pyrid-4-yl éth-1-yl) amino] thiazole**

Refroidir sur bain de glace une solution contenant 1,1 g du composé de l'exemple 49 dans 20 ml de méthanol anhydre et ajouter 0,2 g de borohydrure de sodium par petites quantités. Laisser réagir à température ambiante puis évaporer le solvant. Extraire le résidu à l'acétate d'éthyle. Sécher la phase organique sur sulfate de sodium anhydre. Evaporer à sec et dissoudre le résidu dans l'éther éthylique.

Précipiter à l'hexane pour obtenir le produit attendu sous forme de poudre orange.

Rendement : 72 %
Point de fusion 113°C
Le spectre de résonance magnétique nucléaire du proton de ce produit est indiqué dans le tableau VII.

**Exemple 51**

**4-(2,4-dichloro phényl) 5-méthyl 2-[N-propyl-2-ène N-(1-pyrid-4-yl éth-1-yl) amino] thiazole**

Dans un tricol de 100 ml, dissoudre sous argon 2,0 g de 4-(2,4-dichloro phényl) 5-méthyl 2-[N-(1-pyrid-4-yl éth-1-yl) amino] thiazole (préparé à partir de N-(1-pyrid-4-yl éth-1-yl) thiourée et de 2-bromo 1-(2,4-dichloro phényl) propan-1-one ***(Composé 15)***, selon le procédé décrit dans l'exemple 1), dans 20 ml de tétrahydrofurane anhydre.

Additionner 0,4 g d'amidure de lithium et agiter pendant 1 heure à 50°C. Ajouter 0,43 g de bromure d'allyle et chauffer à 60°C pendant 48 heures.

Evaporer à sec, puis ajouter 10 ml d'une solution aqueuse d'hydroxyde de sodium à 10 %. Extraire à l'acétate d'éthyle, sécher la phase organique sur sulfate de sodium anhydre et évaporer à sec. Purifier le résidu en utilisant comme éluant un mélange d'acétate d'éthyle et d'hexane (75 : 25 V/V), pour obtenir le produit attendu sous forme d'huile.

Rendement : 20 %
Le spectre de résonance magnétique nucléaire du proton est indiqué dans le tableau VII.

Pour obtenir l'oxalate de 4-(2,4-dichloro phényl) 5-méthyl 2-[N-propyl-2-ène N-(1-pyrid-4-yl éth-1-yl) amino] thiazole, dissoudre 0,4 g de base dans un minimum d'isopropanol et ajouter 0,18 g d'acide oxalique préalablement dissous dans l'isopropanol.

Evaporer à sec et recristalliser d'abord dans un mélange d'isopropanol et d'éther éthylique (50 : 50 V/V) puis dans l'isopropanol.

Point de fusion : 150°C.

**Exemple 52**

**4-(4-chloro 2-hydroxy phényl) 5-méthyl 2-[N-propyl N-(1-pyrid-4-yl éth-1-yl) amino] thiazole**

Porter à reflux pendant 24 heures 700 mg de 4-(4-chloro 2-méthoxy phényl) 5-méthyl 2-[N-propyl N-(1-pyrid-4-yl éth-1-yl) amino] thiazole, composé de l'exemple 22, en solution dans 30 ml d'acide bromhydrique concentré. Evaporer à sec et reprendre le résidu dans de l'eau saturée en carbonate de potassium. Extraire au chlorure de méthylène, puis évaporer le solvant organique. Purifier le résidu par chromatographie sur colonne de silice en utilisant comme éluant un mélange d'acétate d'éthyle et de méthanol (9 : 1 V/V), pour obtenir le produit attendu sous forme d'huile.

Rendement : 67 %
Le spectre de résonance magnétique nucléaire du proton du composé de cet exemple, est indiqué dans le tableau VII.

**Exemple 53**

**4-(2-chloro 4-hydroxy phényl) 5-méthyl 2-[N-propyl N-(1-pyrid-4-yl éth-1-yl) amino] thiazole**

Ce composé a été obtenu à partir du composé de l'exemple 27 selon le procédé décrit dans l'exemple 52.
Le spectre de résonance magnétique nucléaire du proton du 4-(2-chloro 4-hydroxy phényl) 5-méthyl 2-[N-propyl N-(1-pyrid-4-yl éth-1-yl) amino] thiazole, est indiqué dans le tableau VII.

**Exemple 54 à 88**

Selon le procédé décrit dans l'exemple 1, ont été obtenus les composés des exemples 54 à 87, en utilisant les dérivés de bromoacétones et des thiourées adéquates.

Leurs caractéristiques spectrales sont indiquées dans le tableau VII.

Exemple 89

**5-bromo 4-(2,4-dichloro phényl) 2-[N-(α-cyclopropyl benzyl) N-propyl amino] thiazole**

Ce composé a été préparé à partir du 4-(2,4-dichlorophényl) 2-[N-(α-cyclopropyl benzyl) N-propylamino] thiazole composé de l'exemple 82, et selon le procédé décrit dans l'exemple 46.
Le spectre de résonance magnétique nucléaire du proton est indiqué dans le tableau VII.

Exemple 90

**4-(2,4-dichloro phényl) 5-iodo 2-[N-(α-cyclopropyl benzyl) N-propyl amino] thiazole**

Dissoudre 496 mg de 5-bromo 4-(2,4-dichloro phényl) 2-[N-(α-cyclopropyl benzyl) N-propyl amino] thiazole, composé de l'exemple 89, dans 20 ml d'éther éthylique et refroidir cette solution à -70°C, puis ajouter lentement 0,8 ml d'une solution de *tert*-butyl lithium (1,5 N dans le pentane). Couler ensuite lentement 305 mg d'iode en solution dans 20 ml de tetrahydrofurane. Ramener lentement la température à 0°C et hydrolyser avec une solution aqueuse saturée en chlorure de sodium et laver avec une solution de thiosulfate.

Purifier comme il est indiqué dans l'exemple 1 pour obtenir le produit attendu.
Le spectre de résonance magnétique nucléaire du proton 4-(2,4-dichloro phényl) 5-iodo 2-[N-(α-cyclopropyl benzyl) N-propyl amino] thiazole est indiqué dans le tableau VII.

Exemple 91

**4-(2-chloro 4-iodo phényl) 5-méthyl 2-[N-(α-cyclopropyl benzyl) N-propyl amino] thiazole**

Ce composé a été préparé à partir du N-(α-cyclopropyl benzyl) N-propyl thiourée ***(Composé 75)*** et de 2-bromo 1-(2-chloro 4-iodo phényl) propan-1-one ***(Composé 18)*** selon le procédé décrit dans l'exemple 1.
Le spectre de résonance magnétique nucléaire du proton de ce composé est indiqué dans le tableau VII.

Exemple 92

**4-(2,4-dichloro phényl)-5-méthyl 2-[N-(2,2-dicyclopropyl éth-1-yl) N-propyl amino] thiazole**

Ce composé a été obtenu à partir de 2-bromo 1-(2,4-dichloro phényl) propan-1-one ***(Composé 15)*** et de N-(2,2-dicyclopropyl éth-1-yl) N-propyl thiourée ***(Composé 115)*** selon le procédé décrit dans l'exemple 1
Point de fusion : gomme

Spectre de résonance magnétique nucléaire du proton (solvant $CDCl_3$) :
0,0-0,7 ppm, m, 11H ; 0,9 ppm, m, 3H ; 1,4-1,9 ppm, m, 2H ; 2,10 ppm, s, 3H ; 3,3-3,5 ppm, m, 4H ; 7,1-7,4 ppm, m, 3H.

Exemple 93

**4-(2,4-dichloro phényl) 5-méthyl 2-[N-(α-cyclopropyl benzyl) N-propyl amino] thiazole - isomère (+) et 4-(2,4-dichloro phényl) 5-méthyl 2-[N-(α-cyclopropyl benzyl) N-propyl amino] thiazole - isomère (-)**

Les deux stéréoisomères du 4-(2,4-dichloro phényl) 5-méthyl 2-[N-(α-cyclopropyl benzyl) N-propyl amino] thiazole (composé de l'exemple 1), ont été obtenus selon deux méthodes.

METHODE A

Etape A

α-cyclopropyl benzylamine

Mettre sous agitation à 50°C pendant 4 jours sous argon, 100 g de cyclopropyl phényl cétone dans 2000 ml de méthanol avec 500 g d'acétate d'ammonium préalablement séché et 50 g de cyanoborohydrure de sodium en présence

de tamis moléculaire 4 Å. Après refroidissement, filtrer le tamis moléculaire puis additionner de l'acide chlorhydrique concentré pour amener le pH à 2. Evaporer la solution à sec sous vide et reprendre le résidu dans de l'eau. La phase aqueuse est lavée à l'éther éthylique puis alcalinisée par addition d'une solution d'hydroxyde de potassium concentré pour que le pH soit supérieur à 10. Extraire à deux reprises par du chlorure de méthylene, laver avec une solution saturée en chlorure de sodium, sécher sur sulfate de magnésium anhydre puis concentrer sous vide pour obtenir l'α-cyclopropyl benzylamine, qui est utilisée sans aucune autre purification à l'étape suivante.
Rendement : 76 %

Etape B

α-cyclopropyl benzylamine isomère (+) et α-cyclopropyl benzylamine - isomère (-)

A 275 ml d'éthanol absolu, ajouter 80,6 g d'acide L-tartrique-(+) et porter au reflux. Additionner alors goutte à goutte 79 g de l'α-cyclopropyl benzylamine obtenue à l'étape précédente. A la fin de l'addition, refroidir lentement le milieu réactionnel et obtenir ainsi des cristaux. Recristalliser ces cristaux à 5 reprises dans de l'éthanol absolu. La pureté optique de l'amine obtenue est suivie par chromatographie en phase gazeuse avec le réactif de Mosher. L'α-cyclopropyl benzylamine - isomère (+) est ainsi obtenue sous forme de sel tartrique avec une pureté optique supérieure à 96 %.
Rendement : 25 %
Point de fusion : 150°C
$[\alpha]_{365}^{20}$ = + 138,6° (C = 0,56 % dans le méthanol)

L'α-cyclopropyl benzylamine isomère (+) est obtenue à partir du sel après solubilisation dans l'eau, alcalinisation de la solution, extraction au chlorure de méthylène, séchage sur sulfate de magnésium anhydre et évaporation sous vide.
$[\alpha]_{365}^{20}$ = + 159,6° (C = 0,99 % dans le méthanol)

Réunir les solutions éthanoliques du précédent dédoublement et évaporer à sec. Reprendre le résidu dans l'eau, alcaliniser, extraire au chlorure de méthylène, sécher sur sulfate de magnésium anhydre et évaporer à sec. Salifier avec de l'acide D-tartrique-(-) en appliquant la méthode de salification et du dédoublement indiquée ci-dessus. Après 4 recristallisations dans l'éthanol, le sel tartrique de l'α-cyclopropyl benzylamine - isomére (-), est obtenu avec une pureté optique supérieure à 96 %.
Rendement : 20 %
Point de fusion : 151°C
$[\alpha]_{365}^{20}$ = - 141,9° (C = 0,94 % dans le méthanol)

Etape C

4-(2,4-dichloro phényl) 5-méthyl 2-(N-α-cyclopropyl benzyl amino) thiazole -isomère (-) et 4-(2,4-dichloro phényl) 5-méthyl 2-[(N-α-cyclopropyl benzyl) amino] thiazole -isomère (+)

Procéder comme il est décrit dans la préparation XV, en utilisant comme amine l'α-cyclopropyl benzylamine - isomère (+) pour obtenir la N-α-cyclopropyl benzyl thiourée - isomère (+). Faire réagir ensuite ce dernier composé avec le 2-bromo 1-(2,4-dichloro phényl) propan-1-one ***(Composé 15)*** selon le procédé décrit dans l'exemple 1 pour obtenir le 4-(2,4-dichloro phényl) 5-méthyl 2-(N-α-cyclopropyl benzyl amino) thiazole - isomère (-).
Rendement global : 62 %
$[\alpha]_{365}^{20}$ = - 72,8° (C = 0,82 % dans le méthanol)

Le 4-(2,4-dichloro phényl) 5-méthyl 2-[N-(α-cyclopropyl benzyl) amino] thiazole - isomère (+) a été préparé selon le procédé décrit ci-dessus en utilisant comme amine de départ l'α-cyclopropyl benzylamine - isomère (-).

Etape D

Dissoudre ensuite 1,18 g de 4-(2,4-dichloro phényl) 5-méthyl 2-(N-α-cyclopropyl benzyl amino) thiazole - isomère (-) dans 35 ml de diméthylformamide anhydre et ajouter à 0°C, 145 mg d'hydrure de sodium puis, après arrêt du dégagement gazeux, 370 mg de bromure de propyle. Chauffer 2 heures et 30 minutes à 75°C. Evaporer à sec, hydrolyser avec de l'eau. La phase aqueuse est extraite à l'acétate d'éthyle. La phase organique ainsi obtenue est lavée à l'eau saturée en chlorure de sodium, séchée sur sulfate de magnésium anhydre puis évaporée sous vide pour obtenir un résidu qui est purifié sur colonne de silice en utilisant comme éluant un mélange de cyclohexane et d'acétate d'éthyle (20 : 1 V/V).

Le 4-(2,4-dichloro phényl) 5-méthyl 2-[N-(α-cyclopropyl benzyl) N-propyl amino] thiazole - isomère (-) est obtenu sous forme d'un produit gommeux.

$[\alpha]^{20}_{365}$ = - 461,0° (C = 1,0 % dans le méthanol)

A partir de cette base, et en utilisant les acides adéquats en solution dans l'éthanol, ont été obtenus les sels suivants :

- Chlorhydrate : Point de fusion : 66,5°C
  $[\alpha]^{20}_{365}$ = - 452° (C = 0,62 % dans le méthanol)
  $[\alpha]^{20}_{D}$ = - 81,9° (C = 0,62 % dans le méthanol)
- Para toluènesulfonate : Point de fusion : 72°C

Le 4-(2,4-dichloro phényl) 5-méthyl 2-[N-(α-cyclopropyl benzyl) N-propyl amino] thiazole - isomère (+) a été préparé selon le procédé décrit ci-dessus en utilisant le 4-(2,4-dichloro phényl) 5-méthyl 2-[N-(α-cyclopropyl benzyl) amino] thiazole - isomère (+).

Le chlorhydrate correspondant a été préparé en faisant réagir le 4-(2,4-dichloro phényl) 5-méthyl 2-[N-(α-cyclopropyl benzyl) N-propyl amino] thiazole - isomère (+) avec une solution d'acide chlorhydrique dans le méthanol.

Point de fusion : 71°C

$[\alpha]^{20}_{365}$ = + 439,7 (C = 0,69 % dans le méthanol)

$[\alpha]^{20}_{D}$ = + 78,5° (C = 0,69 % dans le méthanol)

METHODE B

Etape A

α-cyclopropyl benzylamine - isomère (+) et α-cyclopropyl benzylamine -isomère (-)

Préparer la cyclopropyl phényl cétone oxime (E,Z) selon le procédé décrit dans Org. Synth. Coll. Vol II, p. 70, AH Blatt, J. Willey and Sons Inc. Edt, N.Y., London, Sydney, Copyright 1943.

Le mélange ainsi obtenu contient 76 % d'isomère E et 24 % d'isomère Z. Recristalliser plusieurs fois dans le méthanol ou isomériser en milieu acide selon le procédé ci-après.

Dissoudre 2 g de cyclopropyl phényl cétone oxime (E,Z) dans 20 ml d'éther éthylique anhydre et saturer avec de l'acide chlorhydrique gazeux. Filtrer le précipité obtenu, puis additionner 50 ml d'une solution aqueuse de $K_2CO_3$ à 10 %. Extraire le solide par du chlorure de méthyléne. Laver la phase organique à l'eau, sécher sur sulfate de magnésium anhydre et évaporer à sec pour obtenir la cyclopropyl phényl cétone oxime (E).

Pureté : 98 %

Ajouter 1,61 g de l'oxime ainsi obtenue en solution dans 10 ml de diméthylformamide anhydre à une solution contenant 276 mg d'hydrure de sodium dans 10 ml de diméthylformamide anhydre. Après une heure d'agitation à température ambiante, ajouter 0,75 ml d'iodure de méthyle. Après 4 heures d'agitation, verser le milieu réactionnel sur de la glace. Extraire à l'acétate d'éthyle, sécher, évaporer sous vide et purifier sur colonne de silice en utilisant comme éluant un mélange de cyclohexane et d'acétate d'éthyle (40:1 V/V) pour obtenir l'éther méthylique de la cyclopropyl phényl cétone oxime (E). Additionner ensuite à 385 mg de noréphédrine isomère (-) en solution dans 5 ml de tétrahydrofurane anhydre et à - 30°C, 8,6 ml du complexe borane-tetrahydrofurane 1M puis 300 mg de l'éther méthylique de la cyclopropyl phényl cétone oxime (E) en solution dans 3 ml de tetrahydrofurane anhydre. Porter au reflux pendant 2 heures et 30 minutes, puis à 0°C, additionner 10 ml d'eau et 10 ml d'hydroxyde de sodium à 20 %. Porter au reflux pendant 1 heure et 30 minutes. Après refroidissement extraire au chlorure de méthylène, sécher la phase organique et évaporer à sec. Reprendre le résidu dans un excès de méthanol et porter au reflux pendant 5 heures puis évaporer à sec et purifier sur colonne de silice en utilisant comme éluant un mélange de chlorure de méthylène et de méthanol (96 : 4 V/V).

On obtient ainsi l'α-cyclopropyl benzylamine -isomère (+) avec une pureté optique supérieure à 90 %.

Rendement : 40 %

L'α-cyclopropyl benzylamine isomère (-) est obtenue selon le procédé décrit ci-dessus mais en utilisant lors de la réduction l'éther méthylique de la cyclopropyl phényl cétone oxime-(E), la noréphédrine - isomère (+) comme agent chiral.

Etapes B et C

Procéder ensuite comme il décrit dans la méthode A, Etapes C et D, pour obtenir le 4-(2,4-dichloro phényl) 5-méthyl 2-[N-(α-cyclopropyl benzyl) N-propyl amino] thiazole - isomère (+) et le 4-(2,4-dichloro phényl) 5-méthyl 2-[N-(α-cyclopropyl benzyl) N-propyl amino] thiazole - isomère (-).

## TABLEAU VII

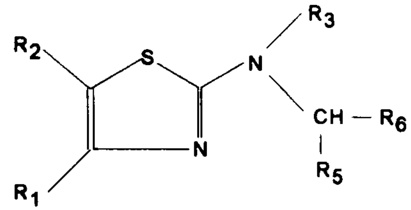

| Exemple | $R_1$ | $R_2$ | $R_3$ | $R_5$ | $R_6$ | Spectre RMN (solvant) |
|---|---|---|---|---|---|---|
| 1 | 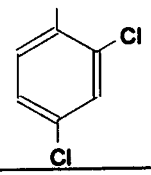 | $-CH_3$ | $-C_3H_7$ | 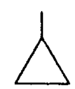 | 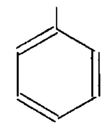 | **($CDCl_3$) Base** 0,40 ppm, m, 1H; 0,62 ppm, m, 2H ; 0,76 ppm, m, 4H ; 1,35 ppm, m, 1H ; 1,65 ppm, q, 2H ; 2,09 ppm, s, 3H ; 3,16 ppm, m, 2H ; 4,62 ppm, d, 1H ; 7,15-7,30 ppm, m, 5H ; 7,37-7,44 ppm, m, 3H. |
| 2 | 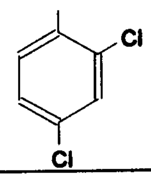 | $-CH_3$ | $-C_3H_7$ | 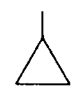 | 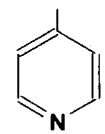 | **($CDCl_3$) Sel disulfate** 0,47-0,94 ppm, m, 4H ; 0,88 ppm, t, 3H ; 1,17 ppm, m, 1H ; 1,76 ppm, m, 2H ; 2,16 ppm, s, 3H ; 3,10-3,29 ppm, dd, 2H ; 4,74 ppm, d, 1H ; 7,35-7,45 ppm, m, 5H ; 8,54 ppm, d, 2H. |
| 3 | 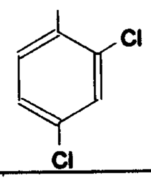 | $-CH_3$ | $-C_3H_7$ | 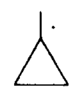 | 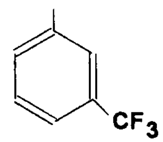 | **($CDCl_3$) Base** 0,40-1,10 ppm, m, 7H ; 1,5 ppm, m, 1H ; 1,85 ppm, m, 2H ; 2,20 ppm, s, 3H ; 3,3 ppm, m, 2H ; 4,97 ppm, d, 1H ; 7,10-8,00 ppm, m, 7H. |
| 4 | 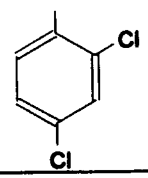 | $-CH_3$ | $-C_3H_7$ | $-CH_3$ | 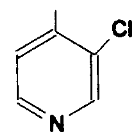 | **($CDCl_3$) Base** 0,8 ppm, t, 3H ; 1,26-1,74 ppm, m, 5H ; 2,15 ppm, s, 3H ; 3,17-3,33 ppm, m, 2H ; 5,51 ppm, q, 1H ; 7,25-7,45 ppm, m, 4H ; 8,44-8,58 ppm, m, 2H. |

## TABLEAU VI (Suite 1)

| EXEMPLE | $R_1$ | $R_2$ | $R_3$ | $R_5$ | $R_6$ | SPECTRE RMN (SOLVANT) |
|---|---|---|---|---|---|---|
| 5 | 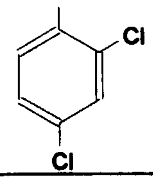 | $-CH_3$ | $-C_3H_7$ | $-CH_3$ | 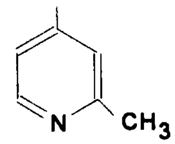 | ($CDCl_3$) Base<br>0,82 ppm, t, 3H ; 1,42-1,74 ppm, m, 5H ; 2,16 ppm, s, 3H ; 2,54 ppm, s, 3H ; 2,98-3,15 ppm, m, 2H ; 5,56 ppm, q, 1H ; 7,04-7,45 ppm, m 5H ; 8,43 ppm, d, 1H. |
| 6 | 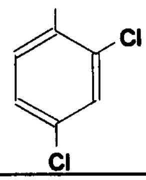 | $-CH_3$ | $-C_3H_7$ | $-CH_3$ | 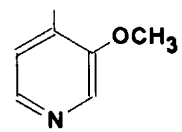 | ($CDCl_3$) Base<br>0,79 ppm, t, 3H ; 1,18-1,78 ppm, m, 5H ; 2,14 ppm, s, 3H ; 3,16-3,32 ppm, m, 2H ; 3,90 ppm, s, 3H ; 5,44 ppm, q, 1H ; 7,16-7,45 ppm, m, 4H ; 8,21-8,25 ppm, m, 2H. |
| 7 | 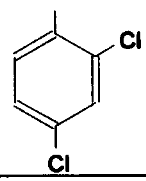 | $-CH_3$ | $-C_3H_7$ | $-C_2H_5$ | 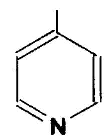 | ($CDCl_3$) Base<br>0,80 ppm, t, 3H ; 1,04 ppm, t, 3H ; 1,48-1,57 ppm, m, 1H ; 1,92-2,17 ppm, m, 6H ; 2,95-3,11 ppm, m, 2H ; 5,40 ppm, t, 1H ; 7,27-7,48 ppm, m, 5H ; 8,55 ppm, d, 2H. |
| 8 | 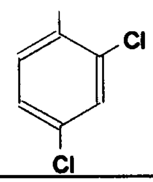 | $-CH_3$ | $-C_3H_7$ | 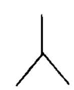 | 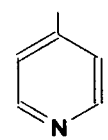 | ($CDCl_3$) Base<br>0,81 ppm, t, 3H ; 0,88 ppm, d, 3H ; 1,04-1,47 ppm, m, 3H ; 2,16 ppm, s, 3H ; 2,44-2,75 ppm, m, 1H ; 2,98-3,17 ppm, m, 2H ; 5,04 ppm, d, 1H ; 7,32-7,49 ppm, m, 5H ; 8,56, d, 2H. |
| 9 | 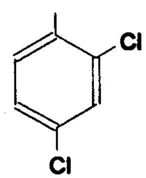 | $-CH_3$ | $-C_3H_7$ | 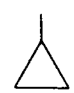 | 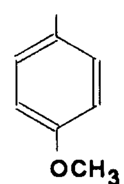 | ($CDCl_3$) Base<br>0,3-1,0 ppm, m, 7H ; 1,40 ppm, m, 1H ; 1,70 ppm, m, 2H ; 2,15 ppm, s, 3H ; 3,15 ppm, m, 2H ; 3,80 ppm, s, 3H ; 4,60 ppm, d, 1H ; 7,0-7,50, m, 7H. |

**TABLEAU VII (Suite 2)**

| EXEMPLE | $R_1$ | $R_2$ | $R_3$ | $R_5$ | $R_6$ | SPECTRE RMN (SOLVANT) |
|---|---|---|---|---|---|---|
| 10 | 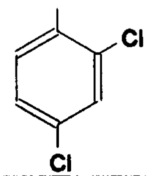 | $-CH_3$ | $-C_3H_7$ | 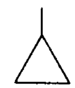 | 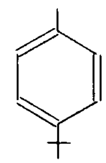 | ($CDCl_3$) Base<br>0,4-1,0 ppm, m, 7H ; 1,30 ppm, s, 9H ; 1,40 ppm, m, 1H ; 1,75 ppm, m, 2H ; 2,15 ppm, s, 3H ; 3,15 ppm, m, 2H ; 4,60 ppm, d, 1H ; 7,2-7,45, m, 7H. |
| 11 | 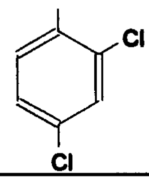 | $-CH_3$ | $-C_3H_7$ | 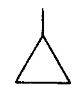 | 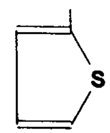 | ($CDCl_3$) Base<br>0,25-1,0 ppm, m, 7H ; 1,2-1,9 ppm, m, 3H ; 2,15 ppm, s, 3H ; 3,25 ppm, m, 2H ; 4,85 ppm, d, 1H ; 6,9-7,5 ppm, m, 6H. |
| 12 | 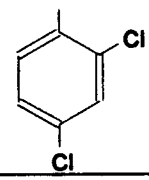 | $-CH_3$ | $-C_3H_7$ | 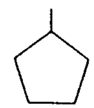 | 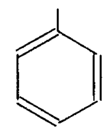 | ($CDCl_3$) Base<br>0,70 ppm, t, 3H ; 0,97-1,62 ppm, m, 10H ; 2,15 ppm, s, 3H ; 2,70-3,24 ppm, m, 3H ; 5,18 ppm, d, 1H ; 7,21-7,47 ppm, m, 8H. |
| 13 | 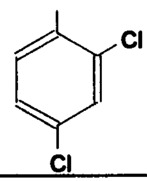 | $-CH_3$ | $-C_3H_7$ | 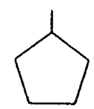 | 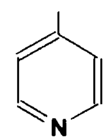 | ($CDCl_3$) Base<br>0,74 ppm, t, 3H ; 0,90-1,90 ppm, m, 10H ; 2,15 ppm, s, 3H ; 2,29-3,20 ppm, m, 3H ; 5,27 ppm, d, 1H ; 7,23-7,48 ppm, m, 5H ; 8,53 ppm, d, 2H. |
| 14 | 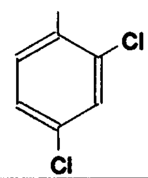 | $-CH_3$ | $-C_3H_7$ | 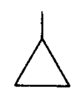 | 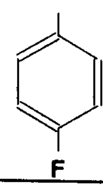 | ($CDCl_3$) Base<br>0,3-1,1 ppm, m, 7H ; 1,4 ppm, m, 1H ; 1,75 ppm, m, 2H ; 2,2 ppm, s, 3H ; 3,25 ppm, m, 2H ; 4,75 ppm, d, 1H ; 6,9-7,6 ppm, m, 7H. |

## TABLEAU VII (Suite 3)

| EXEMPLE | $R_1$ | $R_2$ | $R_3$ | $R_5$ | $R_6$ | SPECTRE RMN (SOLVANT) |
|---|---|---|---|---|---|---|
| 15 | 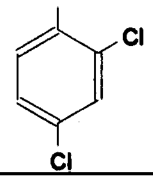 | $-CH_3$ | $-C_3H_7$ | 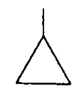 | 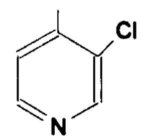 | ($CDCl_3$) Base<br>0,30-0,89 ppm, m, 7H ; 1,26-1,76 ppm, m, 3H ; 2,13 ppm, s, 3H ; 3,30 ppm, t, 2H ; 4,79 ppm, d, 1H ; 7,22-7,45 ppm, m, 3H ; 7,57 ppm, d, 1H ; 8,47 ppm, d, 1H ; 8,54 ppm, s, 1H. |
| 16 | 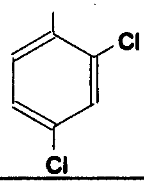 | $-CH_3$ | $-C_3H_7$ | 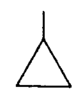 | 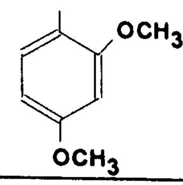 | ($CDCl_3$) Base<br>0,35-1,0 ppm, m, 7H ; 1,1-1,8 ppm, m, 3H ; 2,1 ppm, s, 3H ; 3,25 ppm, m, 2H ; 3,7 ppm, s, 3H ; 3,8 ppm, s, 3H ; 4,55 ppm, d, 1H ; 6,45 ppm, s, 1H ; 7,1-7,60 ppm, m, 5H. |
| 17 | 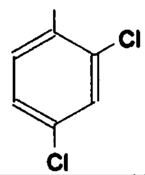 | $-CH_3$ | $-C_3H_7$ | 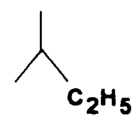 | 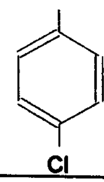 | ($CDCl_3$) Base<br>0,6-1,8 ppm, m, 14H ; 2,15 ppm, s, 3H ; 3,05 ppm, m, 2H ; 5,05, d, 1H ; 7,1-7,5 ppm, m, 7H . |
| 18 | 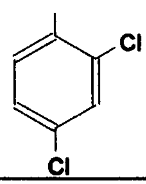 | $-CH_3$ | $-C_3H_7$ | 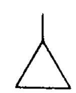 | 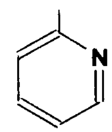 | ($CDCl_3$) Base<br>0,35-0,92 ppm, m, 4H ; 0,85 ppm, t, 3H ; 1,56-1,81 ppm, m, 3H ; 2,12 ppm, s, 3H ; 3,29-3,54 ppm, m, 2H ; 4,68 ppm d, 1H ; 7,10-7,68 ppm, m, 6H ; 8,58 ppm, d, 1H. |
| 19 | 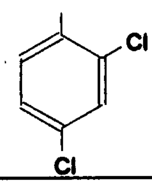 | $-CH_3$ | $-C_3H_7$ | 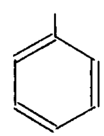 | 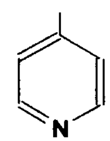 | ($CDCl_3$) Base<br>0,62 ppm, t, 3H ; 0,81-1,67 ppm, m, 2H ; 2,19 ppm, s, 3H ; 3,26 ppm, m, 2H ; 6,75 ppm, s, 1H ; 7,13-7,43 ppm, m, 10H ; 8,56 ppm, d, 2H. |

## TABLEAU VII (Suite 4)

| Exemple | $R_1$ | $R_2$ | $R_3$ | $R_5$ | $R_6$ | Spectre RMN (solvant) |
|---|---|---|---|---|---|---|
| 20 | 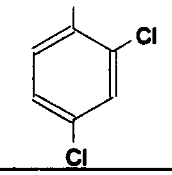 | $-CH_3$ | 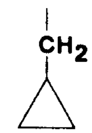 | $-CH_3$ | 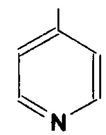 | **($CDCl_3$) Base** 0,00-0,20 ppm, m, 2H ; 0,42-0,52 ppm, m, 2H ; 1,03-1,10 ppm, m, 1H ; 1,67 ppm, d, 3H ; 3,10 ppm, dd, 2H ; 5,54 ppm, q, 1H ; 7,23-7,34 ppm, m, 4H ; 7,45 ppm, d, 1H ; 8,55 ppm, d, 1H. |
| 21 | 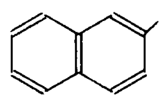 | $-CH_3$ | $-C_3H_7$ | $-CH_3$ | 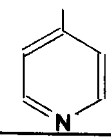 | **($CDCl_3$) Base** 0,87 ppm, t, 3H ; 1,55-1,75 ppm, m, 3H+2H ; 2,51 ppm, s, 3H ; 3,05-3,20 ppm, m, 2H ; 5,71 ppm, q, 1H ; 7,20-7,50 ppm, m, 4H ; 7,70-7,90 ppm, m, 4H ; 8,03 ppm, s, 1H ; 8,59 ppm, d, 2H. |
| 22 | 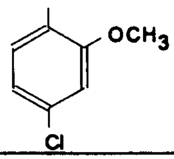 | $-CH_3$ | $-C_3H_7$ | $-CH_3$ | 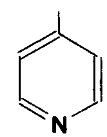 | **(DMSO-$d_6$) Sel oxalate** 0,89 ppm, t, 3H ; 1,50-1,70 ppm, m, 2H+3H ; 2,13 ppm, s, 3H ; 3,20-3,35 ppm, m, 2H ; 3,86 ppm, s, 3H ; 5,50 ppm, q, 1H ; 7,10 ppm, dd, 1H ; 7,21 ppm, d, 1H ; 7,33 ppm, d, 1H ; 7,45 ppm, d, 2H ; 8,63 ppm, d, 2H. |
| 23 | 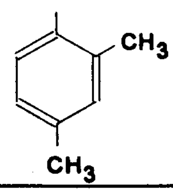 | $-CH_3$ | $-C_3H_7$ | $-CH_3$ | 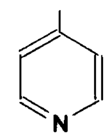 | **($CDCl_3$) Base** 0,83 ppm, t, 3H ; 1,50-1,70 ppm, m, 3H+2H ; 2,17 ppm, s, 3H ; 2,19 ppm, s, 3H ; 2,33 ppm, s, 3H ; 2,90-3,05 ppm, m, 2H ; 5,50-5,70 ppm, q, 1H ; 7,00-7,15 ppm, m, 2H ; 7,17 ppm, d, 1H ; 7,25 ppm, d, 2H ; 8,55 ppm, d, 2H. |
| 24 | 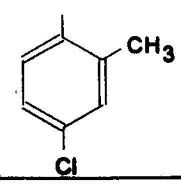 | $-CH_3$ | $-C_3H_7$ | $-CH_3$ | 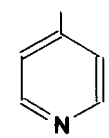 | **($CDCl_3$) Base** 0,84 ppm, t, 3H ; 1,5-1,7 ppm, m, 3H+2H ; 2,15 ppm, s, 3H ; 2,19 ppm, s, 3H ; 3,0-3,3 ppm, m, 2H ; 5,60 ppm, q, 1H ; 7,1-7,4 ppm, m, 5H ; 8,55 ppm, d, 2H. |

## TABLEAU VII (Suite 5)

| EXEMPLE | $R_1$ | $R_2$ | $R_3$ | $R_5$ | $R_6$ | SPECTRE RMN (SOLVANT) |
|---|---|---|---|---|---|---|
| 25 | 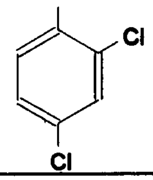 | H | $-C_3H_7$ | $-CH_3$ | 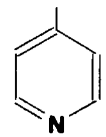 | ($CDCl_3$) Base 0,87 ppm, t, 3H ; 1,59-1,71 ppm, m, 3H+2H ; 3,10-3,23 ppm, m, 2H ; 5,65 ppm, q, 1H ; 7,14 ppm, s, 1H ; 7,23-7,30 ppm, m, 3H ; 7,43 ppm, d, 1H ; 7,88 ppm, d, 1H ; 8,57 ppm, d, 2H. |
| 26 | 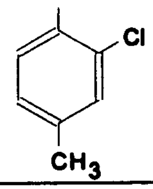 | $-CH_3$ | $-C_3H_7$ | $-CH_3$ | 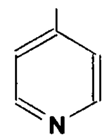 | ($CDCl_3$) Base 0,83 ppm, t, 3H ; 1,50-1,70 ppm, m, 3H+2H ; 2,17 ppm, s, 3H ; 2,35 ppm, s, 3H ; 3,0-3,3 ppm, m, 2H ; 5,7 ppm, q, 1H ; 7,09 ppm, d, 1H ; 7,35-7,40 ppm, m, 4H ; 8,55 ppm, d, 2H. |
| 27 | 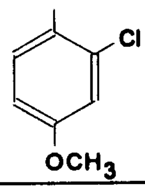 | $-CH_3$ | $-C_3H_7$ | $-CH_3$ | 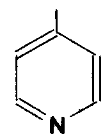 | ($CDCl_3$) Base 0,83 ppm, t, 3H ; 1,50-1,76 ppm, m, 3H+2H ; 2,17 ppm, s, 3H ; 3,0-3,1 ppm, m, 2H ; 3,81 ppm, s, 3H ; 5,68 ppm, q, 1H ; 6,64 ppm, dd, 1H ; 6,98 ppm, d, 1H ; 7,20-7,30 ppm, m, 3H ; 8,57 ppm, m, 2H. |
| 28 | 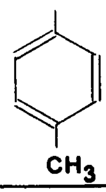 | $-CH_3$ | $-C_3H_7$ | $-CH_3$ | 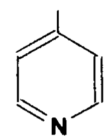 | ($CDCl_3$) Base 0,91 ppm, t, 3H ; 1,61-1,67 ppm, m, 3H+2H ; 2,37 ppm, s, 3H ; 2,42 ppm, s, 3H ; 3,03-3,12 ppm, m, 2H ; 5,66 ppm, q, 1H ; 7,20 ppm, d, 2H ; 7,28 ppm, d, 2H ; 7,50 ppm, d, 2H ; 7,56 ppm, d, 2H. |
| 29 | 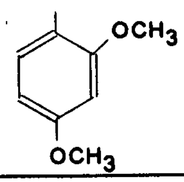 | $-CH_3$ | $-C_3H_7$ | $-CH_3$ | 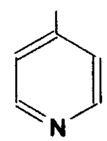 | ($CDCl_3$) Base 0,83 ppm, t, 3H ; 1,58-1,75 ppm, m, 3H+2H ; 2,15 ppm, s, 3H ; 3,23-3,45 ppm, m, 2H ; 3,90 ppm, s, 3H ; 3,99 ppm, s, 3H ; 5,55 ppm, q, 1H ; 6,52 ppm, 2s, 2H ; 7,27 ppm, d, 2H ; 7,57 ppm, s, 1H ; 8,56 ppm, d, 2H. |

## TABLEAU VII (Suite 6)

| EXEMPLE | R1 | R2 | R3 | R5 | R6 | SPECTRE RMN (SOLVANT) |
|---|---|---|---|---|---|---|
| 30 | 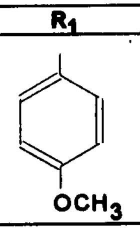 | $-CH_3$ | $-C_3H_7$ | $-CH_3$ | 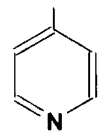 | **($CDCl_3$) Base** 0,85 ppm, t, 3H ; 1,50-1,60 ppm, m, 5H ; 2,41 ppm, s, 3H ; 3,08 ppm, m, 2H ; 3,83 ppm, s, 3H ; 5,67 ppm, q, 1H ; 7,25 ppm, m, 4H ; 7,90 ppm, m, 4H. |
| 31 | 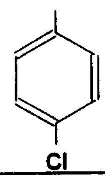 | $-CH_3$ | $-C_3H_7$ | $-CH_3$ | 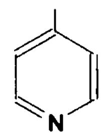 | **($CDCl_3$) Base** 0,85 ppm, t, 3H ; 1,56-1,66 ppm, m, 3H+2H ; 2,41 ppm, s, 3H ; 3,04-3,17 ppm, m, 2H ; 5,62 ppm, q, 1H ; 7,27-7,38 ppm, m, 4H ; 7,55 ppm, d, 2H ; 8,56 ppm, d, 2H. |
| 32 | 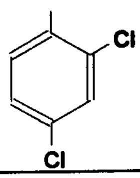 | $-CH_3$ | $-C_3H_7$ | $-CH_3$ | 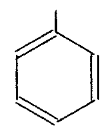 | **($CDCl_3$) Base** 0,77 ppm, t, 3H ; 1,22-1,66 ppm, m, 5H ; 2,16 ppm, s, 3H ; 2,90-3,14 ppm, m, 2H ; 5,57 ppm, q, 1H ; 7,20-7,47 ppm, m, 8H. |
| 33 | 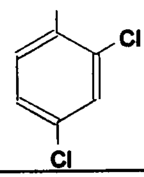 | $-CH_3$ | $-C_3H_7$ | $-CH_3$ | 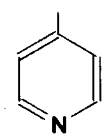 | **(DMSO-$d_6$) Sel oxalate** 0,92 ppm, t, 3H ; 1,72 ppm, m, 2H ; 1,74 ppm, d, 3H ; 2,16 ppm, s, 3H ; 3,22 ppm, t, 2H ; 5,68 ppm, q, 1H ; 7,23 ppm, m, 2H ; 7,41 ppm, s, 1H ; 7,77 ppm, d, 2H ; 8,79 ppm, d, 2H. |
| 34 | 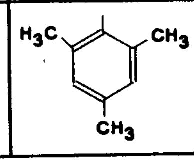 | H | $-C_3H_7$ | $-CH_3$ | 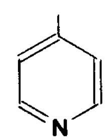 | **(DMSO-$d_6$) Sel fumarate** 0,83 ppm, t, 3H ; 1,63 ppm, m, 5H ; 1,99 ppm, s, 6H ; 2,24 ppm,s, 3H ; 3,25 ppm, t, 2H ; 5,36 ppm, q, 1H ; 6,51 ppm, s, 1H ; 6,64 ppm, s, 2H ; (acide fumarique) ; 6,84 ppm, s, 2H ; 7,30 ppm, d, 2H ; 8,52 ppm, d, 2H. |

## TABLEAU VII (Suite 7)

| EXEMPLE | R1 | R2 | R3 | R5 | R6 | SPECTRE RMN (SOLVANT) |
|---|---|---|---|---|---|---|
| 35 | 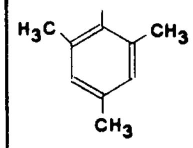 | H | $-C_3H_7$ | $-CH_3$ | 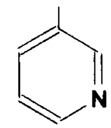 | ($DMSO-d_6$) Base<br>1,0 ppm, t, 3H ; 1,6 ppm, m, 2H ; 1,8 ppm, d, 3H ; 2,2 ppm, s, 6H ; 2,4 ppm, s, 3H ; 3,4 ppm, m, 2H ; 5,5 ppm, q, 1H ; 6,6 ppm, s, 1H ; 7,0 ppm, s, 2H ; 7,5 ppm, m, 1H ; 7,9 ppm, m, 1H ; 8,65 ppm, m, 2H. |
| 36 | 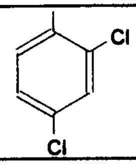 | $-CH_3$ | — $CH_2$ — 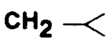 | $-CH_3$ | 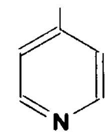 | ($DMSO-d_6$) Sel oxalate<br>0,84 ppm, m, 6H ; 1,64 ppm, d, 3H ; 2,0 ppm, m, 1H ; 2,11 ppm, s, 3H ; 3,12 ppm, t, 2H ; 5,29 ppm, q, 1H ; 7,42 ppm, m, 4H ; 7,68 ppm, s, 1H ; 8,56 ppm, m, 2H. |
| 37 | 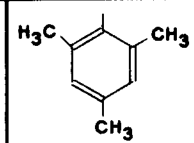 | H | $-CH_3$ | $-CH_3$ | 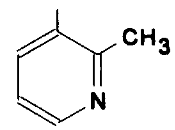 | ($DMSO-d_6$) Base<br>1,50 ppm, d, 3H ; 2,1 ppm, s, 6H ; 2,3 ppm, s, 3H ; 2,43 ppm, s, 3H ; 2,7 ppm, s, 3H ; 5,7 ppm, q, 1H ; 6,55 ppm, s, 1H ; 6,9 ppm, s, 2H ; 7,3 ppm, m, 1H ; 7,85 ppm, d, 1H ; 8,42 ppm, d, 1H. |
| 38 | 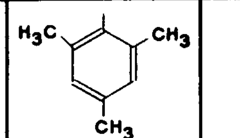 | H | $-C_2H_5$ | $-CH_3$ | 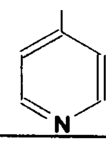 | ($DMSO-d_6$) Base<br>1,20 ppm, t, 3H ; 1,75 ppm, d, 3H ; 2,15 ppm, s, 6H ; 2,3 ppn., s, 3H ; 3,5 ppm, m, 2H ; 5,5 ppm, q, 2H ; 6,65 ppm, s, 1H ; 7,0 ppm, s, 2H ; 7,4 ppm, m, 2H ; 8,6 ppm, m, 2H. |
| 39 | 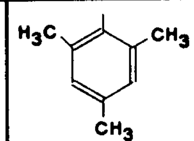 | H | 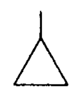 | $-CH_3$ | 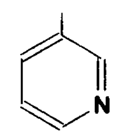 | ($DMSO-d_6$) Sel chlorhydrate<br>1,10 ppm, m, 4H ; 1,90 ppm, d, 3H ; 2,00 ppm, s, 6H ; 2,3 ppm, s, 3H ; 3,0 ppm, m, 1H ; 5,6 ppm, q, 1H ; 6,90 ppm, s, 1H ; 7,0 ppm, s, 2H ; 8,2 ppm, m, 1H ; 8,6 ppm, m, 1H ; 8,9 ppm, m, 2H. |
| 40 | 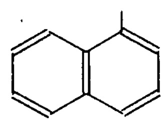 | $-CH_3$ | $-C_3H_7$ | $-CH_3$ | 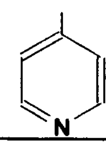 | ($CDCl_3$) Base<br>0,88 ppm, t, 3H ; 1,50-1,75 ppm, m, 5H ; 2,19 ppm, s, 3H ; 3,10-3,25 ppm, m, 2H ; 5,87 ppm, q, 1H ; 7,25-7,55 ppm, m, 6H ; 7,80-7,90 ppm, m, 3H ; 8,59 ppm, d, 2H. |

**TABLEAU VII (Suite 8)**

| EXEMPLE | $R_1$ | $R_2$ | $R_3$ | $R_5$ | $R_6$ | SPECTRE RMN (SOLVANT) |
|---|---|---|---|---|---|---|
| 41 | 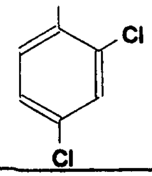 | $-CH_3$ | 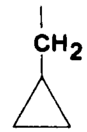 | $-CH_3$ | 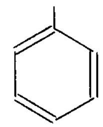 | ($CDCl_3$) Base<br>0,31-1,22 ppm, m, 5H ; 1,67 ppm, d, 3H ; 2,16 ppm, s, 3H ; 2,84-3,28 ppm, m, 2H ; 5,47 ppm, q, 1H ; 7,21-7,49 ppm, m, 8H. |
| 42 | 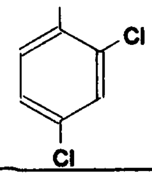 | $-CH_3$ | $-C_3H_7$ | 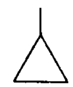 | 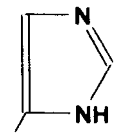 | ($CDCl_3$) Base<br>0,33-0,93 ppm, m, 7H ; 1,40-1,83 ppm, m, 3H ; 2,16 ppm, s, 3H ; 3,22-3,37 ppm, m, 2H ; 4,33 ppm, d, 1H ; 7,27-7,31 ppm, m, 4H ; 7,50 ppm, s, 1H. |
| 43 | 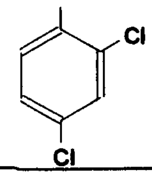 | $-CH_3$ | $-C_3H_7$ | $-CH_3$ | 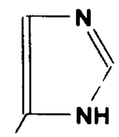 | ($CDCl_3$) Base<br>0,84 ppm, t, 3H ; 1,44-1,81 ppm, m, 5H ; 2,16 ppm, s, 3H ; 3,15 ppm, t, 2H ; 5,40 ppm, q, 1H ; 7,21-7,38 ppm, m, 4H ; 7,50 ppm, s, 1H. |
| 44 | 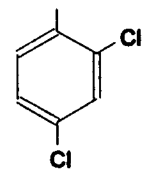 | $-CH_3$ | $-C_3H_7$ | 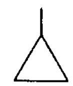 | 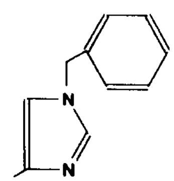 | ($CDCl_3$) Base<br>0,31-0,92 ppm, m, 7H ; 1,41-1,83 ppm, m, 3H ; 2,10 ppm, s, 3H ; 3,22-3,50 ppm, m, 2H ; 4,50 ppm, d, 1H ; 5,04 ppm, s, 2H ; 6,87 ppm, s, 1H ; 7,10-7,46 ppm, m, 9H. |
| 45 | 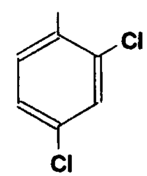 | $-CH_3$ | $-C_3H_7$ | 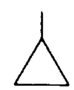 | 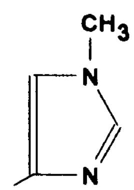 | ($CDCl_3$) Base<br>0,33-0,95 ppm, m, 7H ; 1,39 ppm, m, 3H ; 2,11 ppm, s, 3H ; 3,10-3,57 ppm, m, 2H ; 3,62 ppm, s, 3H ; 4,46 ppm, d, 1H ; 6,85 ppm, s, 1H ; 7,19-7,44 ppm, m, 5H. |

## TABLEAU VII (Suite 9)

| EXEMPLE | $R_1$ | $R_2$ | $R_3$ | $R_5$ | $R_6$ | SPECTRE RMN (SOLVANT) |
|---|---|---|---|---|---|---|
| 46 | 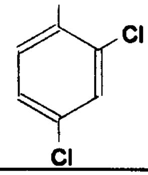 | -Br | $-C_3H_7$ | $-CH_3$ | 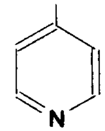 | ($CDCl_3$) Base 0,84 ppm, t, 3H ; 1,54-1,66 ppm, m, 5H ; 3,03-3,13 ppm, m, 2H ; 5,61 ppm, q, 1H ; 7,24-7,40 ppm, m, 4H ; 7,47 ppm, d, 1H ; 8,57 ppm, d, 2H. |
| 47 | 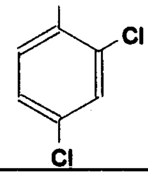 | $-CH_3$ | $-C_3H_7$ | 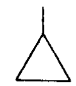 | 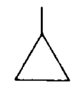 | ($CDCl_3$) Base 0,30-0,72 ppm, m, 8H ; 0,89-1,26 ppm, m, 5H ; 1,84 ppm, m, 2H ; 2,12 ppm, s, 3H ; 3,11 ppm, t, 1H ; 3,38 ppm, t, 2H ; 7,28-7,45 ppm, m, 3H. |
| 48 | 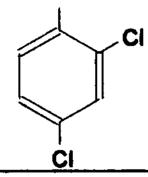 | $-CH_3$ | $-C_3H_7$ | 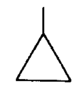 | 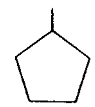 | ($CDCl_3$) Base 0,27-0,78 ppm, m, 4H ; 0,93 ppm, t, 3H ; 1,29-2,01 ppm, m, 12H ; 2,11 ppm, s, 3H ; 3,00-3,47 ppm, m, 3H ; 7,18-7,43 ppm, m, 3H. |
| 49 | 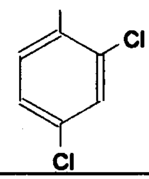 | -CHO | $-C_3H_7$ | $-CH_3$ | 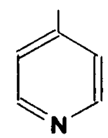 | ($CDCl_3$) Base 0,87 ppm, t, 3H ; 1,54-1,72 ppm, m, 5H ; 3,11-3,26 ppm, m, 2H ; 5,82 ppm, s, 1H ; 7,24 ppm, d, 2H ; 7,32-7,42 ppm, m, 2H ; 7,52 ppm, dd, 1H ; 8,60 ppm, d, 2H ; 9,42 ppm, s, 1H. |
| 50 | 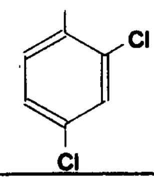 | $-CH_2OH$ | $-C_3H_7$ | $-CH_3$ | 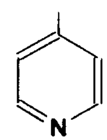 | ($CDCl_3$) Base 0,84 ppm, t, 3H ; 1,55-1,75 ppm, m, 5H ; 2,26 ppm, s, 1H ; 3,05-3,16 ppm, m, 2H ; 4,33 ppm, s, 2H ; 5,68 ppm, q, 1H ; 7,24-7,34 ppm, m, 4H ; 7,45 ppm, s, 1H ; 8,52 ppm, d, 2H. |

## TABLEAU VII (Suite 10)

| EXEMPLE | $R_1$ | $R_2$ | $R_3$ | $R_5$ | $R_6$ | SPECTRE RMN (SOLVANT) |
|---|---|---|---|---|---|---|
| 51 | 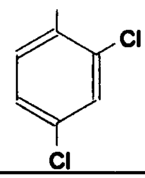 | $-CH_3$ | $-CH_2-CH=CH_2$ | $-CH_3$ | 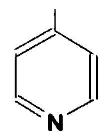 | ($CDCl_3$) Base<br>1,52-1,56 ppm, d, 3H ; 2,07 ppm, s, 3H ; 3,69-3,73 ppm, m, 2H ; 5,05-5,17 ppm, m, 2H ; 5,61-5,70 ppm, m, 2H ; 7,14-7,37 ppm, m, 5H ; 8,46-8,49 ppm, m, 2H. |
| 52 | 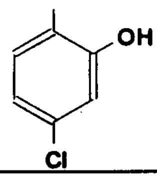 | $-CH_3$ | $-C_3H_7$ | $-CH_3$ | 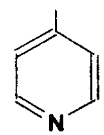 | ($CDCl_3$) Base<br>0,88 ppm, t, 3H ; 1,50-1,70 ppm, m, 5H ; 2,46 ppm, s, 3H ; 3,00-3,25 ppm, m, 2H ; 5,23 ppm, q, 1H ; 6,83 ppm, dd, 1H ; 6,94 ppm, d, 1H ; 7,21 ppm, d, 2H ; 7,33 ppm, d, 1H ; 8,58 ppm, d, 2H. |
| 53 | 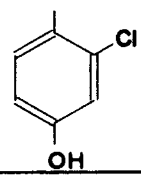 | $-CH_3$ | $-C_3H_7$ | $-CH_3$ | 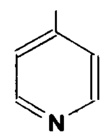 | ($CDCl_3$) Base<br>0,87 ppm, t, 3H ; 1,65-1,68 ppm, m, 5H ; 2,14 ppm, s, 3H ; 3,16-3,18 ppm, m, 2H ; 5,50 ppm, q, 1H ; 6,60 ppm, dd, 1H ; 6,75 ppm, s, 1H ; 7,07 ppm, d, 2H ; 7,30 ppm, d, 1H ; 8,54 ppm, dd, 2H. |
| 54 | 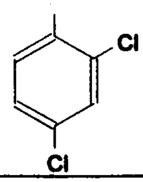 | $-CH_3$ | $-C_3H_7$ | 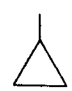 | 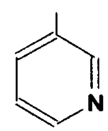 | ($CDCl_3$) Base<br>0,42 ppm, m, 1H ; 0,65 ppm, m, 2H ; 0,82 ppm, m, 4H ; 1,35 ppm, m, 1H ; 1,68 ppm, m, 2H ; 2,11 ppm, s, 3H ; 3,22 ppm, m, 2H ; 4,74 ppm, d, 1H ; 7,08-7,24 ppm, m, 3H ; 7,73 ppm, d, 1H ; 8,45 ppm, d, 1H ; 8,76 ppm, d, 1H. |
| 55 | 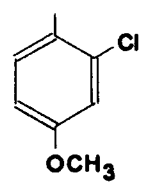 | $-CH_3$ | $-C_3H_7$ | 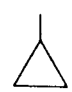 | 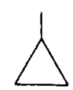 | ($CDCl_3$) Base<br>0,3-0,7 ppm, m, 8H ; 0,8-1,2 ppm, m, 5H ; 2,11 ppm, s, 3H ; 3,13 ppm, t, 1H ; 3,36 ppm, m, 2H ; 3,79 ppm, s, 3H ; 6,7-7,0 ppm, m, 2H ; 7,28 ppm, d, 1H. |

## TABLEAU VII (Suite 11)

| EXEMPLE | R1 | R2 | R3 | R5 | R6 | SPECTRE RMN (SOLVANT) |
|---|---|---|---|---|---|---|
| 56 | 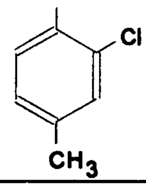 | $-CH_3$ | $-C_3H_7$ | 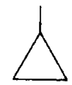 | 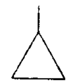 | ($CDCl_3$) Base<br>0,3-0,7 ppm, m, 8H ; 0,9-1,25 ppm, m, 5H ; 1,7-2,0 ppm, m, 2H ; 2,12 ppm, s, 3H ; 2,33 ppm, s, 3H ; 3,14 ppm, t, 1H ; 3,37 ppm, m, 2H ; 7,0-7,3 ppm, m, 3H. |
| 57 | 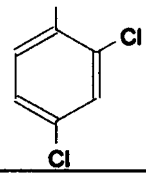 | $-CH_3$ | $-C_3H_7$ | 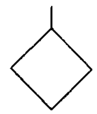 | 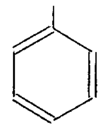 | ($CDCl_3$) Base<br>0,75 ppm, m, 3H ; 1,2-2,1 ppm, m, 8H ; 2,06 ppm, s, 3H ; 2,8-3,2 ppm, m, 3H ; 5,53 ppm, d, 1H ; 7,2-7,5 ppm, m, 8H. |
| 58 | 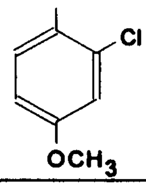 | $-CH_3$ | $-C_3H_7$ | 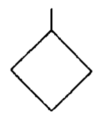 | 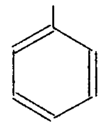 | ($CDCl_3$) Base<br>0,75 ppm, m, 3H ; 1,3-2,1 ppm, m, 8H ; 2,16 ppm, s, 3H ; 2,8-3,2 ppm, m, 3H ; 3,80 ppm, s, 3H ; 5,65 ppm, d, 1H ; 6,8-7,4 ppm, m, 8H. |
| 59 | 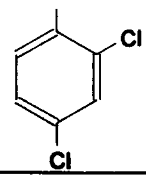 | $-CH_3$ | $-C_3H_7$ | 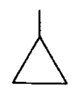 | 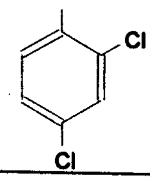 | ($CDCl_3$) Base<br>0,3-1,0 ppm, m, 7H ; 1,2-1,8 ppm, m, 3H ; 2,13 ppm, s, 3H ; 3,25 ppm, m, 2H ; 4,82 ppm, d, 1H ; 7,1-7,7 ppm, m, 6H. |
| 60 | 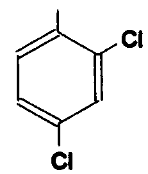 | $-CH_3$ | $-C_3H_7$ | 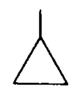 | 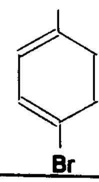 | ($CDCl_3$) Base<br>0,3-1,0 ppm, m, 7H ; 1,2-1,8 ppm, m, 3H ; 2,15 ppm, s, 3H ; 3,20 ppm, m, 2H ; 4,67 ppm, d, 1H ; 7,1-7,7 ppm, m, 7H. |

**TABLEAU VII (Suite 12)**

| EXEMPLE | $R_1$ | $R_2$ | $R_3$ | $R_5$ | $R_6$ | SPECTRE RMN (SOLVANT) |
|---|---|---|---|---|---|---|
| 61 | 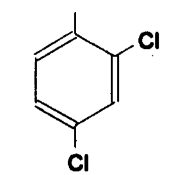 | $-CH_3$ | $-C_3H_7$ | 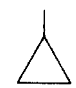 | 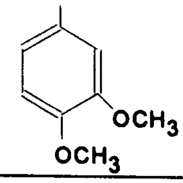 | ($CDCl_3$) Base<br>0,35-1,0 ppm, m, 7H ; 1,2-1,9 ppm, m, 3H ; 2,18 ppm, s, 3H ; 3,2 ppm, m, 2H ; 3,85 ppm, s, 3H ; 3,90 ppm, s, 3H ; 4,66 ppm, d, 1H ; 6,75-7,5 ppm, m, 6H. |
| 62 | 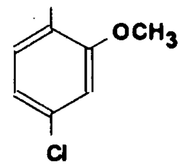 | $-CH_3$ | $-C_3H_7$ | 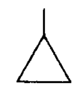 | 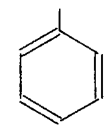 | ($CDCl_3$) Base<br>0,4-1,1 ppm, m, 7H ; 1,3-1,9 ppm, m, 3H ; 2,25 ppm, s, 3H ; 3,36 ppm, m, 2H ; 3,88 ppm, s, 3H ; 4,85 ppm, d, 1H ; 6,95-7,8 ppm, m, 8H. |
| 63 | 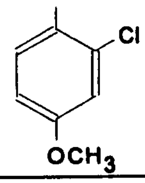 | $-CH_3$ | $-C_3H_7$ | 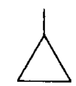 | 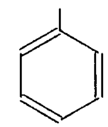 | ($CDCl_3$) Base<br>0,54 ppm, m, 1H ; 0,7-1 ppm, m, 6H ; 1,5 ppm, m, 1H ; 1,84 ppm, m, 2H ; 2,25 ppm, s, 3H ; 3,31 ppm, q, 2H ; 3,82 ppm, s, 3H ; 4,84 ppm, d, 1H ; 6,90 ppm, m, 1H ; 7,07 ppm, m, 1H ; 7,37 ppm, m, 4H ; 7,60 ppm, m, 2H. |
| 64 | 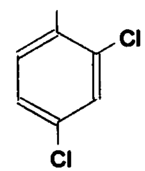 | $-CH_3$ | $-C_3H_7$ | 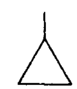 | 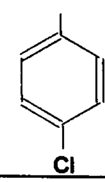 | ($CDCl_3$) Base<br>0,3-1,0 ppm, m, 7H ; 1,1-1,8 ppm, m, 3H ; 2,13 ppm, s, 3H ; 3,15 ppm, m, 2H ; 4,70 ppm, d, 1H ; 7-7,5 ppm, m, 7H. |
| 65 | 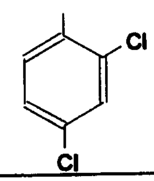 | $-CH_3$ | $-C_3H_7$ | 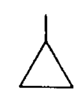 | 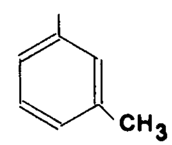 | ($CDCl_3$) Base<br>0,3-0,95 ppm, m, 7H ; 1,1-1,9 ppm, m, 3H ; 2,13 ppm, s, 3H ; 2,32 ppm, s, 3H ; 3,15 ppm, m, 2H ; 4,62 ppm, d, 1H ; 6,85-7,5 ppm, m, 7H. |

## TABLEAU VII (Suite 13)

| EXEMPLE | $R_1$ | $R_2$ | $R_3$ | $R_5$ | $R_6$ | SPECTRE RMN (SOLVANT) |
|---|---|---|---|---|---|---|
| 66 | 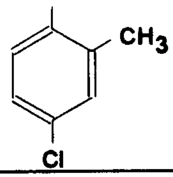 | $-CH_3$ | $-C_3H_7$ | 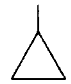 | 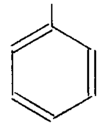 | ($CDCl_3$) Base 0,3-0,95 ppm, m, 7H ; 1,15-1,8 ppm, m, 3H ; 2,16 ppm, s, 3H ; 2,27 ppm, s, 3H ; 3,15 ppm, m, 2H ; 4,68 ppm, d, 1H ; 7,0-7,5 ppm, m, 8H. |
| 67 | 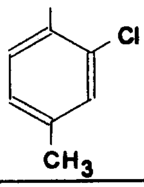 | $-CH_3$ | $-C_3H_7$ | 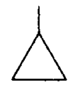 | 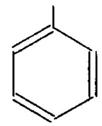 | ($CDCl_3$) Base 0,3-1,0 ppm, m, 7H ; 1,1-1,8 ppm, m, 3H ; 2,18 ppm, s, 3H ; 2,34 ppm, s, 3H ; 3,1 ppm, m, 2H ; 4,73 ppm, d, 1H ; 6,9-7,5 ppm, m, 8H. |
| 68 | 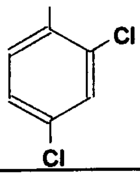 | $-CH_3$ | $-C_3H_7$ | $-CH_3$ | 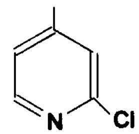 | ($CDCl_3$) Base 0,86 ppm, m, 3H ; 1,5-1,7 ppm, m, 5H ; 2,16 ppm, s, 3H ; 3,0-3,2 ppm, m, 2H ; 5,63 ppm, q, 1H ; 7,1-7,4 ppm, m, 5H ; 8,31 ppm, d, 1H. |
| 69 | 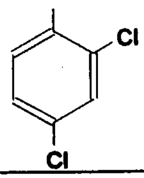 | $-CH_3$ | $-C_3H_7$ | 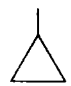 | 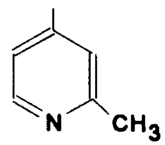 | ($CDCl_3$) Base 0,5-0,9 ppm, m, 7H ; 1,3-1,4 ppm, m, 1H ; 1,6-1,8 ppm, m, 2H ; 2,16 ppm, s, 3H ; 2,54 ppm, s, 3H ; 3,1-3,3 ppm, m, 2H ; 4,68 ppm, d, 1H ; 7,20 ppm, d 1H ; 7,43 ppm, s, 1H ; 8,41 ppm, d, 1H. |
| 70 | 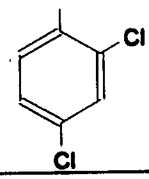 | $-CH_3$ | $-C_3H_7$ | $-CH_3$ | 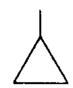 | ($CDCl_3$) Base 0,3-0,5 ppm, m, 4H ; 0,95 ppm, m, 4H ; 1,29 ppm, d, 3H ; 1,79 ppm, m, 2H ; 2,13 ppm, s, 3H ; 3,27-3,52 ppm, m, 3H ; 7,2-7,5 ppm, m, 3H. |

## TABLEAU VII (Suite 14)

| EXEMPLE | $R_1$ | $R_2$ | $R_3$ | $R_5$ | $R_6$ | SPECTRE RMN (SOLVANT) |
|---|---|---|---|---|---|---|
| 71 | 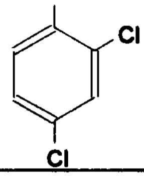 | $-CH_3$ | $-C_3H_7$ | 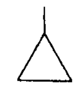 | 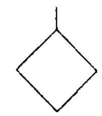 | **($CDCl_3$) Base** 0,2-1,0 ppm, m, 8H ; 1,5-2,1 ppm, m, 8H ; 2,16 ppm, s, 3H ; 2,5-2,8 ppm, m, 1H ; 3,1-3,4 ppm, m, 3H ; 7,1-7,4 ppm, m, 3H. |
| 72 | 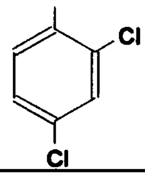 | $-CH_3$ | $-C_3H_7$ | $-CH_2-CH_2-CH=CH_2$ | 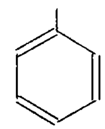 | **($CDCl_3$) Base** 0,73 ppm, m, 3H ; 1,45 ppm, m, 2H ; 2,15 ppm, m, 7H ; 3,03 ppm, m, 2H ; 4,93-5,10 ppm, m, 2H ; 5,42 ppm, t, 1H ; 5,80 ppm, m, 1H ; 7,21-7,45 ppm, m, 8H. |
| 73 | 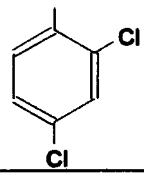 | $-CH_3$ | $-C_3H_7$ | 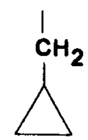 | 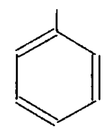 | **($CDCl_3$) Base** 0,15 ppm, m, 2H ; 0,40 ppm, m, 2H ; 0,71 ppm, m, 4H ; 1,45 ppm, m, 2H ; 1,92 ppm, m, 2H ; 2,12 ppm, s, 3H ; 3,06 ppm, m, 2H ; 5,33 ppm, t, 1H ; 7,15-7,43 ppm, m, 8H. |
| 74 | 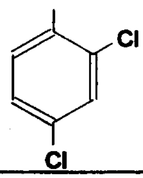 | $-CH_3$ | $-C_3H_7$ | 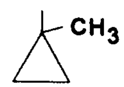 | 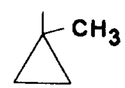 | **($CDCl_3$) Base** 0,3-0,5 ppm, m, 4H ; 0,5-0,7 ppm, m, 4H ; 0,89 ppm, m, 3H ; 1,21 ppm, s, 6H ; 2,7-2,9 ppm, m, 2H ; 2,13 ppm, s, 3H ; 3,3-3,5 ppm, m, 3H ; 7,2-7,4 ppm, m, 3H. |
| 75 | 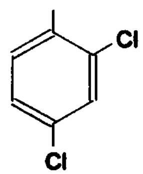 | $-CH_3$ | $-C_3H_7$ | 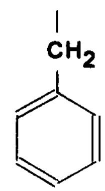 | 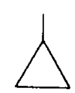 | **($CDCl_3$) Base** 0,1-0,15 ppm, m, 2H ; 0,25-0,6 ppm, m, 2H ; 0,9 ppm, m, 3H ; 1,1-1,3 ppm, m, 1H ; 1,7-1,85 ppm, m, 2H ; 2,14 ppm, s, 3H ; 2,9-3,4 ppm, m, 5H ; 7,1-7,6 ppm, m, 8H. |

## TABLEAU VII (Suite 15)

| EXEMPLE | R1 | R2 | R3 | R5 | R6 | SPECTRE RMN (SOLVANT) |
|---|---|---|---|---|---|---|
| 76 | 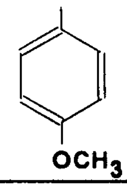 | $-CH_3$ | $-C_3H_7$ | 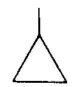 | 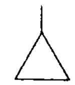 | ($CDCl_3$) Base 0,4-0,5 ppm, m, 8H ; 0,9-1,2 ppm, m, 5H ; 1,7-1,9 ppm, m, 2H ; 2,37 ppm, s, 3H ; 3,04 ppm, t, 1H ; 3,40 ppm, m, 2H ; 3,82 ppm, s, 3H ; 6,91 ppm, d, 2H ; 7,54 ppm, d, 2H. |
| 77 | 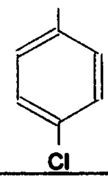 | $-CH_3$ | $-C_3H_7$ | 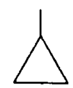 | 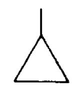 | ($CDCl_3$) Base 0,2-0,8 ppm, m, 8H ; 0,98 ppm, t, 3H ; 1 ppm, m, 2H ; 1,85 ppm, q, 2H ; 2,37 ppm, s, 3H ; 3,0 ppm, t, 1H ; 2,4 ppm, m, 2H ; 7,24-7,6 ppm, m, 4H. |
| 78 | 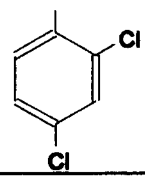 | $-CH_3$ | $-C_3H_7$ | 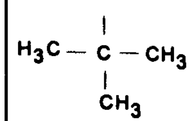 | 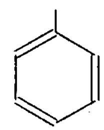 | ($CDCl_3$) Base 0,71 ppm, t, 3H ; 1,0 ppm, m, 1H ; 1,18 ppm, s, 9H ; 1,70 ppm, m, 1H ; 2,17 ppm, s, 3H ; 3,40 ppm, m, 2H ; 5,28 ppm, s, 1H ; 7,24-7,56 ppm, m, 8H. |
| 79 | 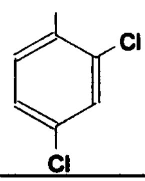 | $-CH_3$ | $-C_3H_7$ | 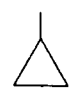 | 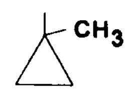 | ($CDCl_3$) Base 0,3-0,9 ppm, m, 9H ; 0,95 ppm, t, 3H ; 1,10 ppm, s, 3H ; 1,80 ppm, m, 2H ; 2,13 ppm, s, 3H ; 3,5 ppm, m, 3H ; 7,4 ppm, m, 3H. |
| 80 | 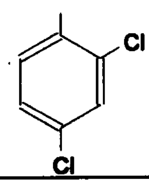 | $-CH_3$ | $-C_3H_7$ | 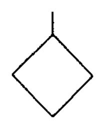 | 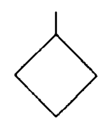 | ($CDCl_3$) Base 0,9 ppm, t, 3H ; 1,9 ppm, m, 16H ; 2,1 ppm, t, 2H ; 2,13 ppm, s, 3H ; 3,2 ppm, m, 2H ; 4,0 ppm, t, 1H ; 7,5 ppm, m, 3H. |

## TABLEAU VII (Suite 16)

| EXEMPLE | $R_1$ | $R_2$ | $R_3$ | $R_5$ | $R_6$ | SPECTRE RMN (SOLVANT) |
|---|---|---|---|---|---|---|
| 81 | 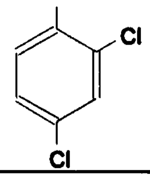 | $-CH_3$ | $-C_3H_7$ | 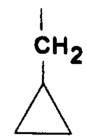 | 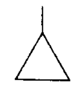 | ($CDCl_3$) Base<br>0,02-1,0 ppm, m, 11H ; 1,3-2,1 ppm, m, 7H ; 2,12 ppm, s, 3H ; 3,1-3,4 ppm, m, 3H ; 7,1-7,4 ppm, m, 3H. |
| 82 | 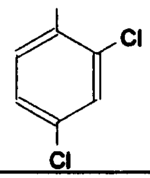 | H | $-C_3H_7$ | 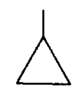 | 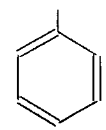 | ($CDCl_3$) Base<br>0,3-1 ppm, m, 7H ; 1,2-1,9 ppm, m, 3H ; 3,25 ppm, m, 2H ; 4,73 ppm, d, 1H ; 7-7,6 ppm, m, 8H ; 7,95 ppm, m, 1H. |
| 83 | 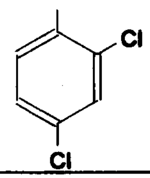 | $-CH_3$ | 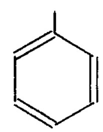 | 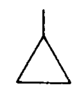 | 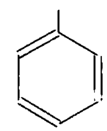 | ($CDCl_3$) Base<br>0,3-1,3 ppm, m, 5H ; 2,04 ppm, s, 3H ; 5,2 ppm, d, 1H ; 7,0-7,5 ppm, m, 13H. |
| 84 | 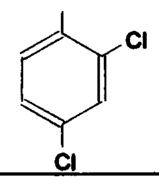 | $-CH_3$ | 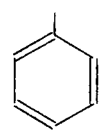 | 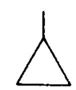 | 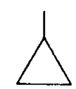 | ($CDCl_3$) Base<br>0,3-0,9 ppm, m, 10H ; 1,99 ppm, s, 3H ; 3,6 ppm, t, 1H ; 7,43 ppm, m, 8H. |
| 85 | 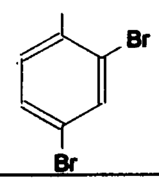 | $-CH_3$ | $-C_3H_7$ | 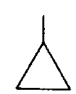 | 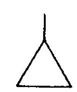 | ($CDCl_3$) Base<br>0,5-0,8 ppm, m, 8H ; 1,05 ppm, t, 3H ; 1,6-1,9 ppm, m, 4H ; 2,06 ppm, s, 3H ; 3,67 ppm, m, 3H ; 7,2-7,8 ppm, m, 3H. |

**TABLEAU VII (Suite 17)**

| Exemple | $R_1$ | $R_2$ | $R_3$ | $R_5$ | $R_6$ | Spectre RMN (solvant) |
|---|---|---|---|---|---|---|
| **86** | 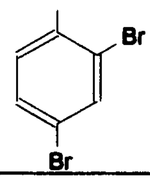 | $-CH_3$ | $-C_3H_7$ | 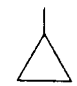 | 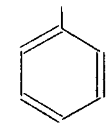 | **($CDCl_3$) Chlorhydrate** 0,6-0,9 ppm, m, 7H ; 1,1-1,8 ppm, m, 3H ; 2,09 ppm, s, 3H ; 3,6 ppm, m, 2H ; 4,9 ppm, m, 1H ; 7,6 ppm, m, 7H ; 7,81 ppm, m, 1H. |
| **87** | 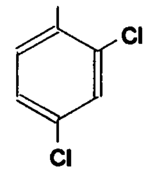 | $-CH_3$ | $-C_3H_7$ | 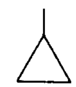 | 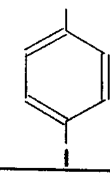 | **($CDCl_3$) Base** 0,3-1 ppm, m, 7H ; 1,2-1,9 ppm, m, 3H ; 2,15 ppm, s, 3H ; 3,2 ppm, m, 2H ; 4,68 ppm, d, 1H ; 7,15-7,8 ppm, m, 7H. |
| **88** | 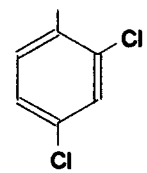 | $-CH_3$ | $-C_3H_7$ | 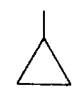 | 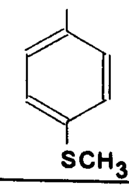 | **($CDCl_3$) Base** 0,3-0,9 ppm, m, 7H ; 1,1-1,8 ppm, m, 3H ; 2,13 ppm, s, 3H ; 2,44 ppm, s, 3H ; 3,1 ppm, m, 2H ; 4,66 ppm, d, 1H ; 7-7,4 ppm, m, 7H. |
| **89** | 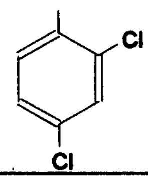 | Br | $-C_3H_7$ | 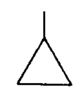 | 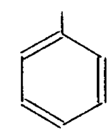 | **($CDCl_3$) Base** 0,6-1,1 ppm, m, 7H ; 1,3-2,0 ppm, m, 3H ; 3,25 ppm, m, 2H ; 4,81 ppm, d, 1H ; 7,2-7,65 ppm, m, 8H. |

TABLEAU VII (Suite 18)

| EXEMPLE | $R_1$ | $R_2$ | $R_3$ | $R_5$ | $R_6$ | SPECTRE RMN (SOLVANT) |
|---|---|---|---|---|---|---|
| 90 | 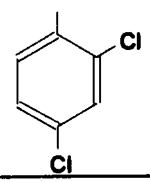 | I | $-C_3H_7$ | 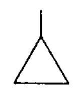 | 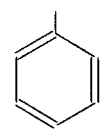 | ($CDCl_3$) Base 0,5-1,1 ppm, m, 7H ; 1,3-2,0 ppm, m, 3H ; 3,32 ppm, m, 2H ; 4,80 ppm, d, 1H ; 7,2-7,7 ppm, m, 8H. |
| 91 | 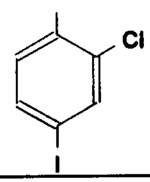 | $-CH_3$ | $-C_3H_7$ | 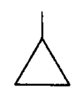 | 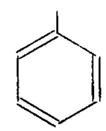 | ($CDCl_3$) Base 0,4-1,1 ppm, m, 7H ; 1,3-1,95 ppm, m, 3H ; 2,19 ppm, s, 3H ; 3,25 ppm, m, 2H ; 4,75 ppm, d, 1H ; 7,1-7,9 ppm, m, 8H. |

**PREPARATION PHARMACEUTIQUE**

**Exemple 94**

**Gélules dosées à 20 mg de sulfate de 4-(2,4-dichloro phényl) 2-[N-(dicyclopropyl méthyl) N-propyl amino] 5-méthyl thiazole.**

| | |
|---|---|
| Sulfate de 4-(2,4-dichloro phényl) 2-[N-(dicyclopropyl méthyl) N-propyl amino] 5-méthyl thiazole | 20 mg |
| Amidon de maïs | 15 mg |
| Lactose | 25 mg |
| Talc | 5 mg |

Pour une gélule N°3

**Revendications**

**Revendications pour les Etats contractants suivants : AT, BE, CH, DE, DK, FR, GB, GR, IE, IT, LI, LU, MC, NL, PT, SE**

1. Composés de formule I :

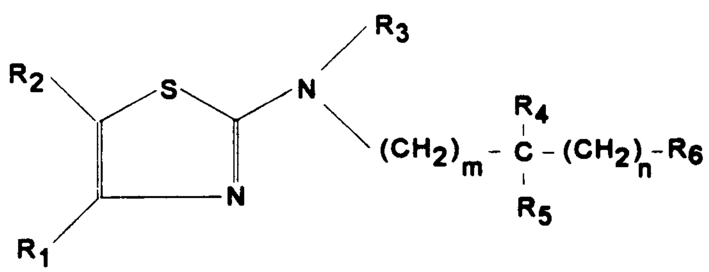

(I)

dans laquelle,

- $R_1$ représente un radical de formule $A_1$ ou un radical de formule $A_2$ :

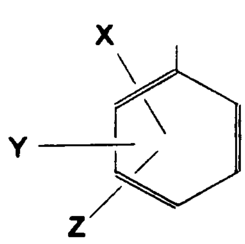

$(A_1)$

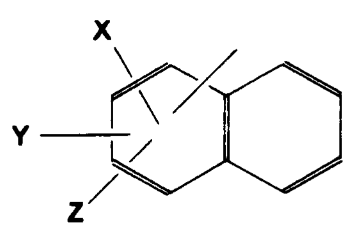

$(A_2)$

(dans lesquelles X, Y, Z, identiques ou différents, représentent chacun un atome d'hydrogène, un atome d'halogène, un radical alcoxy de 1 à 5 atomes de carbone, un radical alkyle de 1 à 5 atomes de carbone, un radical hydroxy, un radical cyano, un radical nitro, un radical trifluorométhyle ou un radical aralkyle de 7 à 9 atomes de carbone),
- $R_2$ représente un atome d'hydrogène, un atome d'halogène, un radical alkyle de 1 à 5 atomes de carbone, un radical hydroxyméthyle ou un radical formyle,
- $R_3$ représente un radical alkyle de 1 à 5 atomes de carbone, un radical cycloalkyle de 3 à 8 atomes de carbone, un radical alcényle de 2 à 6 atomes de carbone, un radical cycloalkylalkyle de 4 à 8 atomes de carbone, ou un radical phényle,

- $R_4$ représente un atome d'hydrogène, un radical alkyle de 1 à 5 atomes de carbone, un radical cycloalkyle de 3 à 6 atomes de carbone, ou un radical cycloalkyle alkyle de 4 à 8 atomes de carbone ayant une chaîne linéaire ou ramifiée,
- $R_5$ représente un radical alkyle de 1 à 5 atomes de carbone, un radical cycloalkyle de 3 à 8 atomes de carbone éventuellement substitué par des radicaux alkyle de 1 à 5 atomes de carbone, un radical cycloalkylalkyle de 4 à 8 atomes de carbone ayant une chaîne linéaire ou ramifiée, un radical alcényle de 2 à 6 atomes de carbone, ou un radical de formule B :

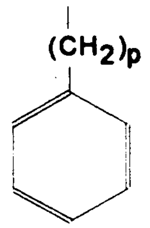

(B)

(dans laquelle p est égal à 0, 1, 2, ou 3),

- $R_6$ représente un radical phényle, un radical pyridyle, un radical imidazolyle, un radical pyrrolyle, un radical thiényle ou un radical furyle (éventuellement substitués par un ou plusieurs atomes d'halogène, par des radicaux alcoxy de 1 à 5 atomes de carbone, par des radicaux alkyle de 1 à 5 atomes de carbone, par des radicaux hydroxy, par des radicaux cyano, par des radicaux nitro, par des radicaux trifluorométhyle par des radicaux méthylthio ou par des radicaux de formule B), ou un radical cycloalkyle de 3 à 8 atomes de carbone éventuellement substitué par des radicaux alkyle de 1 à 5 atomes de carbone,
- m, n, identiques ou différents représentent chacun 0 ou 1,

leurs stéréoisomères possibles et leurs sels d'addition à un acide minéral ou organique.

**2.** Composés de formule I selon la revendication 1, dans laquelle,

- $R_1$ représente un radical de formule $A_1$,
- $R_2$ représente un atome d'halogène ou un radical alkyle de 1 à 5 atomes de carbone,
- $R_3$ représente un radical alkyle de 1 à 5 atomes de carbone, un radical cycloalkyle de 3 à 8 atomes de carbone, ou un radical alcényle de 2 à 6 atomes de carbone,
- $R_5$ représente un radical alkyle de 1 à 5 atomes de carbone, un radical cycloalkyle de 3 à 8 atomes de carbone, éventuellement substitué par des radicaux alkyle de 1 à 5 atomes de carbone, ou un radical cycloalkylalkyle de 4 à 8 atomes de carbone ayant une chaîne linéaire ou ramifiée et,
- $R_4$, $R_6$, m et n, ont la même signification que dans la formule I, selon la revendication 1,

leurs stéréoisomères ainsi que leurs sels d'addition à un acide minéral ou organique.

**3.** Composés de formule I selon la revendication 1, répondant à la formule $I_A$ :

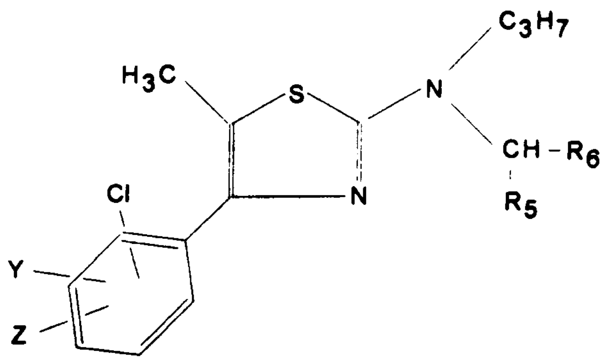

$(I_A)$

dans laquelle,

- Y et Z ont la même signification que pour la formule I, selon la revendication 1,
- $R_5$ représente un radical alkyle de 1 à 5 atomes de carbone ou un radical cycloalkyle de 3 à 8 atomes de carbone, éventuellement substitué par des radicaux alkyle de 1 à 5 atomes de carbone ou un radical cycloalkylalkyle de 4 à 8 atomes de carbone ayant une chaîne linéaire ou ramifiée,
- $R_6$ représente un radical phényle ou un radical pyridyle (éventuellement substitué par un ou plusieurs atomes d'halogène, des radicaux alcoxy de 1 à 5 atomes de carbone ou par des radicaux alkyle de 1 à 5 atomes de carbone, des radicaux hydroxy, des radicaux cyano, des radicaux nitro, des radicaux trifluorométhyle ou des radicaux méthylthio), un radical imidazolyle, éventuellement substitué par un radical alkyle de 1 à 5 atomes de carbone, ou un radical cycloalkyle de 3 à 8 atomes de carbone, éventuellement sustitué par des radicaux alkyle de 1 à 5 atomes de carbone,

leurs stéréoisomères et leurs sels d'addition à un acide minéral ou organique.

**4.** Le 4-(2,4-dichloro phényl) 5-méthyl 2-[N-(cyclopropyl pyrid-4-yl méthyl) N-propyl amino] thiazole, composé répondant à la formule I, selon la revendication 1, et ses sels d'addition à un acide minéral ou organique.

**5.** Le 4-(2,4-dichloro phényl) 5-méthyl 2-[N-(α-cyclopropyl benzyl) N-propyl amino] thiazole, composé répondant à la formule I, selon la revendication 1, et ses sels d'addition à un acide minéral ou organique.

**6.** Le 4-(2,4-dichloro phényl) 5-méthyl 2-[N-dicyclopropyl méthyl) N-propyl amino] thiazole, composé répondant à la formule I, selon la revendication 1, et ses sels d'addition à un acide minéral ou organique.

**7.** Le 4-(2,4-dichloro phényl) 5-méthyl 2-[N-(cyclopentyl cyclopropyl méthyl) N-propyl amino] thiazole, composé répondant à la formule I, selon la revendication 1, et ses sels d'addition à un acide minéral ou organique.

**8.** Le 4-(2-chloro-4-méthyl-phényl) 5-méthyl-2-[N-(dicyclopropylméthyl) N-propyl- amino] thiazole, composé répondant à la formule I, selon la revendication 1, et ses sels d'addition à un acide minéral ou organique.

**9.** Procédé de préparation des composés de formule I, selon la revendication 1, caractérisé en ce que l'on fait réagir un dérivé carbonylé alpha-halogéné de formule II :

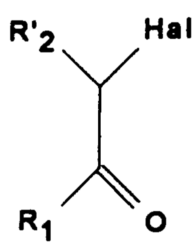

(II)

dans laquelle,

$R_1$ a la même signification que pour la formule I, selon la revendication 1 et $R'_2$ représente un atome d'hydrogène ou un radical alkyle de 1 à 5 atomes de carbone, et Hal représente un atome d'halogène,

soit

avec une thiourée de formule III :

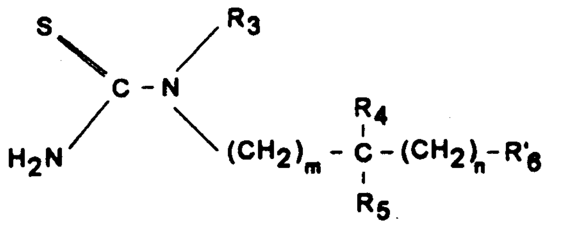

(III)

dans laquelle $R_3$, $R_4$, $R_5$, m et n, ont la même signification que pour la formule I, selon la revendication 1, et $R'_6$ a la signification donnée pour $R_6$ dans la formule I, selon la revendication 1, sauf dans le cas où $R_6$ contient des fonctions avec des atomes d'azote réactifs où $R'_6$ désigne alors le radical correspondant à $R_6$ dans lequel un hydrogène de ladite fonction réactive a été remplacée par un groupe protecteur résistant à l'hydrolyse en milieu alcalin,

pour former un composé de formule I' :

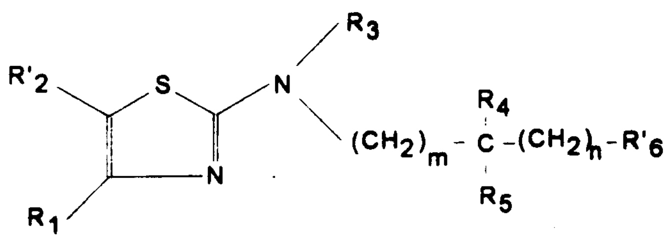

(I')

dans laquelle,

- $R_1$, $R_3$, $R_4$, $R_5$ m et n ont la même signification que pour la formule I, selon la revendication 1,
- $R'_2$ a la signification donnée pour la formule II, et,
- $R'_6$ a la signification donnée pour la formule III

soit

avec une thiourée de formule $III_A$ :

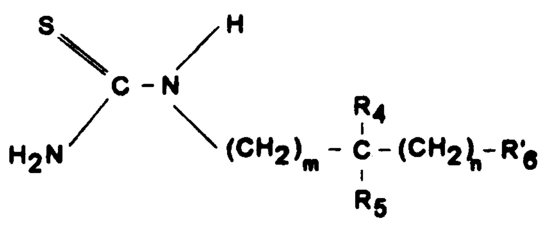

($III_A$)

dans laquelle $R_4$, $R_5$, m et n ont la même signification que pour la formule I, selon la revendication 1, et $R'_6$ a la signification donnée pour la formule III,

pour former les composés de formule IV :

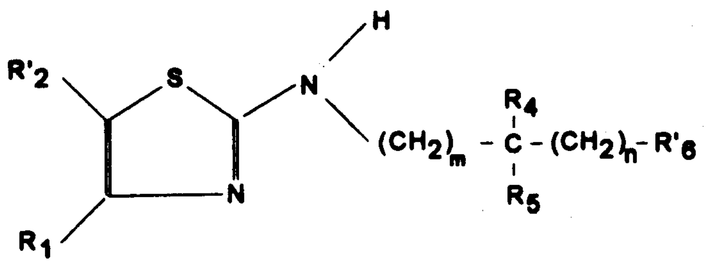

(IV)

dans laquelle $R_1$, $R_4$, $R_5$, m et n ont la même signification que pour la formule I, selon la revendication 1, $R'_2$ a la signification donnée pour la formule II et $R'_6$ a la signification donnée pour la formule III,

que l'on fait réagir avec un halogénure de formule V :

$$Hal - R_3 \qquad (V)$$

dans laquelle Hal représente un atome d'halogène et $R_3$ a la même signification que pour la formule I, selon la revendication 1,

pour former le composé de formule I',

et ensuite,

- l'on soumet les composés de formule I', dans laquelle $R'_2$ représente un atome d'hydrogéne,
  * soit à l'action d'un halogène, pour former les composés de formule I, selon la revendication 1, dans laquelle $R_2$ représente un atome d'halogène, lesquels ensuite, lorsque $R_2$ représente un atome de brome, peuvent être soumis à l'action d'un autre halogène pour former les composés de formule I dans laquelle $R_2$ représente cet atome d'halogène,
  * soit à l'action de chlorure d'oxalyle pour préparer les composés de formule I, selon la revendication 1, dans laquelle $R_2$ représente un radical formyle, lesquels peuvent être soumis à une réduction pour obtenir les composés de formule I, selon la revendication 1, dans laquelle $R_2$ représente un radical hydroxyméthyle,

  ou
- l'on soumet les composés de formule I' dans laquelle $R'_6$ représente un radical $R_6$ contenant des fonctions avec des atomes d'azote réactifs ayant un groupe protecteur, à une hydrolyse acide, pour obtenir les composés de formule I, selon la revendication 1, dans laquelle $R_6$ représente un radical contenant une amine primaire ou secondaire,

et le cas échéant, les composés de formule I, selon la revendication 1,

sont ensuite séparés en leurs stéréoisomères possibles et/ou salifiés par un acide organique ou minéral pour former les sels correspondants.

**10.** Utilisation des composés de formule I, selon l'une quelconque des revendications 1 à 8, pour la préparation d'un médicament utilisable dans le traitement des maladies nécessitant une modulation de l'action du facteur de libération de l'hormone corticotrope (CRF).

**11.** Composition pharmaceutique contenant comme principe actif un composé selon l'une quelconque des revendications 1 à 8, sous forme de base ou sous forme de sel avec un acide minéral ou organique pharmaceutiquement acceptable, en association ou en mélange avec un excipient inerte, non toxique, pharmaceutiquement acceptable.

**12.** Composés de formule IV :

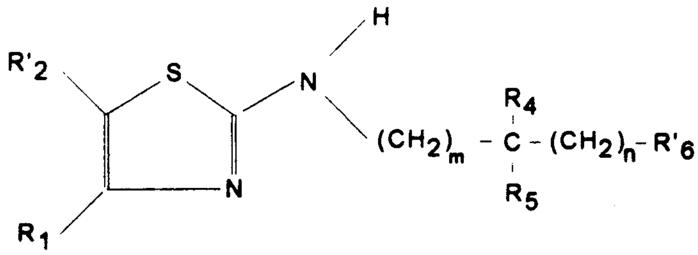

(IV)

dans laquelle,

- $R_1$, $R_4$, m et n ont la même signification que pour la formule I, selon la revendication 1,
- $R'_2$ représente un atome d'hydrogène ou un radical alkyle de 1 à 5 atomes de carbone,
- $R_5$ représente un radical alkyle de 1 à 5 atomes de carbone, un radical cycloalkyle de 3 à 8 atomes de carbone éventuellement substitué par des radicaux alkyle de 1 à 5 atomes de carbone, un radical cycloalkylalkyle de 4 à 8 atomes de carbone ayant une chaîne linéaire ou ramifiée, un radical alcényle de 2 à 6 atomes de carbone, ou un radical de formule B :

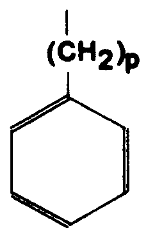

(B)

(dans laquelle p est égal à 1, 2, ou 3),

et

- $R'_6$ a la signification donnée pour $R_6$ dans la formule I, selon la revendication 1, sauf dans le cas où $R_6$ contient des fonctions avec des atomes d'azote réactifs où $R'_6$ désigne alors le radical correspondant à $R_6$ dans lequel un hydrogène de ladite fonction réactive a été remplacée par un groupe protecteur résistant à l'hydrolyse en milieu alcalin.

**Revendications pour l'Etat contractant suivant : ES**

**1.** Composés de formule I :

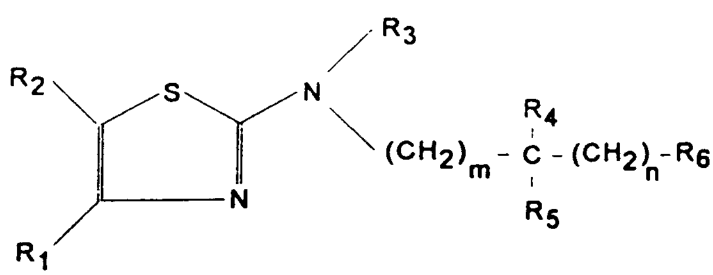

(I)

dans laquelle,

- $R_1$ représente un radical de formule $A_1$ ou un radical de formule $A_2$ :

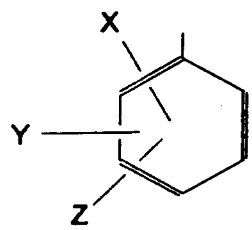 ($A_1$)

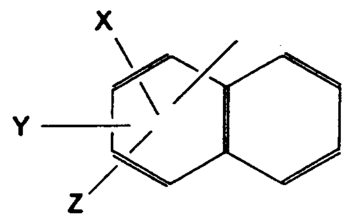 ($A_2$)

(dans lesquelles X, Y, Z, identiques ou différents, représentent chacun un atome d'hydrogène, un atome d'halogène, un radical alcoxy de 1 à 5 atomes de carbone, un radical alkyle de 1 à 5 atomes de carbone, un radical hydroxy, un radical cyano, un radical nitro, un radical trifluorométhyle ou un radical aralkyle de 7 à 9 atomes de carbone),

- $R_2$ représente un atome d'hydrogène, un atome d'halogène, un radical alkyle de 1 à 5 atomes de carbone, un radical hydroxyméthyle ou un radical formyle,
- $R_3$ représente un radical alkyle de 1 à 5 atomes de carbone, un radical cycloalkyle de 3 à 8 atomes de carbone, un radical alcényle de 2 à 6 atomes de carbone, un radical cycloalkylalkyle de 4 à 8 atomes de carbone, ou un radical phényle,
- $R_4$ représente un atome d'hydrogène, un radical alkyle de 1 à 5 atomes de carbone, un radical cycloalkyle de 3 à 6 atomes de carbone, ou un radical cycloalkyle alkyle de 4 à 8 atomes de carbone ayant une chaîne linéaire ou ramifiée,
- $R_5$ représente un radical alkyle de 1 à 5 atomes de carbone, un radical cycloalkyle de 3 à 8 atomes de carbone éventuellement substitué par des radicaux alkyle de 1 à 5 atomes de carbone, un radical cycloalkylalkyle de 4 à 8 atomes de carbone ayant une chaîne linéaire ou ramifiée, un radical alcényle de 2 à 6 atomes de carbone, ou un radical de formule B :

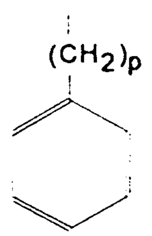 (B)

(dans laquelle p est égal à 0, 1, 2, ou 3),

- $R_6$ représente un radical phényle, un radical pyridyle, un radical imidazolyle, un radical pyrrolyle, un radical thiényle ou un radical furyle (éventuellement substitués par un ou plusieurs atomes d'halogène, par des radicaux alcoxy de 1 à 5 atomes de carbone, par des radicaux alkyle de 1 à 5 atomes de carbone, par des radicaux hydroxy, par des radicaux cyano, par des radicaux nitro, par des radicaux trifluorométhyle par des radicaux méthylthio ou par des radicaux de formule B), ou un radical cycloalkyle de 3 à 8 atomes de carbone éventuellement substitué par des radicaux alkyle de 1 à 5 atomes de carbone,
- m, n, identiques ou différents représentent chacun 0 ou 1,

leurs stéréoisomères possibles et leurs sels d'addition à un acide minéral ou organique.

**2.** Composés de formule I selon la revendication 1, dans laquelle,

- $R_1$ représente un radical de formule $A_1$,
- $R_2$ représente un atome d'halogène ou un radical alkyle de 1 à 5 atomes de carbone,
- $R_3$ représente un radical alkyle de 1 à 5 atomes de carbone, un radical cycloalkyle de 3 à 8 atomes de carbone, ou un radical alcényle de 2 à 6 atomes de carbone,
- $R_5$ représente un radical alkyle de 1 à 5 atomes de carbone, un radical cycloalkyle de 3 à 8 atomes de carbone, éventuellement substitué par des radicaux alkyle de 1 à 5 atomes de carbone, ou un radical cycloalkylalkyle de 4 à 8 atomes de carbone ayant une chaîne linéaire ou ramifiée et,
- $R_4$, $R_6$, m et n, ont la même signification que dans la formule I, selon la revendication 1,

leurs stéréoisomères ainsi que leurs sels d'addition à un acide minéral ou organique.

**3.** Composés de formule I selon la revendication 1, répondant à la formule $I_A$ :

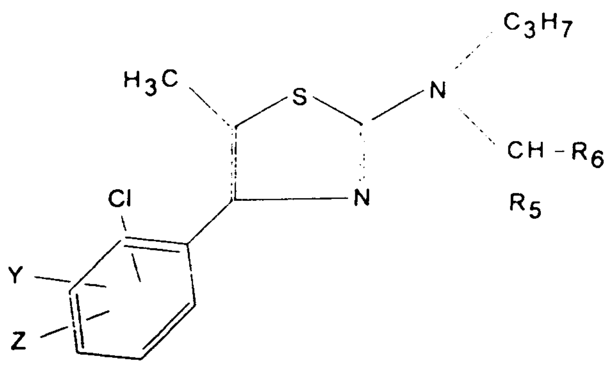

$(I_A)$

dans laquelle,

- Y et Z ont la même signification que pour la formule I, selon la revendication 1,
- $R_5$ représente un radical alkyle de 1 à 5 atomes de carbone ou un radical cycloalkyle de 3 à 8 atomes de carbone, éventuellement substitué par des radicaux alkyle de 1 à 5 atomes de carbone ou un radical cycloalkylalkyle de 4 à 8 atomes de carbone ayant une chaîne linéaire ou ramifiée,
- $R_6$ représente un radical phényle ou un radical pyridyle (éventuellement substitué par un ou plusieurs atomes d'halogène, des radicaux alcoxy de 1 à 5 atomes de carbone ou par des radicaux alkyle de 1 à 5 atomes de carbone, des radicaux hydroxy, des radicaux cyano, des radicaux nitro, des radicaux trifluorométhyle ou des radicaux méthylthio), un radical imidazolyle, éventuellement substitué par un radical alkyle de 1 à 5 atomes de carbone, ou un radical cycloalkyle de 3 à 8 atomes de carbone, éventuellement sustitué par des radicaux alkyle de 1 à 5 atomes de carbone,

leurs stéréoisomères et leurs sels d'addition à un acide minéral ou organique.

**4.** Le 4-(2,4-dichloro phényl) 5-méthyl 2-[N-(cyclopropyl pyrid-4-yl méthyl) N-propyl amino] thiazole, composé répondant à la formule I, selon la revendication 1, et ses sels d'addition à un acide minéral ou organique.

**5.** Le 4-(2,4-dichloro phényl) 5-méthyl 2-[N-($\alpha$-cyclopropyl benzyl) N-propyl amino] thiazole, composé répondant à la formule I, selon la revendication 1, et ses sels d'addition à un acide minéral ou organique.

**6.** Le 4-(2,4-dichloro phényl) 5-méthyl 2-[N-dicyclopropyl méthyl) N-propyl amino] thiazole, composé répondant à la formule I, selon la revendication 1, et ses sels d'addition à un acide minéral ou organique.

**7.** Le 4-(2,4-dichloro phényl) 5-méthyl 2-[N-(cyclopentyl cyclopropyl méthyl) N-propyl amino] thiazole, composé répondant à la formule I, selon la revendication 1, et ses sels d'addition à un acide minéral ou organique.

**8.** Le 4-(2-chloro-4-méthyl-phényl) 5-méthyl-2-[N-(dicyclopropylméthyl) N-propyl- amino] thiazole, composé répondant à la formule I, selon la revendication 1, et ses sels d'addition à un acide minéral ou organique.

**9.** Procédé de préparation des composés de formule I

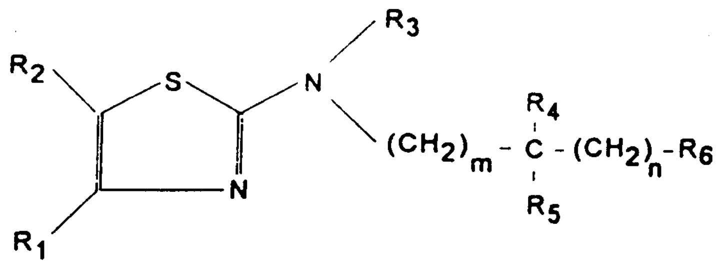

(I)

dans laquelle,

- $R_1$ représente un radical de formule $A_1$ ou un radical de formule $A_2$ :

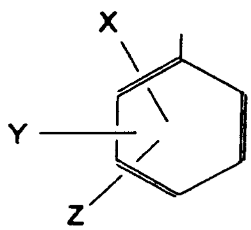

($A_1$)

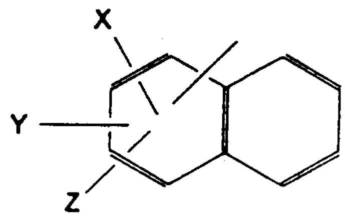

($A_2$)

(dans lesquelles X, Y, Z, identiques ou différents, représentent chacun un atome d'hydrogène, un atome d'halogène, un radical alcoxy de 1 à 5 atomes de carbone, un radical alkyle de 1 à 5 atomes de carbone, un radical hydroxy, un radical cyano, un radical nitro, un radical trifluorométhyle ou un radical aralkyle de 7 à 9 atomes de carbone),
- $R_2$ représente un atome d'hydrogène, un atome d'halogène, un radical alkyle de 1 à 5 atomes de carbone, un radical hydroxyméthyle ou un radical formyle,
- $R_3$ représente un radical alkyle de 1 à 5 atomes de carbone, un radical cycloalkyle de 3 à 8 atomes de carbone, un radical alcényle de 2 à 6 atomes de carbone, un radical cycloalkylalkyle de 4 à 8 atomes de carbone, ou un radical phényle,
- $R_4$ représente un atome d'hydrogène, un radical alkyle de 1 à 5 atomes de carbone, un radical cycloalkyle de 3 à 6 atomes de carbone, ou un radical cycloalkyle alkyle de 4 à 8 atomes de carbone ayant une chaîne linéaire ou ramifiée,
- $R_5$ représente un radical alkyle de 1 à 5 atomes de carbone, un radical cycloalkyle de 3 à 8 atomes de carbone éventuellement substitué par des radicaux alkyle de 1 à 5 atomes de carbone, un radical cycloalkylalkyle de 4 à 8 atomes de carbone ayant une chaîne linéaire ou ramifiée, un radical alcényle de 2 à 6 atomes de carbone, ou un radical de formule B :

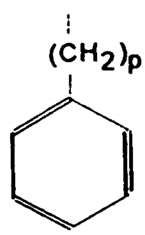

(B)

(dans laquelle p est égal à 0, 1, 2, ou 3),
- $R_6$ représente un radical phényle, un radical pyridyle, un radical imidazolyle, un radical pyrrolyle, un radical thiényle ou un radical furyle (éventuellement substitués par un ou plusieurs atomes d'halogène, par des radicaux alcoxy de 1 à 5 atomes de carbone, par des radicaux alkyle de 1 à 5 atomes de carbone, par des radicaux hydroxy, par des radicaux cyano, par des radicaux nitro, par des radicaux trifluorométhyle par des radicaux

méthylthio ou par des radicaux de formule B), ou un radical cycloalkyle de 3 à 8 atomes de carbone éventuellement substitué par des radicaux alkyle de 1 à 5 atomes de carbone,

- m, n, identiques ou différents représentent chacun 0 ou 1,

leurs stéréoisomères possibles et leurs sels d'addition à un acide minéral ou organique,
caractérisé en ce que l'on fait réagir un dérivé carbonylé alpha-halogéné de formule II :

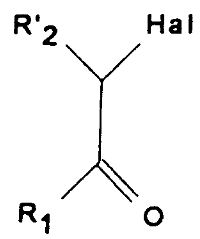

(II)

dans laquelle,
$R_1$ a la même signification que pour la formule I, selon la revendication 1 et $R'_2$ représente un atome d'hydrogène ou un radical alkyle de 1 à 5 atomes de carbone, et Hal représente un atome d'halogène,
soit
avec une thiourée de formule III :

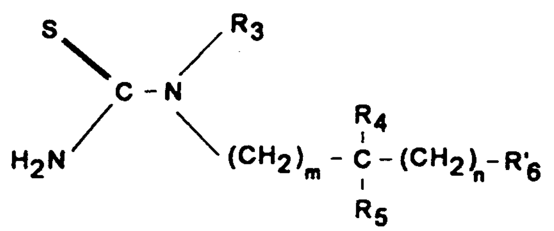

(III)

dans laquelle $R_3$, $R_4$, $R_5$, m et n, ont la même signification que pour la formule I, selon la revendication 1, et $R'_6$ a la signification donnée pour $R_6$ dans la formule I, selon la revendication 1, sauf dans le cas où $R_6$ contient des fonctions avec des atomes d'azote réactifs où $R'_6$ désigne alors le radical correspondant à $R_6$ dans lequel un hydrogène de ladite fonction réactive a été remplacée par un groupe protecteur résistant à l'hydrolyse en milieu alcalin,
pour former un composé de formule I' :

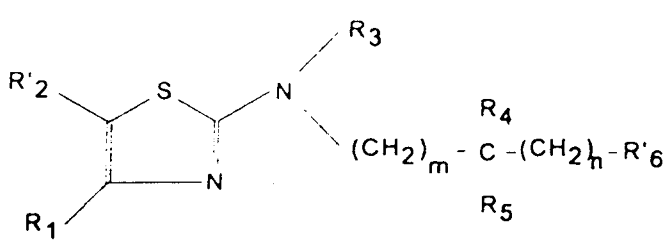

(I')

dans laquelle,

- $R_1$, $R_3$, $R_4$, $R_5$ m et n ont la même signification que pour la formule I, selon la revendication 1,
- $R'_2$ a la signification donnée pour la formule II,
  et,
- $R'_6$ a la signification donnée pour la formule III

<u>soit</u>

avec une thiourée de formule $III_A$ :

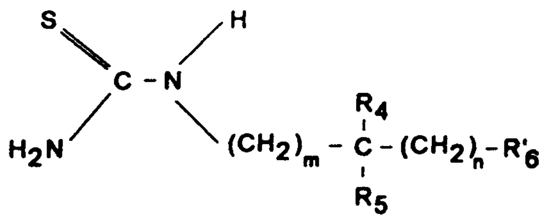

$(III_A)$

dans laquelle $R_4$, $R_5$, m et n ont la même signification que pour la formule I, selon la revendication 1, et $R'_6$ a la signification donnée pour la formule III,

pour former les composés de formule IV :

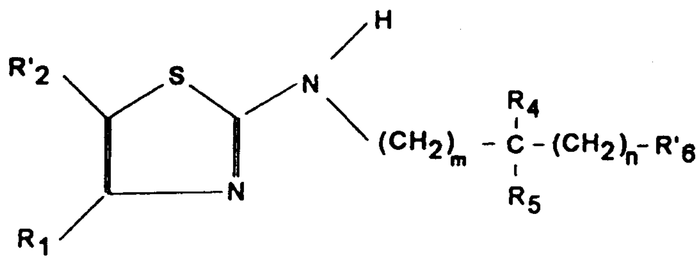

(IV)

dans laquelle $R_1$, $R_4$, $R_5$, m et n ont la même signification que pour la formule I, selon la revendication 1, $R'_2$ a la signification donnée pour la formule II et $R'_6$ a la signification donnée pour la formule III,

que l'on fait réagir avec un halogénure de formule V :

$$Hal - R_3 \qquad (V)$$

dans laquelle Hal représente un atome d'halogène et $R_3$ a la même signification que pour la formule I, selon la revendication 1,

pour former le composé de formule I',

<u>et ensuite,</u>

- l'on soumet les composés de formule I', dans laquelle $R'_2$ représente un atome d'hydrogène,

  * soit à l'action d'un halogène, pour former les composés de formule I, selon la revendication 1, dans laquelle $R_2$ représente un atome d'halogène, lesquels ensuite, lorsque $R_2$ représente un atome de brome, peuvent être soumis à l'action d'un autre halogène pour former les composés de formule I dans laquelle $R_2$ représente cet atome d'halogène,
  * soit à l'action de chlorure d'oxalyle pour préparer les composés de formule I, selon la revendication 1, dans laquelle $R_2$ représente un radical formyle, lesquels peuvent être soumis à une réduction pour obtenir les composés de formule I, selon la revendication 1, dans laquelle $R_2$ représente un radical hydroxyméthyle,

ou

- l'on soumet les composés de formule I' dans laquelle $R'_6$ représente un radical $R_6$ contenant des fonctions avec des atomes d'azote réactifs ayant un groupe protecteur, à une hydrolyse acide, pour obtenir les composés de formule I, selon la revendication 1, dans laquelle $R_6$ représente un radical contenant une amine primaire ou secondaire,

<u>et le cas échéant, les composés de formule I, selon la revendication 1,</u>

sont ensuite séparés en leurs stéréoisomères possibles et/ou salifiés par un acide organique ou minéral pour former les sels correspondants.

**10.** Utilisation des composés de formule I, selon l'une quelconque des revendications 1 à 8, pour la préparation d'un médicament utilisable dans le traitement des maladies nécessitant une modulation de l'action du facteur de libération de l'hormone corticotrope (CRF).

**11.** Composition pharmaceutique contenant comme principe actif un composé selon l'une quelconque des revendications 1 à 8, sous forme de base ou sous forme de sel avec un acide minéral ou organique pharmaceutiquement acceptable, en association ou en mélange avec un excipient inerte, non toxique, pharmaceutiquement acceptable.

**12.** Composés de formule IV :

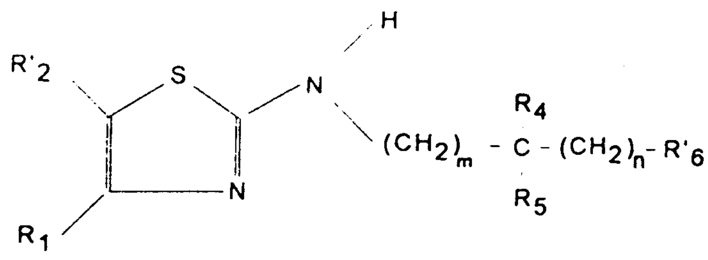

(IV)

dans laquelle,

- $R_1$, $R_4$, m et n ont la même signification que pour la formule I, selon la revendication 1,
- $R'_2$ représente un atome d'hydrogène ou un radical alkyle de 1 à 5 atomes de carbone,
- $R_5$ représente un radical alkyle de 1 à 5 atomes de carbone, un radical cycloalkyle de 3 à 8 atomes de carbone éventuellement substitué par des radicaux alkyle de 1 à 5 atomes de carbone, un radical cycloalkylalkyle de 4 à 8 atomes de carbone ayant une chaîne linéaire ou ramifiée, un radical alcényle de 2 à 6 atomes de carbone, ou un radical de formule B :

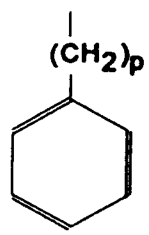

**(B)**

(dans laquelle p est égal à 1, 2, ou 3),

et

- $R'_6$ a la signification donnée pour $R_6$ dans la formule I, selon la revendication 1, sauf dans le cas où $R_6$ contient des fonctions avec des atomes d'azote réactifs où $R'_6$ désigne alors le radical correspondant à $R_6$ dans lequel un hydrogène de ladite fonction réactive a été remplacée par un groupe protecteur résistant à l'hydrolyse en milieu alcalin.

**13.** Procédé selon la revendication 9, cractérisé en ce que l'on prépare un composé de formule I dans laquelle :

- $R_1$ représente un radical de formule $A_1$,
- $R_2$ représente un atome d'halogène ou un radical alkyle de 1 à 5 atomes de carbone,
- $R_3$ représente un radical alkyle de 1 à 5 atomes de carbone, un radical cycloalkyle de 3 à 8 atomes de carbone, ou un radical alcényle de 2 à 6 atomes de carbone,
- $R_5$ représente un radical alkyle de 1 à 5 atomes de carbone, un radical cycloalkyle de 3 à 8 atomes de carbone, éventuellement substitué par des radicaux alkyle de 1 à 5 atomes de carbone, ou un radical cycloalkyle de 4 à 8 atomes de carbone ayant une chaîne linéaire ou ramifiée et,
- $R_4$, $R_6$, m et n, ont la même signification que dans la formule I, selon la revendication 1,

ou l'un de ses stéréoisomères ou sels d'addition à un acide minéral ou organique.

**14.** Procédé selon la revendication 9, caractérisé en ce que l'on prépare un composé de formule $I_A$

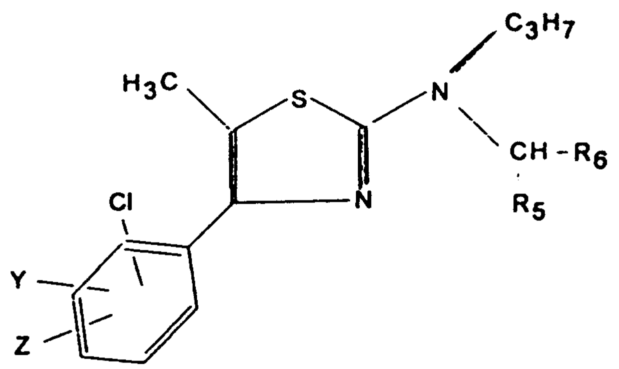

($I_A$)

dans laquelle,

- Y et Z ont la même signification que pour la formule I, selon la revendication 1,
- $R_5$ représente un radical alkyle de 1 à 5 atomes de carbone ou un radical cycloalkyle de 3 à 8 atomes de carbone, éventuellement substitué par des radicaux alkyle de 1 à 5 atomes de carbone ou un radical cycloalkylalkyle de 4 à 8 atomes de carbone ayant une chaîne linéaire ou ramifiée,
- $R_6$ représente un radical phényle ou un radical pyridyle (éventuellement substitué par un ou plusieurs atomes d'halogène, des radicaux alcoxy de 1 à 5 atomes de carbone ou par des radicaux alkyle de 1 à 5 atomes de carbone, des radicaux hydroxy, des radicaux cyano, des radicaux nitro, des radicaux trifluorométhyle ou des radicaux méthylthio), un radical imidazolyle, éventuellement substitué par un radical alkyle de 1 à 5 atomes de carbone, ou un radical cycloalkyle de 3 à 8 atomes de carbone, éventuellement sustitué par des radicaux alkyle de 1 à 5 atomes de carbone,

ou l'un de ses stéréoisomères ou sels d'addition à un acide minéral ou organique.

**15.** Procédé selon la revendication 9, caractérisé en ce que l'on prépare :

- le 4-(2,4-dichlorophényl) 5-méthyl-2-[N-(cyclopropylpyrid-4-ylméthyl) N-propylamino]thiazole,
- le 4-(2,4-dichlorophényl) 5-méthyl-2-[N-dicyclopropylméthyl) N-propyl amino]thiazole,
- le 4-(2,4-dichlorophényl) 5-méthyl-2-[N-($\alpha$-cyclopropylbenzyl) N-propyl amino] thiazole,
- le 4-(2,4-dichlorophényl) 5-méthyl-2-[N-(cyclopentylcyclopropylméthyl) N-propylamino]thiazole,
- le 4-(2-chloro-4-méthylphényl) 5-méthyl-2-[N-(dicyclopropylméthyl) N-propylamino] thiazole,

ou l'un de leurs sels d'addition à un acide minéral ou organique.

## Claims

**Claims for the following Contracting States : AT, BE, CH, DE, DK, FR, GB, GR, IE, IT, LI, LU, MC, NL, PT, SE**

**1.** Compounds of formula I:

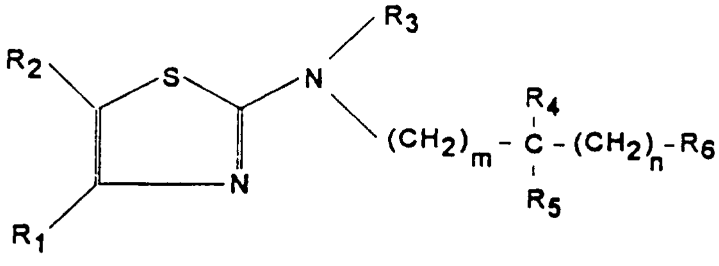

(I)

wherein

- $R_1$ denotes a radical of formula $A_1$ or a radical of formula $A_2$:

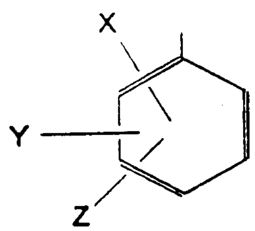

(A₁)

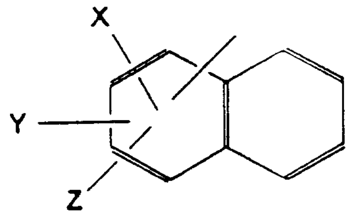

(A₂)

(wherein X, Y and Z, which may be identical or different, each denote a hydrogen atom, a halogen atom, a $C_{1-5}$-alkoxy radical, a $C_{1-5}$-alkyl radical, a hydroxy radical, a cyano radical, a nitro radical, a trifluoromethyl radical or a $C_{7-9}$-aralkyl radical),

- $R_2$ denotes a hydrogen atom, a halogen atom, a $C_{1-5}$-alkyl radical, a hydroxy methyl radical or a formyl radical,
- $R_3$ denotes a $C_{1-5}$-alkyl radical, a $C_{3-8}$-cycloalkyl radical, a $C_{2-6}$-alkenyl radical, a $C_{4-8}$-cycloalkylalkyl radical or a phenyl radical,
- $R_4$ denotes a hydrogen atom, a $C_{1-5}$-alkyl radical, a $C_{3-6}$-cycloalkyl radical or a $C_{4-8}$-cycloalkylalkyl radical having a straight or branched chain,
- $R_5$ denotes a $C_{1-5}$-alkyl radical, a $C_{3-8}$-cycloalkyl radical optionally substituted by $C_{1-5}$-alkyl radicals, a $C_{4-8}$-cycloalkylalkyl radical having a straight or branched chain, a $C_{2-6}$-alkenyl radical or a radical of formula B:

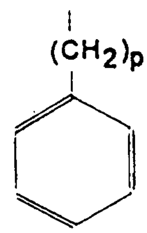

(B)

(wherein p is equal to 0, 1, 2 or 3),

- $R_6$ denotes a phenyl radical, a pyridyl radical, an imidazolyl radical, a pyrrolyl radical, a thienyl radical or a furyl radical (optionally substituted by one or more halogen atoms, by $C_{1-5}$-alkoxy radicals, by $C_{1-5}$-alkyl radicals, by hydroxy radicals, by cyano radicals, by nitro radicals, by trifluoromethyl radicals, by methylthio radicals or by radicals of formula B), or a $C_{3-8}$-cycloalkyl radical optionally substituted by $C_{1-5}$-alkyl radicals,
- m and n, which may be identical or different, each denote 0 or 1,

the possible stereoisomers and the addition salts thereof with an inorganic or organic acid.

**2.** Compounds of formula I according to claim 1, wherein

- $R_1$ denotes a radical of formula $A_1$,
- $R_2$ denotes a halogen atom or a $C_{1-5}$-alkyl radical,
- $R_3$ denotes a $C_{1-5}$-alkyl radical, a $C_{3-8}$-cycloalkyl radical or a $C_{2-6}$-alkenyl radical,
- $R_5$ denotes a $C_{1-5}$-alkyl radical, a $C_{3-8}$-cycloalkyl radical optionally substituted by $C_{1-5}$-alkyl radicals, or a $C_{4-8}$-cycloalkylalkyl radical having a straight or branched chain and
- $R_4$, $R_6$, m and n have the same meanings as in formula I according to claim 1,

the stereoisomers thereof and the addition salts thereof with an inorganic or organic acid.

**3.** Compounds of formula I according to claim 1 corresponding to formula $I_A$:

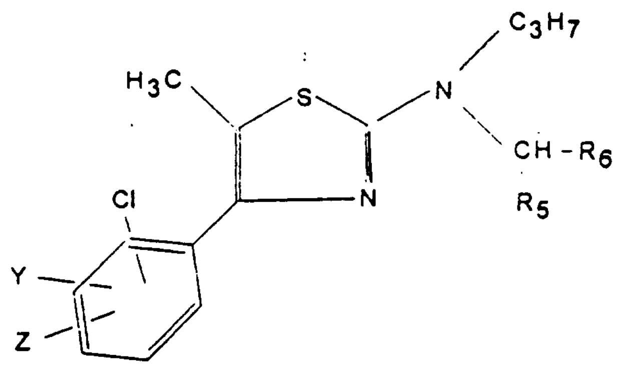

$(I_A)$

wherein

- Y and Z have the same meanings as in formula I according to claim 1,
- $R_5$ denotes a $C_{1-5}$-alkyl radical or a $C_{3-8}$-cycloalkyl radical optionally substituted by $C_{1-5}$-alkyl radicals or a $C_{4-8}$-cycloalkylalkyl radical having a straight or branched chain,
- $R_6$ denotes a phenyl radical or a pyridyl radical (optionally substituted by one or more halogen atoms, $C_{1-5}$-alkoxy radicals or $C_{1-5}$-alkyl radicals, hydroxy radicals, cyano radicals, nitro radicals, trifluoromethyl radicals or methylthio radicals), an imidazolyl radical optionally substituted by a $C_{1-5}$-alkyl radical, or a $C_{3-8}$-cycloalkyl radical optionally substituted by $C_{1-5}$-alkyl radicals,

the stereoisomers thereof and the addition salts thereof with an inorganic or organic acid.

**4.** 4-(2,4-dichlorophenyl)-5-methyl-2-[N-(cyclopropylpyrid-4-yl-methyl)-N-propylamino]thiazole, a compound according to formula I, according to claim 1, and the addition salts thereof with an inorganic or organic acid.

**5.** 4-(2,4-dichlorophenyl)-5-methyl-2-[N-(α-cyclopropyl-benzyl)-N-propylamino]thiazole, a compound according to formula I, according to claim 1, and the addition salts thereof with an inorganic or organic acid.

**6.** 4-(2,4-dichlorophenyl)-5-methyl-2-[N-dicyclopropylmethyl)-N-propylamino]thiazole, a compound according to formula I, according to claim 1, and the addition salts thereof with an inorganic or organic acid.

**7.** 4-(2,4-dichlorophenyl)-5-methyl-2-[N-(cyclopentylcyclopropyl-methyl)-N-propylamino]thiazole, a compound according to formula I, according to claim 1, and the addition salts thereof with an inorganic or organic acid.

**8.** 4-(2-chloro-4-methylphenyl)-5-methyl-2-[N-(dicyclopropyl-methyl)-N-propylamino] thiazole, a coumpound according to formula I, according to claim 1, and the addition salts thereof with an inorganic or organic acid.

**9.** Process for preparing compounds of formula I according to claim 1, characterised in that an alpha-halogenated carbonyl derivative of formula II:

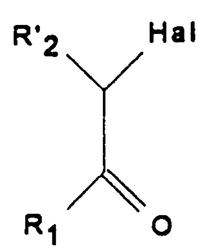

(II)

wherein

$R_1$ has the same meaning as in formula I according to claim 1 and $R'_2$ denotes a hydrogen atom or a $C_{1-5}$-alkyl radical and Hal denotes a halogen atom, is reacted <u>either</u>

with a thiourea of formula III:

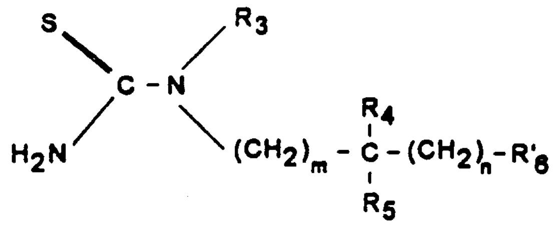

(III)

wherein $R_3$, $R_4$, $R_5$, m and n have the same meanings as in formula I according to claim 1 and $R'_6$ has the meaning given for $R_6$ in formula I according to claim 1, except where $R_6$ contains functions with reactive nitrogen atoms wherein $R'_6$ then denotes the radical corresponding to $R_6$ in which a hydrogen of said reactive function has been replaced by a protecting group which is resistant to hydrolysis in an alkaline medium,

in order to form a compound of formula I':

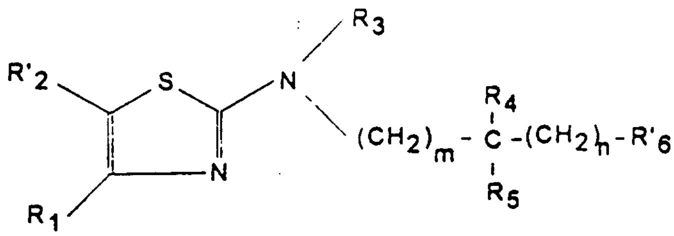

(I')

wherein

- $R_1$, $R_3$, $R_4$, $R_5$, m and n have the same meanings as in formula I according to claim 1,
- $R'_2$ has the meaning given for formula II and
- $R'_6$ has the meaning given for formula III

or

with a thiourea of formula $III_A$:

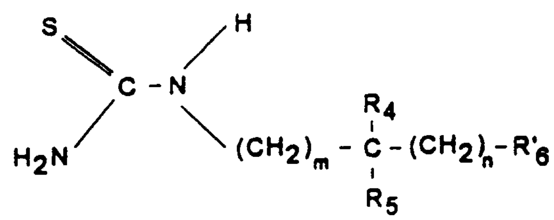

($III_A$)

wherein $R_4$, $R_5$, m and n have the same meaning as in formula I according to claim 1 and $R'_6$ has the meaning given in formula III,

to form the compounds of formula IV:

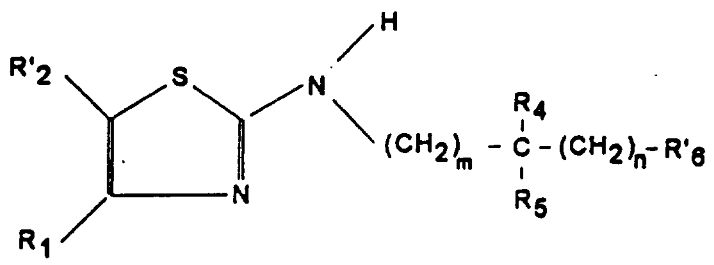

(IV)

wherein $R_1$, $R_4$, $R_5$, m and n have the same meaning as in formula I according to claim 1, $R'_2$ has the meaning given for formula II and $R'_6$ has the meaning given for formula III,

which are reacted with a halide of formula V:

$$Hal\text{-}R_3 \qquad (V)$$

wherein Hal denotes a halogen atom and $R_3$ has the same meaning as in formula I according to claim 1, to form the compound of formula I',

<u>and then</u>

- the compounds of formula I' wherein $R'_2$ denotes a hydrogen atom are reacted
    * either with a halogen, to form the compounds of formula I according to claim 1 wherein $R_2$ denotes a halogen atom, which may then, if $R_2$ denotes a bromine atom, be reacted with another halogen to form the compounds of formula I wherein $R_2$ denotes this halogen atom,
    * or with oxalyl chloride to prepare the compounds of formula I according to claim 1 wherein $R_2$ denotes a formyl radical, which may be subjected to reduction in order to obtain the compounds of formula I according to claim 1 wherein $R_2$ denotes a hydroxymethyl radical,

  or

- the compounds of formula I' wherein $R'_6$ denotes a radical $R_6$ containing functions with reactive nitrogen atoms having a protecting group are subjected to acid hydrolysis, to obtain the compounds of formula I according to claim 1 wherein $R_6$ denotes a radical containing a primary or secondary amine,

<u>and if necessary, the compounds of formula I according to claim 1</u>

are subsequently separated into their possible stereoisomers and/or salified with an organic or inorganic acid to form the corresponding salts.

**10.** Use of the compounds of formula I according to any one of claims 1 to 8 for the preparation of a drug which can be used in the treatment of diseases requiring modification of the action of corticotrophin releasing factor (CRF).

**11.** Pharmaceutical composition containing as active principle a compound according to any one of claims 1 to 8 in the form of a base or in the form of a salt with a pharmaceutically acceptable inorganic or organic acid, in conjunction or admixture with a pharmaceutically acceptable non-toxic inert excipient.

**12.** Compounds of formula IV:

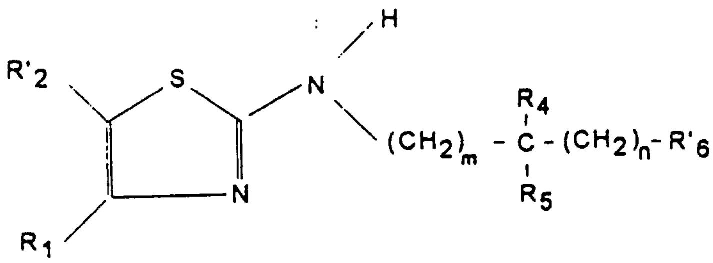

(IV)

wherein

- $R_1$, $R_4$, m and n have the same meanings as in formula I according to claim 1,
- $R'_2$ denotes a hydrogen atom or a $C_{1-5}$-alkyl radical,
- $R_5$ denotes a $C_{1-5}$-alkyl radical, a $C_{3-8}$-cycloalkyl radical optionally substituted by $C_{1-5}$-alkyl radicals, a $C_{4-8}$-cycloalkylalkyl radical having a straight or branched chain, a $C_{2-6}$-alkenyl radical or a radical of formula B:

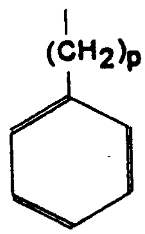

(B)

(wherein p is equal to 1, 2 or 3)

and

- $R'_6$ has the meaning given for $R_6$ in formula I according to claim 1, except where $R_6$ contains functions with reactive nitrogen atoms wherein $R'_6$ then denotes the radical corresponding to $R_6$ in which a hydrogen of said reactive function has been replaced by a protecting group resistant to hydrolysis in an alkaline medium.

**Claims for the following Contracting State : ES**

1. Compounds of formula I:

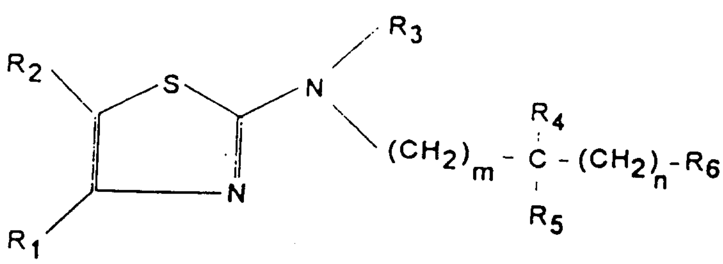

(I) (I)

wherein

- $R_1$ denotes a radical of formula $A_1$ or a radical of formula $A_2$:

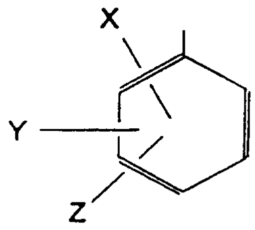

($A_1$)

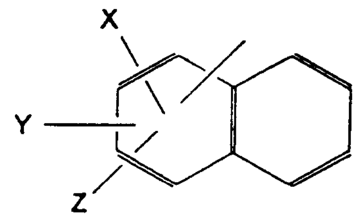

($A_2$)

(wherein X, Y and Z, which may be identical or different, each denote a hydrogen atom, a halogen atom, a $C_{1-5}$-alkoxy radical, a $C_{1-5}$-alkyl radical, a hydroxy radical, a cyano radical, a nitro radical, a trifluoromethyl radical or a $C_{7-9}$-aralkyl radical),

- $R_2$ denotes a hydrogen atom, a halogen atom, a $C_{1-5}$-alkyl radical, a hydroxy methyl radical or a formyl radical,
- $R_3$ denotes a $C_{1-5}$-alkyl radical, a $C_{3-8}$-cycloalkyl radical, a $C_{2-6}$-alkenyl radical, a $C_{4-8}$-cycloalkylalkyl radical or a phenyl radical,
- $R_4$ denotes a hydrogen atom, a $C_{1-5}$-alkyl radical, a $C_{3-6}$-cycloalkyl radical or a $C_{4-8}$-cycloalkylalkyl radical having a straight or branched chain,
- $R_5$ denotes a $C_{1-5}$-alkyl radical, a $C_{3-8}$-cycloalkyl radical optionally substituted by $C_{1-5}$-alkyl radicals, a $C_{4-8}$-cycloalkylalkyl radical having a straight or branched chain, a $C_{2-6}$-alkenyl radical or a radical of formula B:

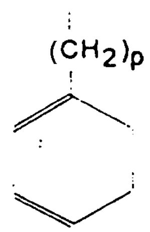

(B)

(wherein p is equal to 0, 1, 2 or 3),

- $R_6$ denotes a phenyl radical, a pyridyl radical, an imidazolyl radical, a pyrrolyl radical, a thienyl radical or a furyl radical (optionally substituted by one or more halogen atoms, by $C_{1-5}$-alkoxy radicals, by $C_{1-5}$-alkyl radicals, by hydroxy radicals, by cyano radicals, by nitro radicals, by trifluoromethyl radicals, by methylthio radicals or by radicals of formula B), or a $C_{3-8}$-cycloalkyl radical optionally substituted by $C_{1-5}$-alkyl radicals,
- m and n, which may be identical or different, each denote 0 or 1,

the possible stereoisomers and the addition salts thereof with an inorganic or organic acid.

2. Compounds of formula I according to claim 1, wherein

- $R_1$ denotes a radical of formula $A_1$,
- $R_2$ denotes a halogen atom or a $C_{1-5}$-alkyl radical,
- $R_3$ denotes a $C_{1-5}$-alkyl radical, a $C_{3-8}$-cycloalkyl radical or a $C_{2-6}$-alkenyl radical,
- $R_5$ denotes a $C_{1-5}$-alkyl radical, a $C_{3-8}$-cycloalkyl radical optionally substituted by $C_{1-5}$-alkyl radicals, or a $C_{4-8}$-cycloalkylalkyl radical having a straight or branched chain and
- $R_4$, $R_6$, m and n have the same meanings as in formula I according to claim 1,

the stereoisomers thereof and the addition salts thereof with an inorganic or organic acid.

3. Compounds of formula I according to claim 1 corresponding to formula $I_A$:

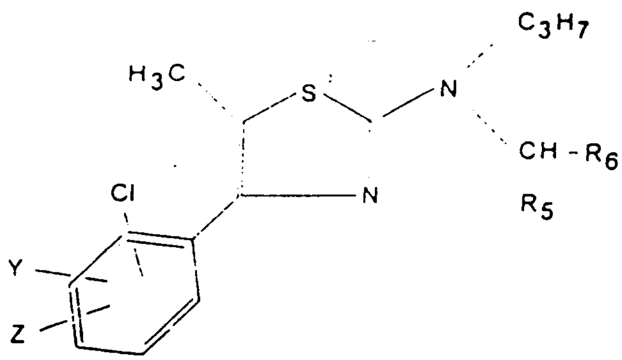

($I_A$)

wherein

- Y and Z have the same meanings as in formula I according to claim 1,
- $R_5$ denotes a $C_{1-5}$-alkyl radical or a $C_{3-8}$-cycloalkyl radical optionally substituted by $C_{1-5}$-alkyl radicals or a $C_{4-8}$-cycloalkylalkyl radical having a straight or branched chain,
- $R_6$ denotes a phenyl radical or a pyridyl radical (optionally substituted by one or more halogen atoms, $C_{1-5}$-alkoxy radicals or $C_{1-5}$-alkyl radicals, hydroxy radicals, cyano radicals, nitro radicals, trifluoromethyl radicals or methylthio radicals), an imidazolyl radical optionally substituted by a $C_{1-5}$-alkyl radical, or a $C_{3-8}$-cycloalkyl radical optionally substituted by $C_{1-5}$-alkyl radicals,

the stereoisomers thereof and the addition salts thereof with an inorganic or organic acid.

**4.** 4-(2,4-dichlorophenyl)-5-methyl-2-[N-(cyclopropylpyrid-4-yl-methyl)-N-propylamino]thiazole, a compound according to formula I, according to claim 1, and the addition salts thereof with an inorganic or organic acid.

**5.** 4-(2,4-dichlorophenyl)-5-methyl-2-[N-(α-cyclopropyl-benzyl)-N-propylamino]thiazole, a compound according to formula I, according to claim 1, and the addition salts thereof with an inorganic or organic acid.

**6.** 4-(2,4-dichlorophenyl)-5-methyl-2-[N-dicyclopropylmethyl)-N-propylamino]thiazole, a compound according to formula I, according to claim 1, and the addition salts thereof with an inorganic or organic acid.

**7.** 4-(2,4-dichlorophenyl)-5-methyl-2-[N-(cyclopentyl-cyclopropyl-methyl)-N-propylamino]thiazole, a compound according to formula I, according to claim 1, and the addition salts thereof with an inorganic or organic acid.

**8.** 4-(2-chloro-4-methylphenyl)-5-methyl-2-[N-(dicyclopropyl-methyl)-N-propylamino] thiazole, a coumpound according to formula I, according to claim 1, and the addition salts thereof with an inorganic or organic acid.

**9.** Process for preparing compounds of formula I:

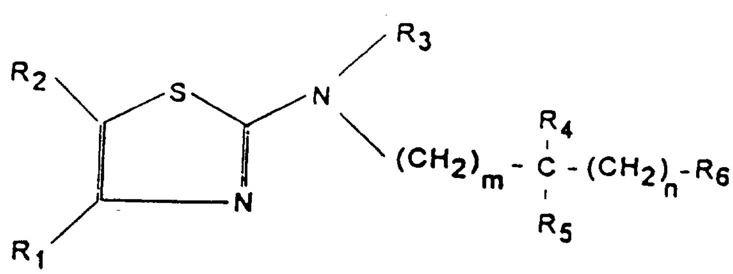

(I)

wherein

- $R_1$ denotes a radical of formula $A_1$ or a radical of formula $A_2$:

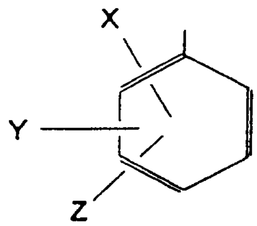

($A_1$)

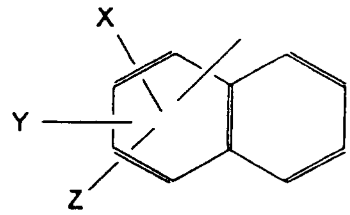

($A_2$)

(wherein X, Y and Z, which may be identical or different, each denote a hydrogen atom, a halogen atom, a $C_{1-5}$-alkoxy radical, a $C_{1-5}$-alkyl radical, a hydroxy radical, a cyano radical, a nitro radical, a trifluoromethyl radical or a $C_{7-9}$-aralkyl radical),

- $R_2$ denotes a hydrogen atom, a halogen atom, a $C_{1-5}$-alkyl radical, a hydroxy methyl radical or a formyl radical,
- $R_3$ denotes a $C_{1-5}$-alkyl radical, a $C_{3-8}$-cycloalkyl radical, a $C_{2-6}$-alkenyl radical, a $C_{4-8}$-cycloalkylalkyl radical or a phenyl radical,
- $R_4$ denotes a hydrogen atom, a $C_{1-5}$-alkyl radical, a $C_{3-6}$-cycloalkyl radical or a $C_{4-8}$-cycloalkylalkyl radical having a straight or branched chain,
- $R_5$ denotes a $C_{1-5}$-alkyl radical, a $C_{3-8}$-cycloalkyl radical optionally substituted by $C_{1-5}$-alkyl radicals, a $C_{4-8}$-cycloalkylalkyl radical having a straight or branched chain, a $C_{2-6}$-alkenyl radical or a radical of formula B:

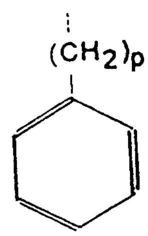

(B)

(wherein p is equal to 0, 1, 2 or 3),

- $R_6$ denotes a phenyl radical, a pyridyl radical, an imidazolyl radical, a pyrrolyl radical, a thienyl radical or a furyl radical (optionally substituted by one or more halogen atoms, by $C_{1-5}$-alkoxy radicals, by $C_{1-5}$-alkyl radicals, by hydroxy radicals, by cyano radicals, by nitro radicals, by trifluoromethyl radicals, by methylthio radicals or by radicals of formula B), or a $C_{3-8}$-cycloalkyl radical optionally substituted by $C_{1-5}$-alkyl radicals,
- m and n, which may be identical or different, each denote 0 or 1,

the possible stereoisomers and the addition salts thereof with an inorganic or organic acid, characterised in that an alpha-halogenated carbonyl derivative of formula II:

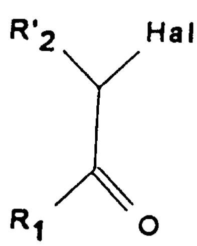

(II)

wherein

$R_1$ has the same meaning as in formula I according to claim 1 and $R'_2$ denotes a hydrogen atom or a $C_{1-5}$-alkyl radical and Hal denotes a halogen atom, is reacted

<u>either</u>

with a thiourea of formula III:

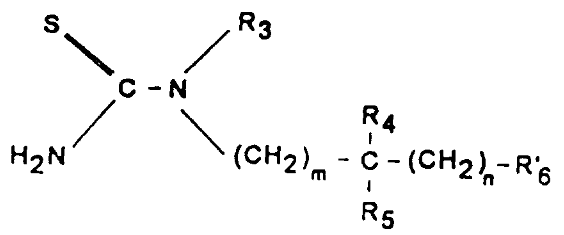

(III)

wherein $R_3$, $R_4$, $R_5$, m and n have the same meanings as in formula I according to claim 1 and $R'_6$ has the meaning given for $R_6$ in formula I according to claim 1, except where $R_6$ contains functions with reactive nitrogen atoms wherein $R'_6$ then denotes the radical corresponding to $R_6$ in which a hydrogen of said reactive function has been replaced by a protecting group which is resistant to hydrolysis in an alkaline medium,

in order to form a compound of formula I':

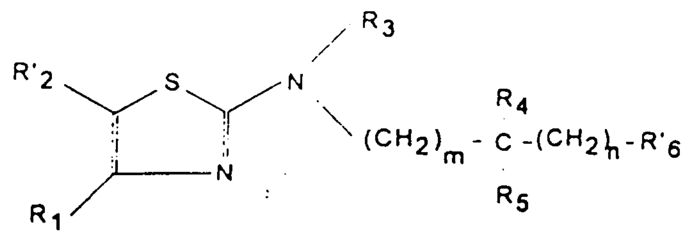

(I')

wherein

- $R_1$, $R_3$, $R_4$, $R_5$, m and n have the same meanings as in formula I according to claim 1,
- $R'_2$ has the meaning given for formula II and
- $R'_6$ has the meaning given for formula III

or

with a thiourea of formula $III_A$:

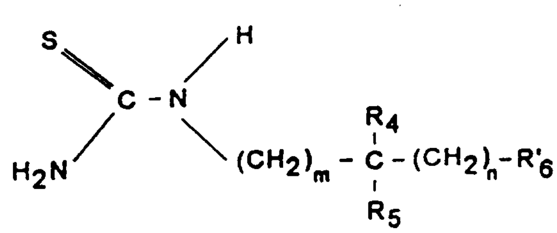

($III_A$)

wherein $R_4$, $R_5$, m and n have the same meaning as in formula I according to claim 1 and $R'_6$ has the meaning given in formula III,

to form the compounds of formula IV:

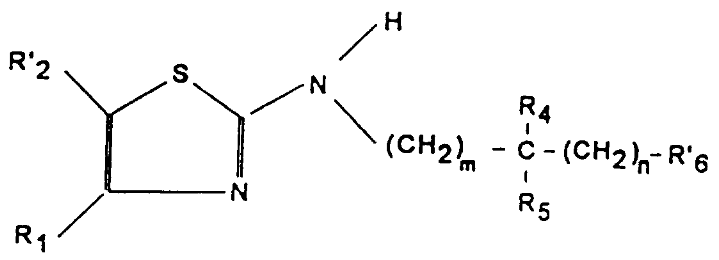

(IV)

wherein $R_1$, $R_4$, $R_5$, m and n have the same meaning as in formula I according to claim 1, $R'_2$ has the meaning given for formula II and $R'_6$ has the meaning given for formula III,
which are reacted with a halide of formula V:

$$Hal\text{-}R_3 \qquad (V)$$

wherein Hal denotes a halogen atom and $R_3$ has the same meaning as in formula I according to claim 1, to form the compound of formula I',
and then

- the compounds of formula I' wherein $R'_2$ denotes a hydrogen atom are reacted

  * either with a halogen, to form the compounds of formula I according to claim 1 wherein $R_2$ denotes a halogen atom, which may then, if $R_2$ denotes a bromine atom, be reacted with another halogen to form the compounds of formula I wherein $R_2$ denotes this halogen atom,
  * or with oxalyl chloride to prepare the compounds of formula I according to claim 1 wherein $R_2$ denotes a formyl radical, which may be subjected to reduction in order to obtain the compounds of formula I according to claim 1 wherein $R_2$ denotes a hydroxymethyl radical,

or

- the compounds of formula I' wherein $R'_6$ denotes a radical $R_6$ containing functions with reactive nitrogen atoms having a protecting group are subjected to acid hydrolysis, to obtain the compounds of formula I according to claim 1 wherein $R_6$ denotes a radical containing a primary or secondary amine,

and if necessary, the compounds of formula I according to claim 1 are subsequently separated into their possible stereoisomers and/or salified with an organic or inorganic acid to form the corresponding salts.

**10.** Use of the compounds of formula I according to any one of claims 1 to 8 for the preparation of a drug which can be used in the treatment of diseases requiring modification of the action of corticotrophin releasing factor (CRF).

**11.** Pharmaceutical composition containing as active principle a compound according to any one of claims 1 to 8 in the form of a base or in the form of a salt with a pharmaceutically acceptable inorganic or organic acid, in conjunction or admixture with a pharmaceutically acceptable non-toxic inert excipient.

**12.** Compounds of formula IV:

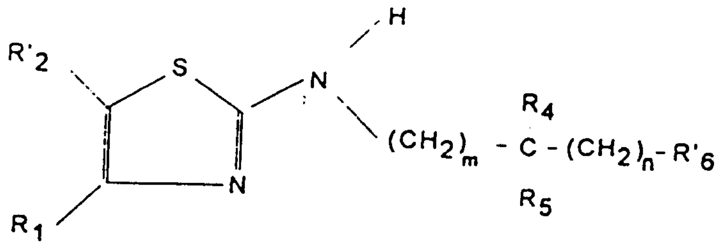

(IV)

wherein

- $R_1$, $R_4$, m and n have the same meanings as in formula I according to claim 1,
- $R'_2$ denotes a hydrogen atom or a $C_{1-5}$-alkyl radical,
- $R_5$ denotes a $C_{1-5}$-alkyl radical, a $C_{3-8}$-cycloalkyl radical optionally substituted by $C_{1-5}$-alkyl radicals, a $C_{4-8}$-cycloalkylalkyl radical having a straight or branched chain, a $C_{2-6}$-alkenyl radical or a radical of formula B:

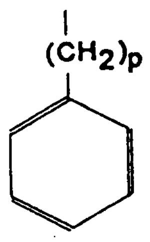

(B)

(wherein p is equal to 1, 2 or 3)

and

- $R'_6$ has the meaning given for $R_6$ in formula I according to claim 1, except where $R_6$ contains functions with reactive nitrogen atoms wherein $R'_6$ then denotes the radical corresponding to $R_6$ in which a hydrogen of said reactive function has been replaced by a protecting group resistant to hydrolysis in an alkaline medium.

**13.** Process according to claim 9, characterized in that a compound of formula I is prepared wherein:

- $R_1$ denotes a radical of formula $A_1$,
- $R_2$ denotes a halogen atom or a $C_{1-5}$-alkyl radical,
- $R_3$ denotes a $C_{1-5}$-alkyl radical, a $C_{3-8}$-cycloalkyl radical or a $C_{2-6}$-alkenyl radical,
- $R_5$ denotes a $C_{1-5}$-alkyl radical, a $C_{3-8}$-cycloalkyl radical optionally substituted by $C_{1-5}$-alkyl radicals, or a $C_{4-8}$-cycloalkyl radical having a straight or branched chain, and
- $R_4$, $R_6$, m and n have the same meanings as in formula I according to claim 1,

or one of the stereoisomers or addition salts with an inorganic or organic acid.

**14.** Process according to claim 9, characterised in that a compound of formula $I_A$ is prepared

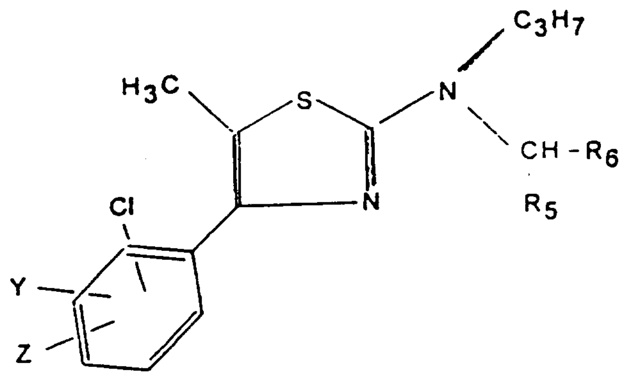

($I_A$)

wherein

- Y and Z have the same meanings as in formula I according to claim 1,
- $R_5$ denotes a $C_{1-5}$-alkyl radical or a $C_{3-8}$-cycloalkyl radical optionally substituted by $C_{1-5}$-alkyl radicals or a $C_{4-8}$-cycloalkylalkyl radical having a straight or branched chain,
- $R_6$ denotes a phenyl radical or a pyridyl radical (optionally substituted by one or more halogen atoms, $C_{1-5}$-alkoxy radicals or by $C_{1-5}$-alkyl radicals, hydroxy radicals, cyano radicals, nitro radicals, trifluoromethyl radicals or methylthio radicals), an imidazolyl radical optionally substituted by a $C_{1-5}$-alkyl radical, or a $C_{3-8}$-cycloalkyl radical optionally substituted by $C_{1-5}$-alkyl radicals,

or one of the stereoisomers thereof or addition salts with an inorganic or organic acid.

**15.** Process according to claim 9, characterised in that the following are prepared:

4-(2,4-dichlorophenyl)-5-methyl-2-[N-(cyclopropylpyrid-4-ylmethyl)-N-propylamino]thiazole,

4-(2,4-dichlorophenyl)-5-methyl-2-[N-dicyclopropylmethyl)-N-propylamino]thiazole,

4-(2,4-dichlorophenyl)-5-methyl-2-[N-(α-cyclopropylbenzyl)-N-propylamino]thiazole,

4-(2,4-dichlorophenyl)-5-methyl-2-[N-(cyclopentylcyclopropylmethyl)-N-propylamino]thiazole,

4-(2-chloro-4-methylphenyl)-5-methyl-2-[N-(dicyclopropylmethyl)-N-propylamino]thiazole,

or one of the addition salts thereof with an inorganic or organic acid.

**Patentansprüche**

**Patentansprüche für folgende Vertragsstaaten : AT, BE, CH, DE, DK, FR, GB, GR, IE, IT, LI, LU, MC, NL, PT, SE**

1. Verbindungen der Formel I:

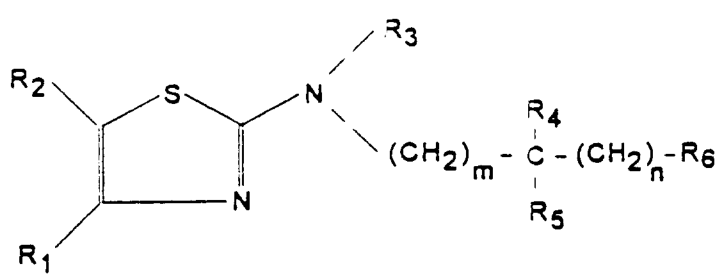

(I)

in der

- $R_1$ eine Gruppe der Formel $A_1$ oder eine Gruppe der Formel $A_2$:

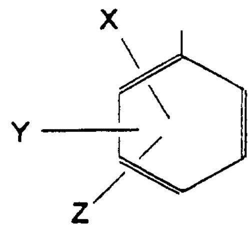

($A_1$)

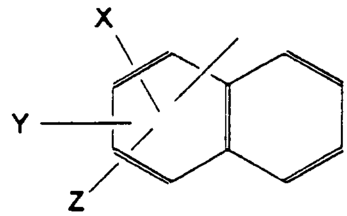

($A_2$)

(in denen X, Y und Z, die gleichartig oder verschieden sein können, jeweils ein Wasserstoffatom, ein Halogenatom, eine Alkoxygruppe mit 1 bis 5 Kohlenstoffatomen, eine Alkylgruppe mit 1 bis 5 Kohlenstoffatomen, eine Hydroxylgruppe, eine Cyanogruppe, eine Nitrogruppe, eine Trifluormethylgruppe oder eine Aralkylgruppe mit 7 bis 9 Kohlenstoffatomen darstellen),
- $R_2$ ein Wasserstoffatom, ein Halogenatom, eine Alkylgruppe mit 1 bis 5 Kohlenstoffatomen, eine Hydroxymethylgruppe oder eine Formylgruppe,
- $R_3$ eine Alkylgruppe mit 1 bis 5 Kohlenstoffatomen, eine Cycloalkylgruppe mit 3 bis 8 Kohlenstoffatomen, eine Alkenylgruppe mit 2 bis 6 Kohlenstoffatomen, eine Cycloalkylalkylgruppe mit 4 bis 8 Kohlenstoffatomen oder eine Phenylgruppe,
- $R_4$ ein Wasserstoffatom, eine Alkylgruppe mit 1 bis 5 Kohlenstoffatomen, eine Cycloalkylgruppe mit 3 bis 6 Kohlenstoffatomen oder eine Cycloalkylalkylgruppe mit 4 bis 8 Kohlenstoffatomen mit einer geraden oder verzweigten Kette,
- $R_5$ eine Alkylgruppe mit 1 bis 5 Kohlenstoffatomen, eine Cycloalkylgruppe mit 3 bis 8 Kohlenstoffatomen, welche gegebenenfalls mit Alkylgruppen mit 1 bis 5 Kohlenstoffatomen substituiert ist, eine Cycloalkylalkylgruppe mit 4 bis 8 Kohlenstoffatomen und einer geraden oder verzweigten Kette, eine Alkenylgruppe mit 2 bis 6 Kohlenstoffatomen oder einen Rest der Formel B:

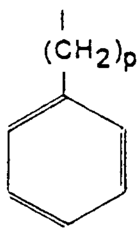

(B)

(in der p den Wert 0, 1, 2 oder 3 besitzt),
- $R_6$ eine Phenylgruppe, eine Pyridylgruppe, eine Imidazolylgruppe, eine Pyrrolylgruppe, eine Thienylgruppe oder eine Furylgruppe (die gegebenenfalls durch eines oder mehrere Halogenatome, Alkoxygruppen mit 1 bis 5 Kohlenstoffatomen, Alkylgruppen mit 1 bis 5 Kohlenstoffatomen, Hydroxylgruppen, Cyanogruppen, Nitrogruppen, Trifluormethylgruppen, Methylthiogruppen oder Gruppen der Formel B substituiert sind) oder eine Cycloalkylgruppe mit 3 bis 8 Kohlenstoffatomen, die gegebenenfalls mit Alkylgruppen mit 1 bis 5 Kohlenstoffatomen substituiert ist, und
- m und n, die gleich oder verschieden sein können, jeweils 0 oder 1 bedeuten,

deren mögliche Stereoisomeren und deren Additionssalze mit einer anorganischen oder organischen Säure.

**2.** Verbindungen der Formel I nach Anspruch 1, in der

- $R_1$ eine Gruppe der Formel $A_1$,
- $R_2$ ein Halogenatom oder eine Alkylgruppe mit 1 bis 5 Kohlenstoffatomen,
- $R_3$ eine Alkylgruppe mit 1 bis 5 Kohlenstoffatomen, eine Cycloalkylgruppe mit 3 bis 8 Kohlenstoffatomen oder eine Alkenylgruppe mit 2 bis 6 Kohlenstoffatomen,
- $R_5$ eine Alkylgruppe mit 1 bis 5 Kohlenstoffatomen, eine Cycloalkylgruppe mit 3 bis 8 Kohlenstoffatomen, die

gegebenenfalls durch Alkylgruppen mit 1 bis 5 Kohlenstoffatomen substituiert ist, oder eine Cycloalkylalkylgruppe mit 4 bis 8 Kohlenstoffatomen mit gerader oder verzweigter Kette bedeuten, und

- $R_4$, $R_6$, m und n die bezüglich der Formel I in Anspruch 1 angegebenen Bedeutungen besitzen,

deren Stereoisomere sowie deren Additionssalze mit einer anorganischen oder organischen Säure.

3. Verbindungen der Formel I nach Anspruch 1 der Formel $I_A$:

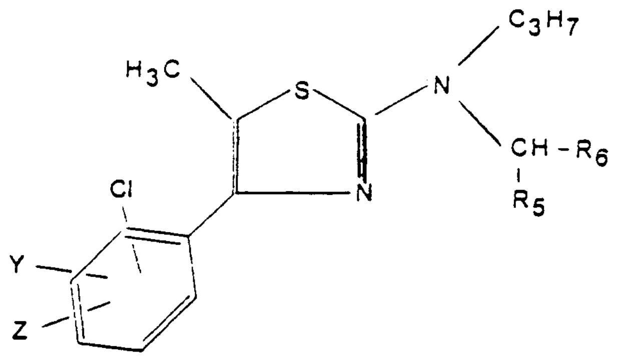

($I_A$)

in der

- Y und Z die bezüglich der Formel I in Anspruch 1 angegebenen Bedeutungen besitzen,
- $R_5$ eine Alkylgruppe mit 1 bis 5 Kohlenstoffatomen oder eine Cycloalkylgruppe mit 3 bis 8 Kohlenstoffatomen, die gegebenenfalls durch Alkylgruppen mit 1 bis 5 Kohlenstoffatomen substituiert ist, oder eine Cycloalkylalkylgruppe mit 4 bis 8 Kohlenstoffatomen mit gerader oder verzweigter Kette und
- $R_6$ eine Phenylgruppe oder eine Pyridylgruppe (die gegebenenfalls durch eines oder mehrere Halogenatome, Alkoxygruppen mit 1 bis 5 Kohlenstoffatomen oder Alkylgruppen mit 1 bis 5 Kohlenstoffatomen, Hydroxylgruppen, Cyanogruppen, Nitrogruppen, Trifluormethylgruppen oder Methylthiogruppen substituiert ist), eine Imidazolylgruppe, die gegebenenfalls durch eine Alkylgruppe mit 1 bis 5 Kohlenstoffatomen substituiert ist, oder eine Cycloalkylgruppe mit 3 bis 8 Kohlenstoffatomen, die gegebenenfalls durch Alkylgruppen mit 1 bis 5 Kohlenstoffatomen substituiert ist,

deren Stereoisomere oder deren Additionssalze mit einer anorganischen oder organischen Säure.

4. Verbindung der Formel I nach Anspruch 1, nämlich 4-(2,4-Dichlorphenyl)-5-methyl-2-[N-(cyclopropyl-pyrid-4-ylmethyl)-N-propylamino]-thiazol und dessen Additionssalze mit einer anorganischen oder organischen Säure.

5. Verbindung der Formel I nach Anspruch 1, nämlich 4-(2,4-Dichlorphenyl)-5-methyl-2-[N-(α-cyclopropylbenzyl)-N-propylamino]-thiazol und dessen Additionssalze mit einer anorganischen oder organischen Säure.

6. Verbindung der Formel I nach Anspruch 1, nämlich 4-(2,4-Dichlorphenyl)-5-methyl-2-[N-(dicyclopropylmethyl)-N-propylamino]-thiazol und dessen Additionssalze mit einer anorganischen oder organischen Säure.

7. Verbindung der Formel I nach Anspruch 1, nämlich 4-(2,4-Dichlorphenyl)-5-methyl-2-[N-(cyclopentyl-cyclopropylmethyl)-N-propylamino]-thiazol und dessen Additionssalze mit einer anorganischen oder organischen Säure.

8. Verbindung der Formel I nach Anspruch 1, nämlich 4-(2-Chlor-4-methylphenyl)-5-methyl-2-[N-(dicyclopropylmethyl)-N-propylamino]-thiazol und dessen Additionssalze mit einer anorganischen oder organischen Säure.

9. Verfahren zur Herstellung der Verbindungen der Formel I nach Anspruch 1, **dadurch gekennzeichnet**, daß man ein α-Halogencarbonyl-Derivat der Formel II:

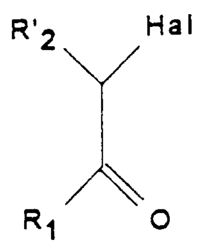

(II)

in der

$R_1$ die bezüglich der Formel I in Anspruch 1 angegebenen Bedeutungen besitzt und $R'_2$ ein Wasserstoffatom oder eine Alkylgruppe mit 1 bis 5 Kohlenstoffatomen und Hal ein Halogenatom bedeuten,

<u>entweder</u>

mit einem Thioharnstoff der Formel III umsetzt:

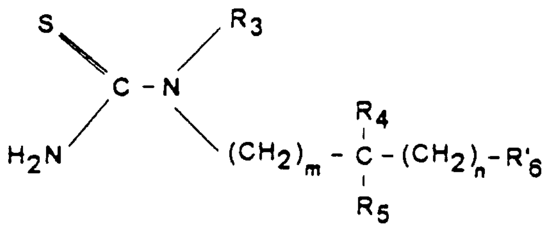

(III)

in der $R_3$, $R_4$, $R_5$, m und n die bezüglich der Formel I in Anspruch 1 angegebenen Bedeutungen besitzen und $R'_6$ die für $R_6$ bezüglich der Formel I in Anspruch 1 angegebenen Bedeutungen besitzt mit Ausnahme des Falls, bei dem $R_6$ Reste mit reaktiven Stickstoffatomen aufweist, in welchem Fall $R'_6$ die entsprechende Gruppe $R_6$ darstellt, in der ein Wasserstoffatom der reaktiven Funktion durch eine gegen die Hydrolyse in alkalischem Medium beständige Schutzgruppe ersetzt ist,
zur Bildung einer Verbindung der Formel I':

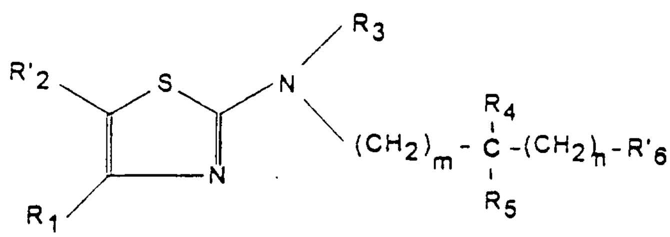

(I')

in der

- $R_1$, $R_3$, $R_4$, $R_5$, m und n die bezüglich der Formel I in Anspruch 1 angegebenen Bedeutungen besitzen,
- $R'_2$ die bezüglich der Formel II angegebenen Bedeutungen besitzt und
- $R'_6$ die bezüglich der Formel III angegebenen Bedeutungen besitzt,

<u>oder</u>

mit einem Thioharnstoff der Formel $III_A$ umsetzt:

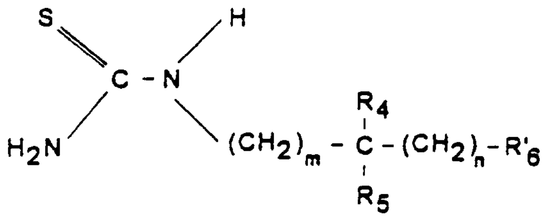

$(III_A)$

in der $R_4$, $R_5$, m und n die bezüglich der Formel I in Anspruch 1 angegebenen Bedeutungen besitzen und $R'_6$ die bezüglich der Formel III angegebenen Bedeutungen aufweist,

zur Bildung der Verbindungen der Formel IV:

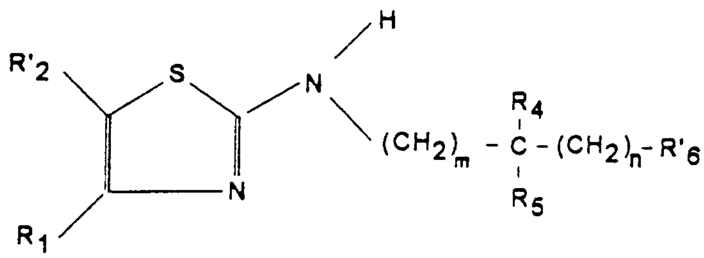

(IV)

in der $R_1$, $R_4$, $R_5$, m und n die bezüglich der Formel I in Anspruch 1 angegebenen Bedeutungen besitzen, $R'_2$ die bezüglich der Formel II angegebenen Bedeutungen besitzt und $R'_6$ die bezüglich der Formel III angegebenen Bedeutungen besitzt,

welche man mit einem Halogenid der Formel V umsetzt:

$$Hal - R_3 \qquad (V)$$

in der Hal ein Halogenatom darstellt und $R_3$ die bezüglich der Formel I in Anspruch 1 angegebenen Bedeutungen besitzt,

zur Bildung der Verbindungen der Formel I' und anschließend

- die Verbindungen der Formel I', in der $R'_2$ ein Wasserstoffatom bedeutet,

  * entweder der Einwirkung eines Halogens unterwirft zur Bildung der Verbindungen der Formel I nach Anspruch 1, in der $R_2$ ein Halogenatom darstellt, welche man anschließend, wenn $R_2$ ein Bromatom bedeutet, der Einwirkung eines anderen Halogenatoms unterwerfen kann zur Bildung der Verbindungen der Formel I, in der $R_2$ dieses Halogenatom darstellt,
  * oder der Einwirkung von Oxalylchlorid unterwirft zur Bildung der Verbindungen der Formel I nach Anspruch 1, in der $R_2$ eine Formylgruppe bedeutet, welche Verbindungen einer Reduktion unterworfen werden können zur Bildung der Verbindungen der Formel I nach Anspruch 1, in der $R_2$ eine Hydroxymethylgruppe darstellt,

  oder
- man die Verbindungen der Formel I', in der $R'_6$ eine Gruppe $R_6$ darstellt, welche Funktionen mit reaktiven Stickstoffatomen aufweist und eine Schutzgruppe umfaßt, einer sauren Hydrolyse unterwirft zur Bildung der Verbindungen der Formel I nach Anspruch 1, in der $R_6$ eine Gruppe bedeutet, die ein primäres oder sekundäres Amin enthält,

und gegebenenfalls die Verbindungen der Formel I nach Anspruch 1

entweder in ihre möglichen Stereoisomeren auftrennt und/oder mit einer anorganischen oder organischen Säure in ihre entsprechenden Salze überführt.

**10.** Verwendung der Verbindungen der Formel I nach irgendeinem der Ansprüche 1 bis 8 zur Herstellung eines Arzneimittels zur Behandlung von Erkrankungen, welche eine Modulierung der Wirkung des Faktors der Freisetzung des corticotropen Hormons (CRF) erforderlich machen.

**11.** Pharmazeutische Zubereitung enthaltend als Wirkstoff eine Verbindung nach irgendeinem der Ansprüche 1 bis 8 in Form der Base oder in Form eines Salzes mit einer anorganischen oder organischen, pharmazeutisch annehmbaren Säure in Kombination oder in Mischung mit einem inerten, nichttoxischen, pharmazeutisch annehmbaren Trägermaterial.

**12.** Verbindungen der Formel IV:

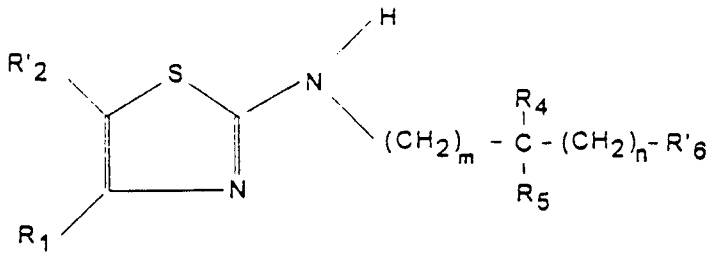

(IV)

in der

- $R_1$, $R_4$, m und n die bezüglich der Formel I in Anspruch 1 angegebenen Bedeutungen besitzen,
- $R'_2$ ein Wasserstoffatom oder eine Alkylgruppe mit 1 bis 5 Kohlenstoffatomen,
- $R_5$ eine Alkylgruppe mit 1 bis 5 Kohlenstoffatomen, eine Cycloalkylgruppe mit 3 bis 8 Kohlenstoffatomen, die gegebenenfalls durch Alkylgruppen mit 1 bis 5 Kohlenstoffatomen substituiert ist, eine Cycloalkylalkylgruppe mit 4 bis 8 Kohlenstoffatomen mit gerader oder verzweigter Kette, eine Alkenylgruppe mit 2 bis 6 Kohlenstoffatomen oder eine Gruppe der Formel B:

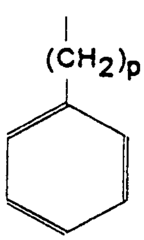

(B)

(in der p 1, 2 oder 3 darstellt),
bedeuten und

- $R'_6$ die bezüglich der $R_6$ bezüglich der Formel I in Anspruch 1 angegebenen Bedeutungen besitzt, mit Ausnahme des Falls, da $R_6$ Reste mit reaktiven Stickstoffatomen aufweist, in welchem Fall $R'_6$ die entsprechende Gruppe $R_6$ darstellt, in der ein Wasserstoffatom der reaktiven Gruppe durch eine in alkalischem Medium hydrolysebeständige Schutzgruppe ersetzt ist.

**Patentansprüche für folgenden Vertragsstaat : ES**

**1.** Verbindungen der Formel I:

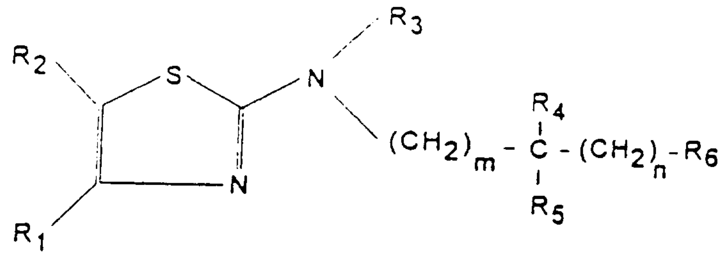

(I)

in der

- $R_1$ eine Gruppe der Formel $A_1$ oder eine Gruppe der Formel $A_2$:

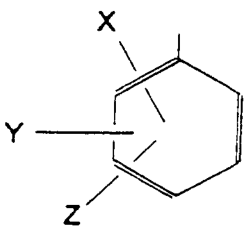

$(A_1)$

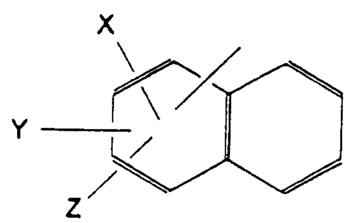

$(A_2)$

(in denen X, Y und Z, die gleichartig oder verschieden sein können, jeweils ein Wasserstoffatom, ein Halogenatom, eine Alkoxygruppe mit 1 bis 5 Kohlenstoffatomen, eine Alkylgruppe mit 1 bis 5 Kohlenstoffatomen, eine Hydroxylgruppe, eine Cyanogruppe, eine Nitrogruppe, eine Trifluormethylgruppe oder eine Aralkylgruppe mit 7 bis 9 Kohlenstoffatomen darstellen),
- $R_2$ ein Wasserstoffatom, ein Halogenatom, eine Alkylgruppe mit 1 bis 5 Kohlenstoffatomen, eine Hydroxymethylgruppe oder eine Formylgruppe,
- $R_3$ eine Alkylgruppe mit 1 bis 5 Kohlenstoffatomen, eine Cycloalkylgruppe mit 3 bis 8 Kohlenstoffatomen, eine Alkenylgruppe mit 2 bis 6 Kohlenstoffatomen, eine Cycloalkylalkylgruppe mit 4 bis 8 Kohlenstoffatomen oder eine Phenylgruppe,
- $R_4$ ein Wasserstoffatom, eine Alkylgruppe mit 1 bis 5 Kohlenstoffatomen, eine Cycloalkylgruppe mit 3 bis 6 Kohlenstoffatomen oder eine Cycloalkylalkylgruppe mit 4 bis 8 Kohlenstoffatomen mit einer geraden oder verzweigten Kette,
- $R_5$ eine Alkylgruppe mit 1 bis 5 Kohlenstoffatomen, eine Cycloalkylgruppe mit 3 bis 8 Kohlenstoffatomen, welche gegebenenfalls mit Alkylgruppen mit 1 bis 5 Kohlenstoffatomen substituiert ist, eine Cycloalkylalkylgruppe mit 4 bis 8 Kohlenstoffatomen und einer geraden oder verzweigten Kette, eine Alkenylgruppe mit 2 bis 6 Kohlenstoffatomen oder einen Rest der Formel B:

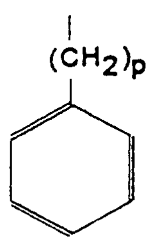

(B)

(in der p den Wert 0, 1, 2 oder 3 besitzt),
- $R_6$ eine Phenylgruppe, eine Pyridylgruppe, eine Imidazolylgruppe, eine Pyrrolylgruppe, eine Thienylgruppe oder eine Furylgruppe (die gegebenenfalls durch eines oder mehrere Halogenatome, Alkoxygruppen mit 1 bis 5 Kohlenstoffatomen, Alkylgruppen mit 1 bis 5 Kohlenstoffatomen, Hydroxylgruppen, Cyanogruppen, Nitrogruppen, Trifluormethylgruppen, Methylthiogruppen oder Gruppen der Formel B substituiert sind) oder eine Cycloalkylgruppe mit 3 bis 8 Kohlenstoffatomen, die gegebenenfalls mit Alkylgruppen mit 1 bis 5 Kohlenstoffatomen substituiert ist, und
- m und n, die gleich oder verschieden sein können, jeweils 0 oder 1 bedeuten,

deren mögliche Stereoisomeren und deren Additionssalze mit einer anorganischen oder organischen Säure.

2. Verbindungen der Formel I nach Anspruch 1, in der

- $R_1$ eine Gruppe der Formel $A_1$,
- $R_2$ ein Halogenatom oder eine Alkylgruppe mit 1 bis 5 Kohlenstoffatomen,
- $R_3$ eine Alkylgruppe mit 1 bis 5 Kohlenstoffatomen, eine Cycloalkylgruppe mit 3 bis 8 Kohlenstoffatomen oder eine Alkenylgruppe mit 2 bis 6 Kohlenstoffatomen,
- $R_5$ eine Alkylgruppe mit 1 bis 5 Kohlenstoffatomen, eine Cycloalkylgruppe mit 3 bis 8 Kohlenstoffatomen, die gegebenenfalls durch Alkylgruppen mit 1 bis 5 Kohlenstoffatomen substituiert ist, oder eine Cycloalkylalkylgruppe mit 4 bis 8 Kohlenstoffatomen mit gerader oder verzweigter Kette bedeuten, und
- $R_4$, $R_6$, m und n die bezüglich der Formel I in Anspruch 1 angegebenen Bedeutungen besitzen,

deren Stereoisomere sowie deren Additionssalze mit einer anorganischen oder organischen Säure.

3. Verbindungen der Formel I nach Anspruch 1 der Formel $I_A$:

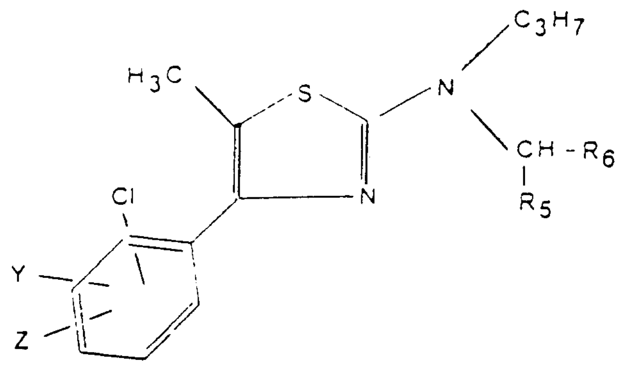

($I_A$)

in der

- Y und Z die bezüglich der Formel I in Anspruch 1 angegebenen Bedeutungen besitzen,
- $R_5$ eine Alkylgruppe mit 1 bis 5 Kohlenstoffatomen oder eine Cycloalkylgruppe mit 3 bis 8 Kohlenstoffatomen, die gegebenenfalls durch Alkylgruppen mit 1 bis 5 Kohlenstoffatomen substituiert ist, oder eine Cycloalkylalkylgruppe mit 4 bis 8 Kohlenstoffatomen mit gerader oder verzweigter Kette und
- $R_6$ eine Phenylgruppe oder eine Pyridylgruppe (die gegebenenfalls durch eines oder mehrere Halogenatome, Alkoxygruppen mit 1 bis 5 Kohlenstoffatomen oder Alkylgruppen mit 1 bis 5 Kohlenstoffatomen, Hydroxylgruppen, Cyanogruppen, Nitrogruppen, Trifluormethylgruppen oder Methylthiogruppen substituiert ist), eine Imidazolylgruppe, die gegebenenfalls durch eine Alkylgruppe mit 1 bis 5 Kohlenstoffatomen substituiert ist, oder eine Cycloalkylgruppe mit 3 bis 8 Kohlenstoffatomen, die gegebenenfalls durch Alkylgruppen mit 1 bis 5 Kohlenstoffatomen substituiert ist,

deren Stereoisomere oder deren Additionssalze mit einer anorganischen oder organischen Säure.

4. Verbindung der Formel I nach Anspruch 1, nämlich 4-(2,4-Dichlorphenyl)-5-methyl-2-[N-(cyclopropyl-pyrid-4-yl-methyl)-N-propylamino]-thiazol und dessen Additionssalze mit einer anorganischen oder organischen Säure.

5. Verbindung der Formel I nach Anspruch 1, nämlich 4-(2,4-Dichlorphenyl)-5-methyl-2-[N-(α-cyclopropylbenzyl)-N-propylamino]-thiazol und dessen Additionssalze mit einer anorganischen oder organischen Säure.

6. Verbindung der Formel I nach Anspruch 1, nämlich 4-(2,4-Dichlorphenyl)-5-methyl-2-[N-(dicyclopropylmethyl)-N-propylamino]-thiazol und dessen Additionssalze mit einer anorganischen oder organischen Säure.

7. Verbindung der Formel I nach Anspruch 1, nämlich 4-(2,4-Dichlorphenyl)-5-methyl-2-[N-(cyclopentyl-cyclopropyl-methyl)-N-propylamino]-thiazol und dessen Additionssalze mit einer anorganischen oder organischen Säure.

8. Verbindung der Formel I nach Anspruch 1, nämlich 4-(2-Chlor-4-methylphenyl)-5-methyl-2-[N-(dicyclopropylmethyl)-N-propylamino]-thiazol und dessen Additionssalze mit einer anorganischen oder organischen Säure.

9. Verfahren zur Herstellung der Verbindungen der Formel I

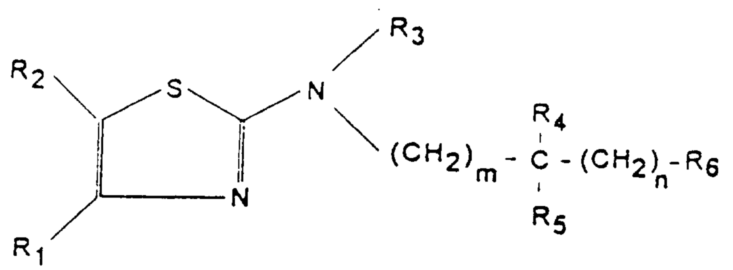

(I)

in der

- $R_1$ eine Gruppe der Formel $A_1$ oder eine Gruppe der Formel $A_2$:

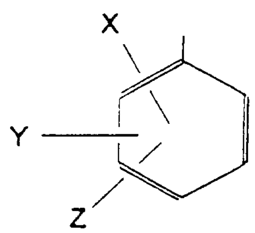

($A_1$)

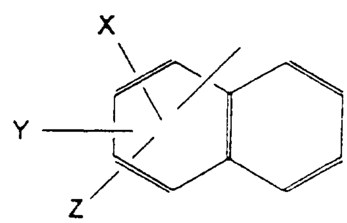

($A_2$)

(in denen X, Y und Z, die gleichartig oder verschieden sein können, jeweils ein Wasserstoffatom, ein Halogenatom, eine Alkoxygruppe mit 1 bis 5 Kohlenstoffatomen, eine Alkylgruppe mit 1 bis 5 Kohlenstoffatomen, eine Hydroxylgruppe, eine Cyanogruppe, eine Nitrogruppe, eine Trifluormethylgruppe oder eine Aralkylgruppe mit 7 bis 9 Kohlenstoffatomen darstellen),
- $R_2$ ein Wasserstoffatom, ein Halogenatom, eine Alkylgruppe mit 1 bis 5 Kohlenstoffatomen, eine Hydroxymethylgruppe oder eine Formylgruppe,
- $R_3$ eine Alkylgruppe mit 1 bis 5 Kohlenstoffatomen, eine Cycloalkylgruppe mit 3 bis 8 Kohlenstoffatomen, eine Alkenylgruppe mit 2 bis 6 Kohlenstoffatomen, eine Cycloalkylalkylgruppe mit 4 bis 8 Kohlenstoffatomen oder eine Phenylgruppe.
- $R_4$ ein Wasserstoffatom, eine Alkylgruppe mit 1 bis 5 Kohlenstoffatomen, eine Cycloalkylgruppe mit 3 bis 6 Kohlenstoffatomen oder eine Cycloalkylalkylgruppe mit 4 bis 8 Kohlenstoffatomen mit einer geraden oder verzweigten Kette,
- $R_5$ eine Alkylgruppe mit 1 bis 5 Kohlenstoffatomen, eine Cycloalkylgruppe mit 3 bis 8 Kohlenstoffatomen welche gegebenenfalls mit Alkylgruppen mit 1 bis 5 Kohlenstoffatomen substituiert ist, eine Cycloalkylalkylgruppe mit 4 bis 8 Kohlenstoffatomen und einer geraden oder verzweigten Kette, eine Alkenylgruppe mit 2 bis 6 Kohlenstoffatomen oder einen Rest der Formel B:

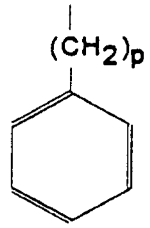

(B)

(in der p den Wert 0, 1, 2 oder 3 besitzt),

- $R_6$ eine Phenylgruppe, eine Pyridylgruppe, eine Imidazolylgruppe, eine Pyrrolylgruppe, eine Thienylgruppe oder eine Furylgruppe (die gegebenenfalls durch eines oder mehrere Halogenatome, Alkoxygruppen mit 1 bis 5 Kohlenstoffatomen, Alkylgruppen mit 1 bis 5 Kohlenstoffatomen, Hydroxylgruppen, Cyanogruppen, Nitrogruppen, Trifluormethylgruppen, Methylthiogruppen oder Gruppen der Formel B substituiert sind) oder eine Cycloalkylgruppe mit 3 bis 8 Kohlenstoffatomen, die gegebenenfalls mit Alkylgruppen mit 1 bis 5 Kohlenstoffatomen substituiert ist, und
- m und n, die gleich oder verschieden sein können, jeweils 0 oder 1 bedeuten,

und von deren möglichen Stereoisomeren und von deren Additionssalzen mit einer anorganischen oder organischen Säure, **dadurch gekennzeichnet**, daß man ein α-Halogencarbonyl-Derivat der Formel II:

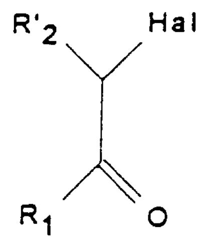

(II)

in der
$R_1$ die bezüglich der Formel I in Anspruch 1 angegebenen Bedeutungen besitzt und $R'_2$ ein Wasserstoffatom oder eine Alkylgruppe mit 1 bis 5 Kohlenstoffatomen und Hal ein Halogenatom bedeuten,
<u>entweder</u>
mit einem Thioharnstoff der Formel III umsetzt:

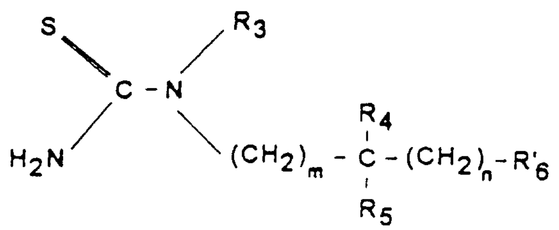

(III)

in der $R_3$, $R_4$, $R_5$, m und n die bezüglich der Formel I in Anspruch 1 angegebenen Bedeutungen besitzen und $R'_6$ die für $R_6$ bezüglich der Formel I in Anspruch 1 angegebenen Bedeutungen besitzt mit Ausnahme des Falls, bei dem $R_6$ Reste mit reaktiven Stickstoffatomen aufweist, in welchem Fall $R'_6$ die entsprechende Gruppe $R_6$ darstellt, in der ein Wasserstoffatom der reaktiven Funktion durch eine gegen die Hydrolyse in alkalischem Medium beständige Schutzgruppe ersetzt ist,
zur Bildung einer Verbindung der Formel I':

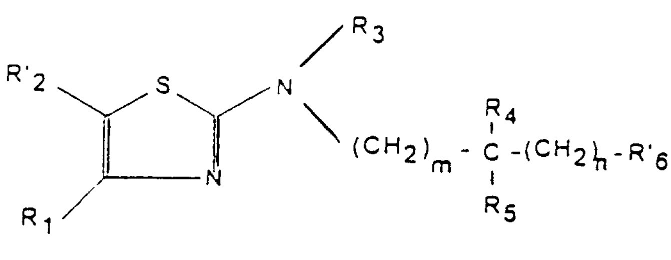

(I')

in der

- $R_1$, $R_3$, $R_4$, $R_5$, m und n die bezüglich der Formel I in Anspruch 1 angegebenen Bedeutungen besitzen.
- $R'_2$ die bezüglich der Formel II angegebenen Bedeutungen besitzt und
- $R'_6$ die bezüglich der Formel III angegebenen Bedeutungen besitzt,

<u>oder</u>

mit einem Thioharnstoff der Formel $III_A$ umsetzt:

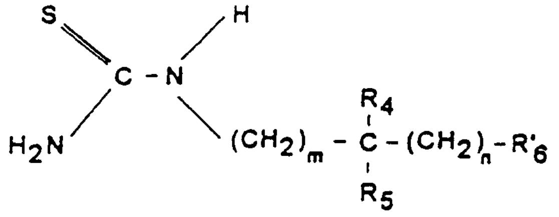

($III_A$)

in der $R_4$, $R_5$, m und n die bezüglich der Formel I in Anspruch 1 angegebenen Bedeutungen besitzen und $R'_6$ die bezüglich der Formel III angegebenen Bedeutungen aufweist,

zur Bildung der Verbindungen der Formel IV:

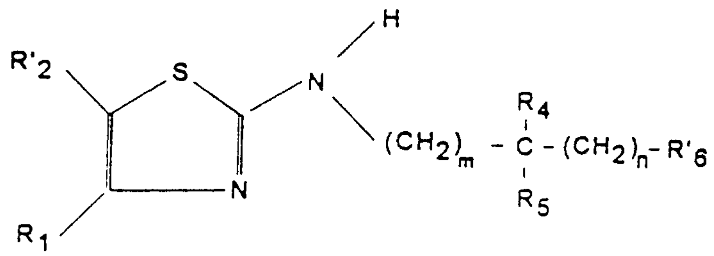

(IV)

in der $R_1$, $R_4$, $R_5$, m und n die bezüglich der Formel I in Anspruch 1 angegebenen Bedeutungen besitzen, $R'_2$ die bezüglich der Formel II angegebenen Bedeutungen besitzt und $R'_6$ die bezüglich der Formel III angegebenen Bedeutungen besitzt,

welche man mit einem Halogenid der Formel V umsetzt:

$$Hal - R_3 \qquad (V)$$

in der Hal ein Halogenatom darstellt und $R_3$ die bezüglich der Formel I in Anspruch 1 angegebenen Bedeutungen besitzt,

zur Bildung der Verbindungen der Formel I'

<u>und anschließend</u>

- die Verbindungen der Formel I', in der $R'_2$ ein Wasserstoffatom bedeutet,
  - * entweder der Einwirkung eines Halogens unterwirft zur Bildung der Verbindungen der Formel I nach Anspruch 1, in der $R_2$ ein Halogenatom darstellt, welche man anschließend, wenn $R_2$ ein Bromatom bedeutet, der Einwirkung eines anderen Halogenatoms unterwerfen kann zur Bildung der Verbindungen der Formel I, in der $R_2$ dieses Halogenatom darstellt,
  - * oder der Einwirkung von Oxalylchlorid unterwirft zur Bildung der Verbindungen der Formel I nach Anspruch 1, in der $R_2$ eine Formylgruppe bedeutet, welche Verbindungen einer Reduktion unterworfen werden können zur Bildung der Verbindungen der Formel I nach Anspruch 1, in der $R_2$ eine Hydroxymethylgruppe darstellt,

  oder
- man die Verbindungen der Formel I', in der $R'_6$ eine Gruppe $R_6$ darstellt, welche Funktionen mit reaktiven Stickstoffatomen aufweist und eine Schutzgruppe umfaßt, einer sauren Hydrolyse unterwirft zur Bildung der Verbindungen der Formel I nach Anspruch 1, in der $R_6$ eine Gruppe bedeutet, die ein primäres oder sekundäres Amin enthält,

<u>und gegebenenfalls die Verbindungen der Formel I nach Anspruch 1</u>

entweder in ihre möglichen Stereoisomeren auftrennt und/oder mit einer anorganischen oder organischen Säure in ihre entsprechenden Salze überführt.

10. Verwendung der Verbindungen der Formel I nach irgendeinem der Ansprüche 1 bis 8 zur Herstellung eines Arzneimittels zur Behandlung von Erkrankungen, welche eine Modulierung der Wirkung des Faktors der Freisetzung des corticotropen Hormons (CRF) erforderlich machen.

**11.** Pharmazeutische Zubereitung enthaltend als Wirkstoff eine Verbindung nach irgendeinem der Ansprüche 1 bis 8 in Form der Base oder in Form eines Salzes mit einer anorganischen oder organischen, pharmazeutisch annehmbaren Säure in Kombination oder in Mischung mit einem inerten, nichttoxischen, pharmazeutisch annehmbaren Trägermaterial.

**12.** Verbindungen der Formel IV:

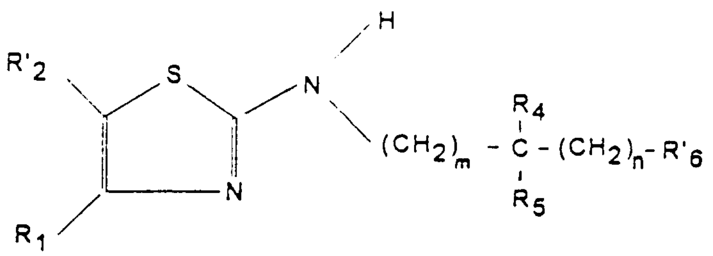

(IV)

in der

- $R_1$, $R_4$, m und n die bezüglich der Formel I in Anspruch 1 angegebenen Bedeutungen besitzen,
- $R'_2$ ein Wasserstoffatom oder eine Alkylgruppe mit 1 bis 5 Kohlenstoffatomen,
- $R_5$ eine Alkylgruppe mit 1 bis 5 Kohlenstoffatomen, eine Cycloalkylgruppe mit 3 bis 8 Kohlenstoffatomen, die gegebenenfalls durch Alkylgruppen mit 1 bis 5 Kohlenstoffatomen substituiert ist, eine Cycloalkylalkylgruppe mit 4 bis 8 Kohlenstoffatomen mit gerader oder verzweigter Kette, eine Alkenylgruppe mit 2 bis 6 Kohlenstoffatomen oder eine Gruppe der Formel B:

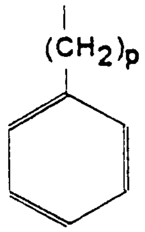

(B)

(in der p 1, 2 oder 3 darstellt),
bedeuten und
- $R'_6$ die bezüglich der $R_6$ bezüglich der Formel I in Anspruch 1 angegebenen Bedeutungen besitzt, mit Ausnahme des Falls, da $R_6$ Reste mit reaktiven Stickstoffatomen aufweist, in welchem Fall $R'_6$ die entsprechende Gruppe $R_6$ darstellt, in der ein Wasserstoffatom der reaktiven Gruppe durch eine in alkalischem Medium hydrolysebeständige Schutzgruppe ersetzt ist.

**13.** Verfahren nach Anspruch 9, **dadurch gekennzeichnet**, daß man eine Verbindung der Formel I herstellt, in der:

- $R_1$ eine Gruppe der Formel $A_1$,
- $R_2$ ein Halogenatom oder eine Alkylgruppe mit 1 bis 5 Kohlenstoffatomen,
- $R_3$ eine Alkylgruppe mit 1 bis 5 Kohlenstoffatomen, eine Cycloalkylgruppe mit 3 bis 8 Kohlenstoffatomen oder eine Alkenylgruppe mit 2 bis 6 Kohlenstoffatomen,
- $R_5$ eine Alkylgruppe mit 1 bis 5 Kohlenstoffatomen, eine Cycloalkylgruppe mit 3 bis 8 Kohlenstoffatomen, die gegebenenfalls durch Alkylgruppen mit 1 bis 5 Kohlenstoffatomen substituiert, oder eine Cycloalkylalkylgruppe mit 4 bis 8 Kohlenstoffatomen mit gerader oder verzweigter Kette bedeuten, und
- $R_4$, $R_6$, m und n die bezüglich der Formel I in Anspruch 1 angegebenen Bedeutungen besitzen,

und von einem seiner Stereoisomeren oder Additionssalzen mit einer anorganischen oder organischen Säure.

**14.** Verfahren nach Anspruch 9, **dadurch gekennzeichnet**, daß man eine Verbindung der Formel $I_A$ herstellt:

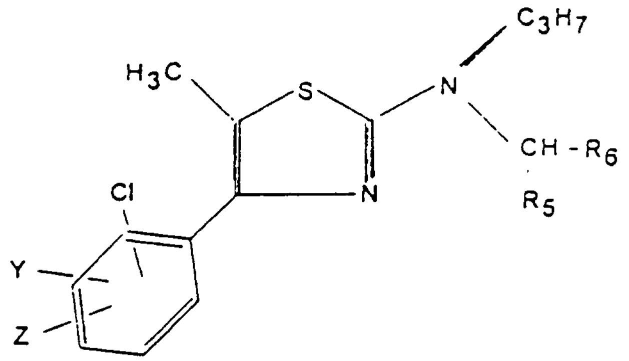

$(I_A)$

in der

- Y und Z die bezüglich der Formel I in Anspruch 1 angegebenen Bedeutungen besitzen,
- $R_5$ eine Alkylgruppe mit 1 bis 5 Kohlenstoffatomen oder eine Cycloalkylgruppe mit 3 bis 8 Kohlenstoffatomen, die gegebenenfalls durch Alkylgruppen mit 1 bis 5 Kohlenstoffatomen substituiert, oder eine Cycloalkylalkylgruppe mit 4 bis 8 Kohlenstoffatomen mit gerader oder verzweigter Kette und
- $R_6$ eine Phenylgruppe oder eine Pyridylgruppe (die gegebenenfalls durch eines oder mehrere Halogenatome, Alkoxygruppen mit 1 bis 5 Kohlenstoffatomen oder Alkylgruppen mit 1 bis 5 Kohlenstoffatomen, Hydroxylgruppen, Cyanogruppen, Nitrogruppen, Trifluormethylgruppen oder Methylthiogruppen substituiert ist), eine Imidazolygruppe, die gegebenenfalls durch eine Alkylgruppe mit 1 bis 5 Kohlenstoffatomen substituiert ist, oder eine Cycloalkylgruppe mit 3 bis 8 Kohlenstoffatomen, die gegebenenfalls durch Alkylgruppen mit 1 bis 5 Kohlenstoffatomen substituiert ist,

und von einem seiner Stereoisomeren oder von dessen Additionssalzen mit einer anorganischen oder organischen Säure.

**15.** Verfahren nach Anspruch 9, **dadurch gekennzeichnet**, daß man:

- 4-(2,4-Dichlorphenyl)-5-methyl-2-[N-(α-cyclopropylbenzyl)-N-propylamino]-thiazol,
- 4-(2,4-Dichlorphenyl)-5-methyl-2-[N-(dicyclopropylmethyl)-N-propylamino]-thiazol,
- 4-(2,4-Dichlorphenyl)-5-methyl-2-[N-(cyclopentyl-cyclopropyl-methyl)-N-propylamlno]-thiazol,
- 4-(2-Chlor-4-methylphenyl)-5-methyl-2-[N-(dicyclopropylmethyl)-N-propylamino]-thiazol

oder eines ihrer Additionssalze mit einer anorganischen oder organischen Säure herstellt.